(19) Europäisches Patentamt
European Patent Office
Office européen des brevets

(11) EP 0 882 695 A1

(12) EUROPEAN PATENT APPLICATION
published in accordance with Art. 158(3) EPC

(43) Date of publication:
**09.12.1998 Bulletin 1998/50**

(21) Application number: **97900763.0**

(22) Date of filing: **23.01.1997**

(51) Int. Cl.$^6$: **C07C 43/166**, C07C 43/176, C07C 43/188, C07C 43/192, C07C 43/215, C07C 43/225, C07C 69/734, C07C 69/757, C07C 69/92, C07C 69/587, C07C 69/608

(86) International application number:
**PCT/JP97/00148**

(87) International publication number:
**WO 97/27166 (31.07.1997 Gazette 1997/33)**

(84) Designated Contracting States:
**DE FR GB**

(30) Priority: **23.01.1996 JP 29880/96**
**07.11.1996 JP 311406/96**

(71) Applicant: **CHISSO CORPORATION**
**Osaka-shi, Osaka-fu 530 (JP)**

(72) Inventors:
- **KOIZUMI, Yasuyuki**
  **Ichihara-shi, Chiba 290 (JP)**
- **MATSUI, Shuichi**
  **Ichihara-shi, Chiba 290 (JP)**
- **MIYAZAWA, Kazutoshi**
  **Ichihara-shi, Chiba 290-01 (JP)**
- **SEKIGUCHI, Yasuko**
  **Ichihara-shi, Chiba 290 (JP)**
- **NAKAGAWA, Etsuo**
  **Ichihara-shi, Chiba 290 (JP)**

(74) Representative: **HOFFMANN - EITLE**
**Patent- und Rechtsanwälte**
**Arabellastrasse 4**
**81925 München (DE)**

# (54) BENZYL ETHER DERIVATIVES, LIQUID CRYSTAL COMPOSITION, AND LIQUID CRYSTAL DISPLAY

(57) A liquid crystal compound represented by formula (1) and having a high elastic constant ratio, a lower viscosity than existing liquid crystal compounds, an excellent compatibility with other liquid crystal compounds particularly at low temperatures, and also a chemical stability, a liquid crystal composition comprising the same, and a liquid crystal display made by using the same (wherein $R_1$ represents an alkyl or alkenyl group; $R_2$ represents an alkylene group; $R_3$ represents an alkenyl group; ring A represents a phenylene group; rings B, C, and D represent each a cyclohexylene or phenylene group; $Z_1$ to $Z_3$ represent each a single bond, an alkylene, -CH=CH- or the like; and n and m are each 0 or 1).

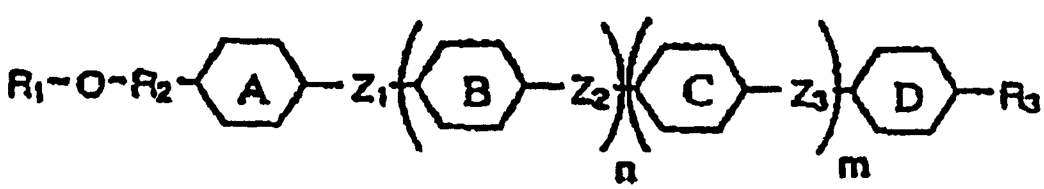

(1)

EP 0 882 695 A1

Printed by Xerox (UK) Business Services
2.16.6/3.4

## Description

### TECHNICAL FIELD

The present invention relates to novel liquid crystalline compounds which develop favorable physical properties in liquid crystal compositions principally for twist nematic (TN) mode or super twist nematic (STN) mode; to liquid crystal compositions having favorable physical properties and comprising the liquid crystalline compound; and to liquid crystal display devices comprising the liquid crystal composition.

### BACKGROUND ART

Liquid crystal display devices employ the optical anisotropy and dielectric anisotropy of liquid crystl substance, and display devices of TN mode or STN mode which use nematic phase have widely been used. Among them, the devices of STN mode are excellent as liquid crystal display devices of simple matrix drive as a whole in the aspects of many characteristics such as large display capacity, high response speed, wide viewing angle, and gradation (tone wedge). Also in color displays, display devices of STN mode have come into wide use on the market from their economical advantage that the devices can be manufactures at a lower production cost than thin film transistor (TFT) display devices.

Liquid crystal compounds used for these liquid crystal display devices are required, as their general properties, that they are chemically stable, that they have a wide temperature range at which the compounds exhibit liquid crystal phase, with room temperature being its center, that they have a low viscosity, and that their driving voltage is low.

Besides, depending on the structure of display devices and driving mode, it is necessary to adjust the value of various physical properties such as dielectric anisotropy ($\Delta\varepsilon$), optical anisotropy ($\Delta n$), ratio of elastic constants K33/K11 (K33: bend elastic constant, K11: splay elastic constant).

However, no single liquid crystal substance has been found which satisfies these requirements at the same time, and thus it is a current situation that several to twenty-odd liquid crystalline compounds and further several liquid non-crystalline compounds, when necessary, are mixed to prepare liquid crystal compositions, and used for liquid crystal display devices. Accordingly, liquid crystalline compounds are required to have a good mutual solubility with other liquid crystal compounds, and a good mutual solubility particularly at low temperatures from the needs of being used in low temperature environment these days.

In order to actualize a high quality picture, the steepness of electrooptical characteristics at threshold value is required of liquid crystal compositions used for display devices of STN mode. The steepness is a function of the ratio of elastic constants (K33/K11), and compositions comprising liquid crystalline compound having a lager value of the ratio are known to have steeper electrooptical characteristics (F. Leenhouts et al., Proceedings of the Japan Display, 388 (1986)).

Further, in order to actualize display devices of a large-sized picture, it is necessary to use a liquid crystal composition having a high response speed. Response speed is known to be a function of viscosity (E. Jakeman et al., Phys. Lett., 39a, 69 (1972), and thus liquid crystalline compounds of a low viscosity are sought for obtaining liquid crystal compositions of a high response speed.

As compounds having a large ratio of elastic constants (K33/K11), compounds having an alkenyl moiety and shown below are known:

(10) 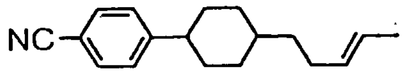 K33/K11 2.28

(11) 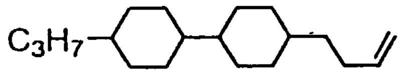 K33/K11 1.78

(12) 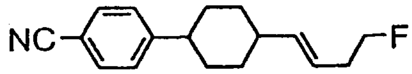 K33/K11 2.28

(13) 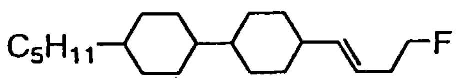 K33/K11 1.90

(14) 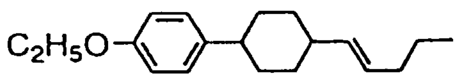

That is, compound of the formula (10) is disclosed in M. Schadt: Mol. Cryst. Liq. Cryst., 122 (1985); compound of the formula (11) is disclosed in Laid-open Japanese Patent Publication No. Sho 61-83136; compounds to which fluorine atom is introduced and expressed by the formula (12) or (13) are disclosed in Laid-open Japanese Patent Publication No. Hei 6-92740; and compound having both an alkenyl group and an alkoxybenzene group, and expressed by the formula (14) is disclosed in Japanese Patent Publication No. Hei 4-30382, respectively.

When the present inventors synthesized the compounds expressed by one of the formulas (10) to (13) according to the conditions described in the literatures mentioned above, added the compounds to a liquid crystal composition (Mother liquid crystal A), respectively, in the same manner as described in Examples 6 to 8 below, and then determined the ratio of elastic constants, it was found that any of the compounds exhibited a large value of the ratio as appended to the structural formula of the compounds described above. However, they had such defects that the alkenyl derivative of the formula (10) or (11) had a poor mutual solubility and that the compound of the formula (12) or (13) exhibited a high viscosity because of the introduction of fluorine atom. Besides, it was found that the compound of the formula (14) exhibited a high viscosity based on a partial structure of an alkoxybenzene.

As the compounds having a large ratio of elastic constants (K33/K11), the compound expressed by the formula (15), having 1,3-butadienyl group, and described in Japanese Patent Application No. Hei 6-151445 is known.

(15) 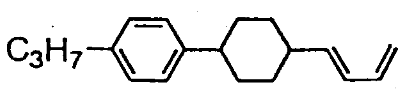 K33/K11 1.98

However, the compound of the formula (15) can not be used as liquid crystal composition since it is unstable because it has a conjugated diene moiety.

DISCLOSURE OF THE INVENTION

An object of the present invention is to provide novel liquid crystalline compounds which have a large ratio of elastic constants and low viscosity compared with existing liquid crystalline compounds, are excellent in mutual solubility with other liquid crystal compounds, particularly in the at low temperatures, and are chemically stable; to provide liquid crystal compositions comprising the liquid crystalline compound; and to provide liquid crystal display devices utilizing the liquid crystal composition.

Compounds having oxygen atom in a partial structure comprising phenylene group and a terminal alkyl group or alkenyl group linking to the phenylene group, and having a terminal alkenyl group linking to cyclohexylene group at the other terminal portion have already been disclosed in DE 4,211,694 A1, WO 90 15,114, and Laid-open Japanese Patent Publication No. Hei 7-53432. However, physical data and specific physical properties, for evaluating the utility as liquid crystalline compound, of the compounds have not been described in those publications and thus the characteristics of the compounds have not been known at all.

Under such circumstances, the compounds expressed by the general formula (1) and characterized by having oxygen atom in a partial structure comprising phenylene group and a terminal alkyl group or alkenyl group linking to the phenylene group, and having a terminal alkenyl group linking to cyclohexylene group at the other terminal portion were conceived by the present inventors; and as the result of various investigations on their physical properties, it has now been found that they had a low viscosity while having a comparatively high clearing point, and a large ratio of elastic constants compared with conventional compounds. Further, it has come to find that the compounds of the present invention are remarkably excellent in mutual solubility with known liquid crystalline compounds, particularly in that at low temperatures, and have improved characteristics than known compounds.

That is, the present invention has the following aspects [1] to [12]:

[1] A liquid crystalline compound expressed by the general formula (1)

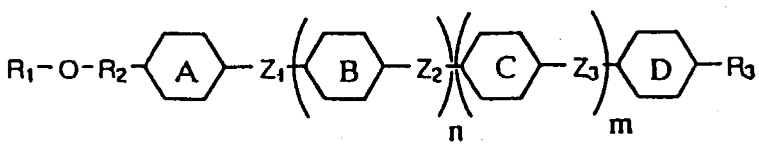

(1)

wherein $R_1$ represents an alkyl group having 1 to 15 carbon atoms or an alkenyl group having 2 to 15 carbon atoms; $R_2$ represents an alkylene group having 1 to 5 carbon atoms; $R_3$ represents an alkenyl group having 2 to 15 carbon atoms; ring A represents 1,4-phenylene group one or more hydrogen atoms on the ring may be replaced by a halogen atom; rings B, C, and D independently represent 1,4-cyclohexylene group, or 1,4-phenylene group one or more hydrogen atoms on the ring may be replaced by a halogen atom; $Z_1$, $Z_2$, and $Z_3$ independently represent an alkylene group having 1 to 5 carbon atoms, -CH=CH-, -C≡C-, $-CH_2-CH_2-CH=CH-$, $-CH=CH-CH_2-CH_2-$, $-CH_2-CH=CH-CH_2-$, or single bond, one or not-adjacent two or more $-CH_2-CH_2-$ groups in these groups may be replaced by $-CH_2O-$, $-OCH_2-$ $-CF_2O-$, $-OCF_2-$, -COO-, or -OCO-; and n and m are 0 or 1.

[2] The liquid crystalline compound recited in the aspect [1] wherein both n and m are 0 in the general formula (1).

[3] The liquid crystalline compound recited in the aspect [2] wherein $R_2$ is methylene group and ring D is 1,4-cyclohexylene group in the general formula (1).

[4] The liquid crystalline compound recited in the aspect [1] wherein n is 0, and m is 1 in the general formula (1).

[5] The liquid crystalline compound recited in the aspect [4] wherein $R_2$ is methylene group, and ring D is 1,4-cyclohexylene group in the general formula (1).

[6] The liquid crystalline compound recited in the aspect [1] wherein both n and m are 1 in the general formula (1).

[7] The liquid crystalline compound recited in the aspect [6] wherein $R_2$ is methylene group, and ring D represents 1,4-cyclohexylene group in the general formula (1).

[8] A liquid crystal composition comprising at least 2 components and comprising at least one compound expressed by the general formula (1).

[9] A liquid crystal composition comprising, as a first component, at least one liquid crystalline compound recited in any one of the aspects [1] to [7]; and comprising, as a second component, at least one compound selected from the group consisting of the compounds expressed by any one of the general formulas (2), (3), and (4)

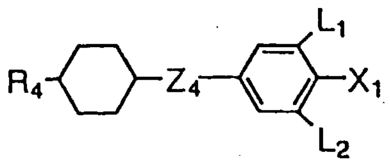

(2)

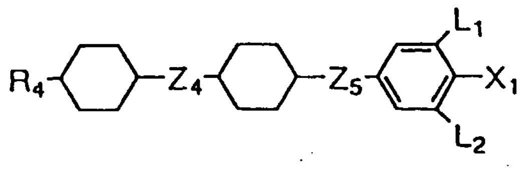

(3)

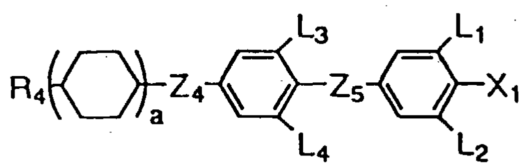

(4)

wherein $R_4$ represents an alkyl group having 1 to 10 carbon atoms; $X_1$ represents F, Cl, $OCF_3$, $OCF_2H$, $CF_3$, $CF_2H$, or $CFH_2$; $L_1$, $L_2$, $L_3$, and $L_4$ independently represent H or F; $Z_4$ and $Z_5$ independently represent $-(CH_2)_2-$, -CH=CH-, or single bond; and a is 1 or 2.

[10] A liquid crystal composition comprising, as a first component, at least one liquid crystalline compound recited in any one of the aspects [1] to [7]; and comprising, as a second component, at least one compound selected from the group consisting of the compounds expressed by any one of the general formulas (5), (6), (7), (8), and (9)

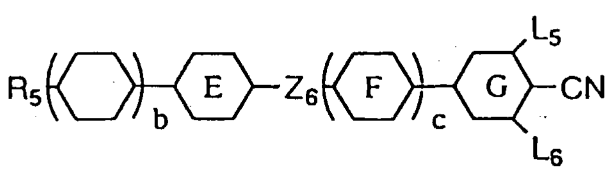

(5)

wherein $R_5$ represents F, an alkyl group having 1 to 10 carbon atoms, or an alkenyl group having 2 to 10 carbon atoms provided that one or not-adjacent two or more methylene ($-CH_2-$) groups in the alkyl or alkenyl group may be replaced by oxygen (-O-) atom; ring E represents trans-1,4-cyclohexylene group, 1,4-phenylene group, pyrimidine-2,5-diyl group, or 1,3-dioxane-2,5-diyl group; ring F represents trans-1,4-cyclohexylene group, 1,4-phenylene group, or pyrimidine-2,5-diyl group; ring G represents trans-1,4-cyclohexylene group or 1,4-phenylene group; $Z_6$ represents $-(CH_2)_2-$, -COO-, or single bond; $L_5$ and $L_6$ independently represent H or F; and b and c are independently 0 or 1,

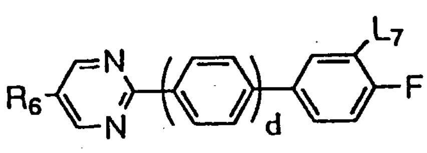

(6)

wherein $R_6$ represents an alkyl group having 1 to 10 carbon atoms; $L_7$ represents H or F; and d is 0 or 1,

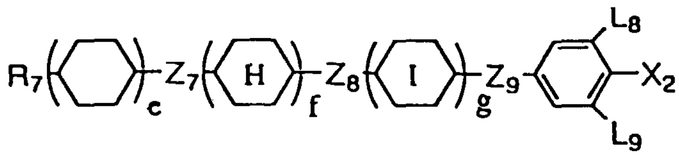

(7)

wherein $R_7$ represents an alkyl group having 1 to 10 carbon atoms, ring H and ring I independently represent trans-1,4-cyclohexylene group or 1,4-phenylene group; $Z_7$ and $Z_8$ independently represent -COO- or single bond; $Z_9$ represents -COO- or -C≡C-; $L_8$ and $L_9$ independently represent H or F; $X_2$ represents F, $OCF_3$, $OCF_2H$, $CF_3$, $CF_2H$, or $CFH_2$; and e, f, and g are independently 0 or 1,

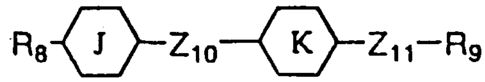

(8)

wherein $R_8$ and $R_9$ independently represent an alkyl group having 1 to 10 carbon atoms or an alkenyl group having 2 to 10 carbon atoms provided that one or not-adjacent two or more methylene ($-CH_2-$) groups in the alkyl or alkenyl group may be replaced by oxygen (-O-) atom; ring J represents trans-1,4-cyclohexylene group, 1,4-phenylene group, or pyrimidine-2,5-diyl group; ring K represents trans-1,4-cyclohexylene group or 1,4-phenylene group; $Z_{10}$ represents -C≡C-, -COO-, $-(CH_2)_2-$, -CH=CH-C≡C-, or single bond; and $Z_{11}$ represents -COO- or single bond,

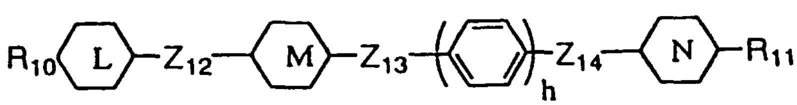

(9)

wherein $R_{10}$ and $R_{11}$ independently represent an alkyl group having 1 to 10 carbon atoms or an alkenyl group having 2 to 10 carbon atoms provided that one or not-adjacent two or more methylene ($-CH_2-$) groups in the alkyl or alkenyl group may be replaced by oxygen (-O-) atom; ring L represents trans-1,4-cyclohexylene group, 1,4-phenylene group, or pyrimidine-2,5-diyl group; ring M represents trans-1,4-cyclohexylene group, pyrimidine-2,5-diyl group, or 1,4-phenylene group one or more hydrogen atoms on the ring may be replaced by fluorine atom; ring N represents trans-1,4-cyclohexylene group or 1,4-phenylene group, $Z_{12}$ and $Z_{14}$ independently represent -COO-, $-(CH_2)_2-$ or single bond; $Z_{13}$ represents -CH=CH-, -C≡C-, -COO-, or single bond; and h is 0 or 1.

[11] A liquid crystal composition comprising, as a first component, at least one liquid crystalline compound recited in any one of the aspects [1] to [7]; comprising, as a part of a second component, at least one compound selected from the group consisting of the compounds expressed by any one of the general formulas (2), (3), and (4); and comprising, as another part of the second component, at least one compound selected from the group consisting of the compounds expressed by any one of the general formulas (5), (6), (7), (8), and (9).

[12] A liquid crystal display device utilizing a liquid crystal composition recited in any one of the aspects [8] to [11].

Preferable compounds of the present invention expressed by the general formula (1) are those expressed by one of the following general formulas (1-1) to (1-13)

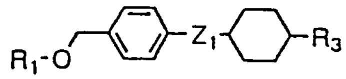 (1-1)

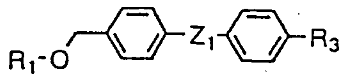 (1-2)

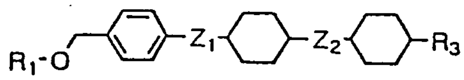 (1-3)

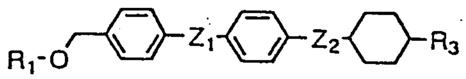 (1-4)

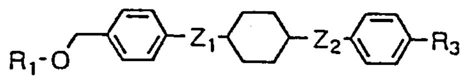 (1-5)

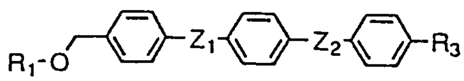 (1-6)

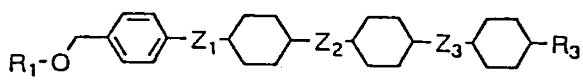 (1-7)

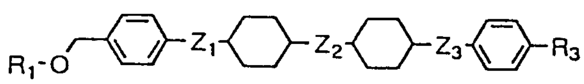 (1-8)

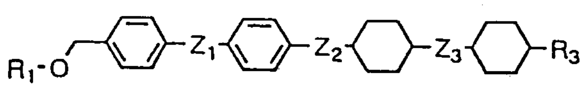 (1-9)

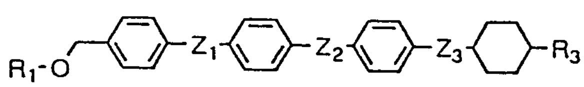 (1-10)

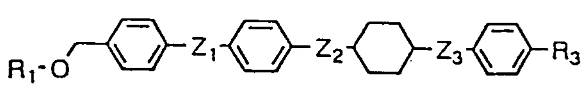 (1-11)

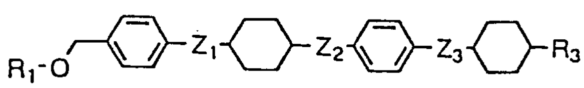 (1-12)

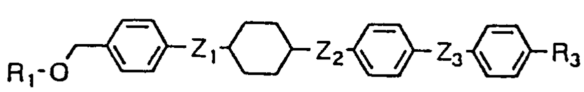 (1-13)

wherein $R_1$, $R_3$, $Z_1$, $Z_2$, and $Z_3$ have the same meaning as described above.

While any of these compounds exhibits preferable characteristics, preferable embodiments of $R_1$, $R_3$, $Z_1$, $Z_2$, and $Z_3$ are mentioned below.

With respect to $R_1$, the followings are preferable:

Methyl, ethyl, n-propyl, n-butyl, n-pentyl, n-hexyl, n-heptyl, n-octyl, n-nonyl, n-decyl, ethenyl, 1-propenyl, 1-butenyl, 1-pentenyl, 1-hexenyl, 1-heptenyl, 1-octenyl, 1-nonenyl, 1-decenyl, 2-propenyl, 2-butenyl, 2-pentenyl, 2-hexenyl, 2-heptenyl, 2-octenyl, 2-nonenyl, 2-decenyl, 3-butenyl, 3-pentenyl, 3-hexenyl, 3-heptenyl, 4-pentenyl, 4-hexenyl, 4-heptenyl, 4-octenyl, 5-hexenyl, 5-heptenyl, 5-octenyl, 5-nonenyl, 5-decenyl, 6-heptenyl, 6-octenyl, and 7-octenyl.

With respect to $R_3$, the followings are preferable:

Ethenyl, 1-propenyl, 1-butenyl, 1-pentenyl, 1-hexenyl, 1-heptenyl, 1-octenyl, 1-nonenyl, 1-decenyl, 2-propenyl, 2-butenyl, 2-pentenyl, 2-hexenyl, 2-heptenyl, 2-octenyl, 2-nonenyl, 2-decenyl, 3-butenyl, 3-pentenyl, 3-hexenyl, 3-heptenyl, 4-pentenyl, 4-hexenyl, 4-heptenyl, 4-octenyl, 5-hexenyl, 5-heptenyl, 5-octenyl, 5-nonenyl, 5-decenyl, 6-heptenyl, 6-octenyl, and 7-octenyl.

As $Z_1$, $Z_2$, and $Z_3$, the followings are independently preferable:

Single bond, methylene, ethylene, butylene, vinylene, 1-butenylene, 2-butenylene, 3-butenylene, ethynylene, -COO-, -OCO-, $-CH_2O-$, $-CF_2O-$, and $-OCF_2-$.

Any of the compounds of the present invention exhibits preferable physical properties, and liquid crystal compositions having characteristics which meet their purposes can be produced by using compounds expressed by the general formula (1) in which $R_1$, $R_2$, $R_3$, $Z_1$, $Z_2$, $Z_3$, and rings A, B, C, and D are properly selected. For instance, when liquid crystal compositions which have a temperature range, at which the compositions show liquid crystal phase, at high temperature side are produced, three-ring or four-ring compounds can be used. When it is not so, two-ring or three-ring compounds can be used.

Value of optical anisotropy can also be adjusted by selecting $R_1$, $R_2$, $R_3$, $Z_1$, $Z_2$, $Z_3$, and rings A, B, C, and D in the general formula (1). That is, when liquid crystal compositions having a large value of optical anisotropy are necessary, it is sufficient to use compounds having many 1,4-phenylene rings, and when liquid crystal compositions having a small value of optical anisotropy are necessary, compounds having many 1,4-cyclohexylene rings are sufficiently used.

Liquid crystal compositions of the present invention comprise at least one compound expressed by the general formula (1) in a ratio of 0.1 to 99.9 % by weight.

Preferable liquid crystal compositions provided by the present invention are obtained by mixing the compound selected from the group of compounds expressed by at least one of the general formulas (2) to (9) depending on the purposes of the compositions to a first component comprising at least one compound expressed by the general formula (1).

As preferable examples of the compounds expressed by one of the general formulas (2) to (4) and used in the liquid crystal compositions of the present invention, the following compounds can be mentioned:

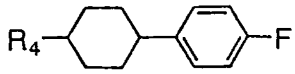 (2-1)

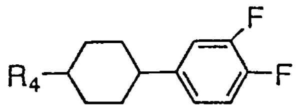 (2-2)

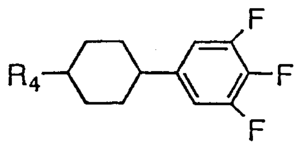 (2-3)

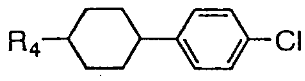 (2-4)

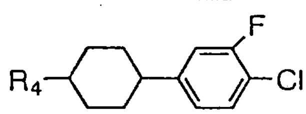 (2-5)

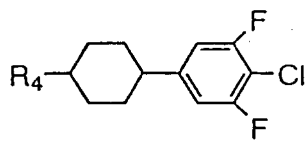 (2-6)

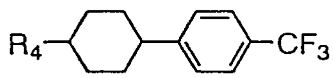 (2-7)

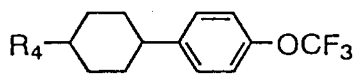 (2-8)

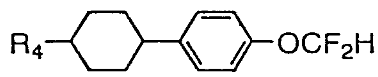 (2-9)

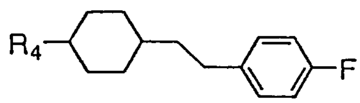 (2-10)

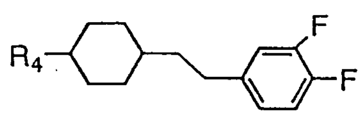 (2-11)

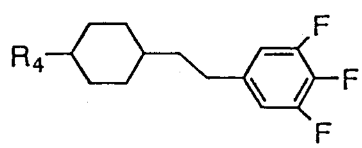 (2-12)

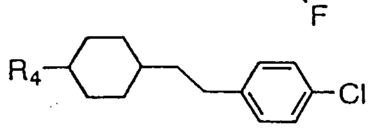 (2-13)

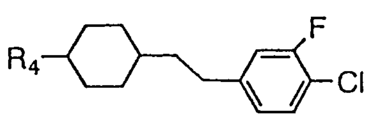 (2-14)

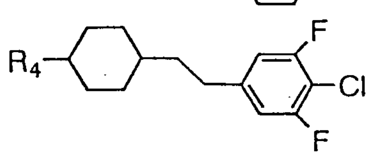 (2-15)

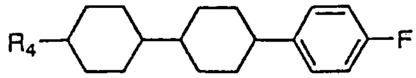
(3-1)

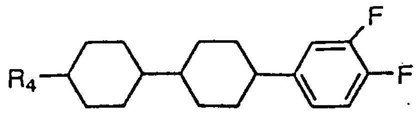
(3-2)

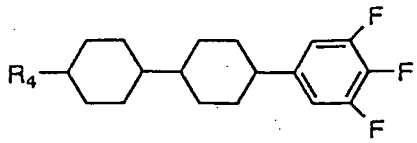
(3-3)

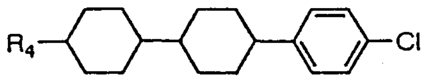
(3-4)

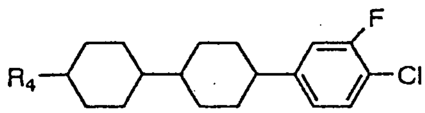
(3-5)

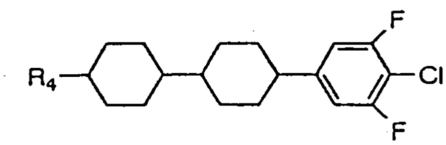
(3-6)

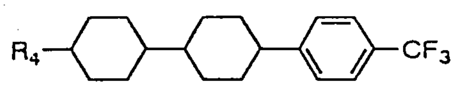
(3-7)

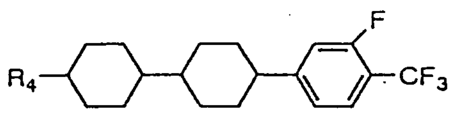
(3-8)

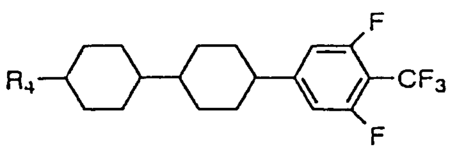
(3-9)

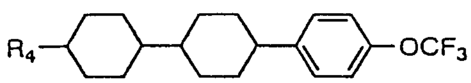
(3-10)

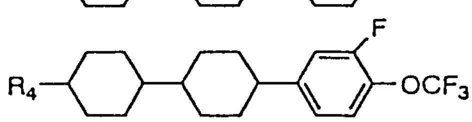
(3-11)

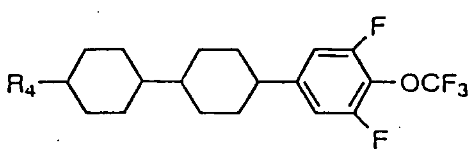
(3-12)

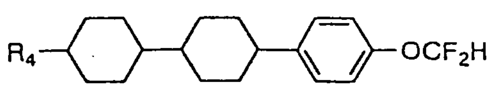
(3-13)

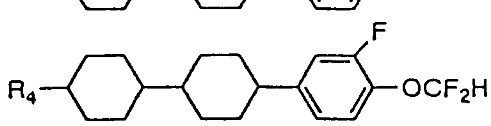
(3-14)

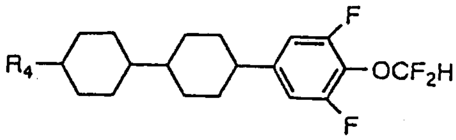

(3-15)

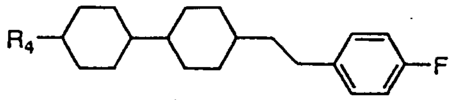

(3-16)

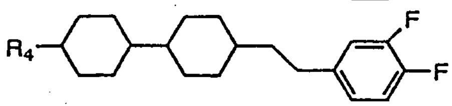

(3-17)

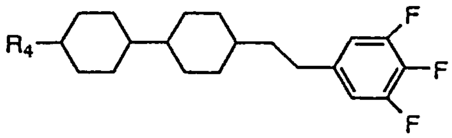

(3-18)

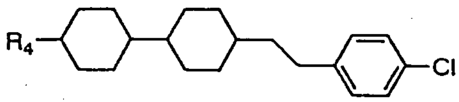

(3-19)

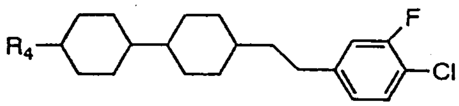

(3-20)

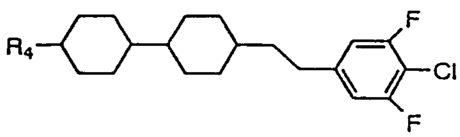

(3-21)

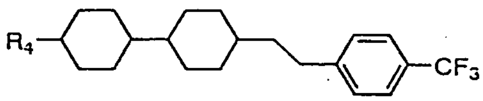

(3-22)

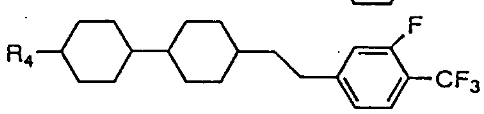

(3-23)

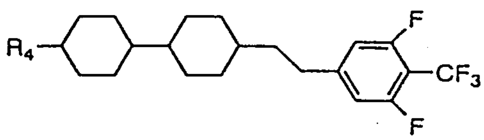

(3-24)

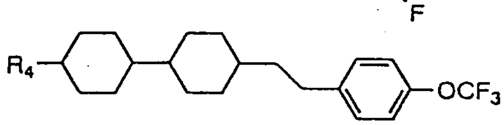

(3-25)

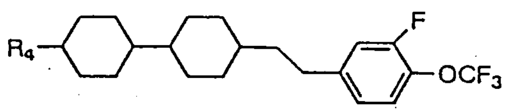

(3-26)

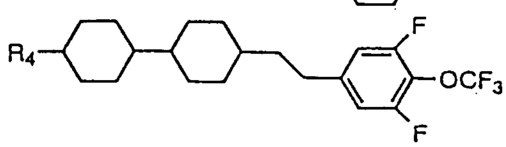

(3-27)

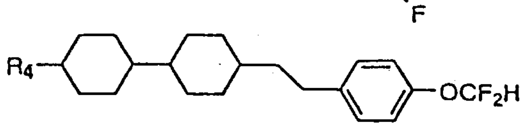

(3-28)

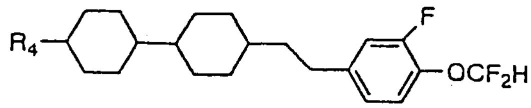
(3-29)

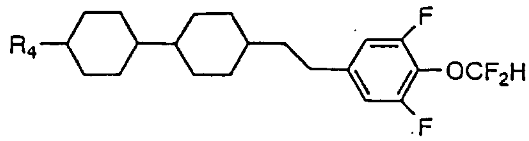
(3-30)

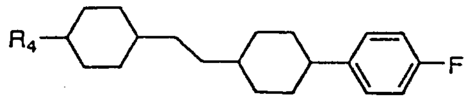
(3-31)

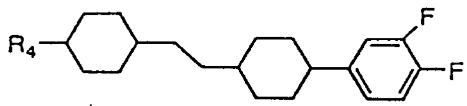
(3-32)

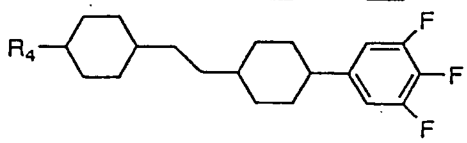
(3-33)

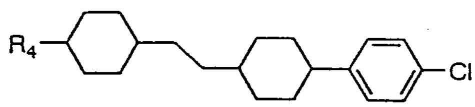
(3-34)

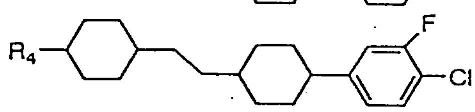
(3-35)

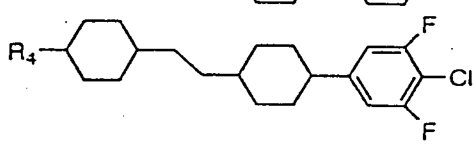
(3-36)

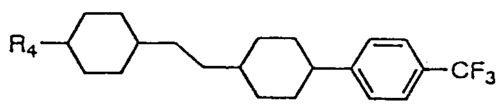
(3-37)

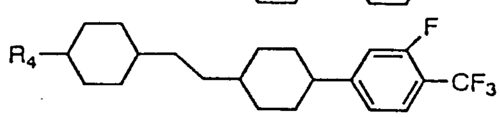
(3-38)

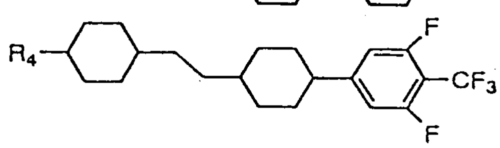
(3-39)

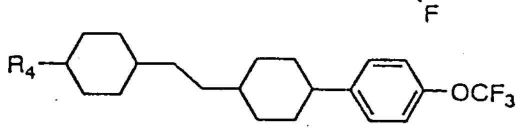
(3-40)

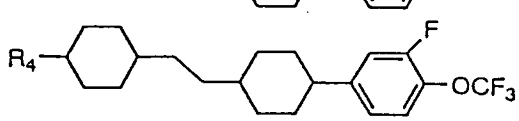
(3-41)

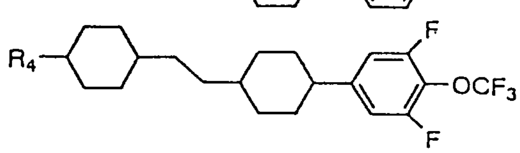
(3-42)

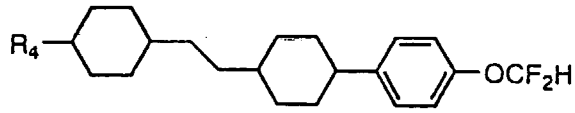

(3-43)

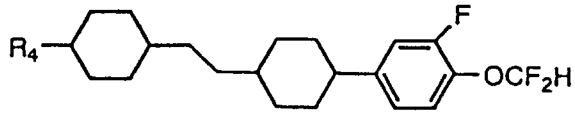

(3-44)

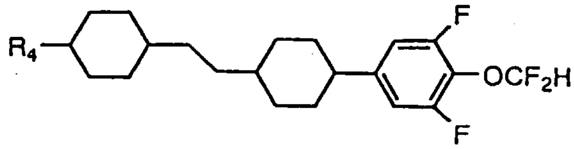

(3-45)

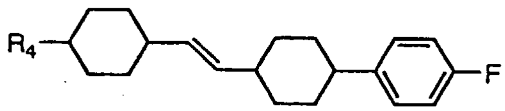

(3-46)

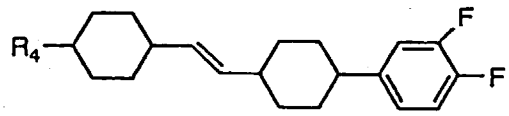

(3-47)

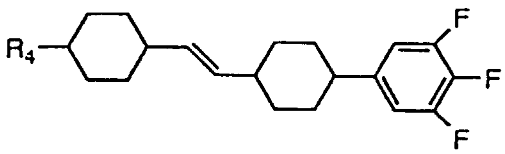

(3-48)

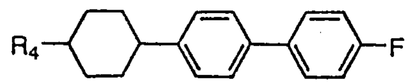

(4-1)

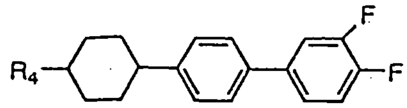

(4-2)

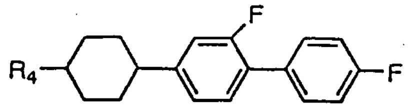

(4-3)

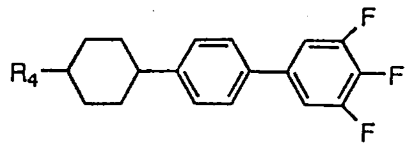

(4-4)

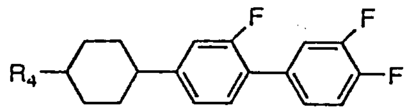

(4-5)

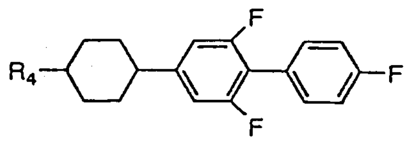

(4-6)

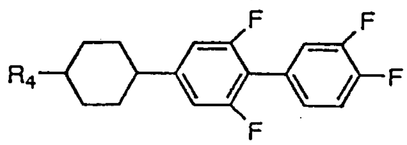

(4-7)

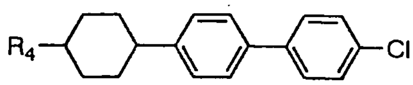

(4-8)

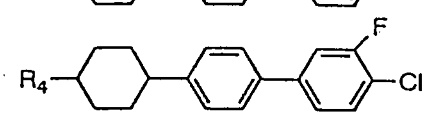

(4-9)

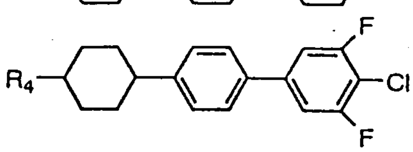

(4-10)

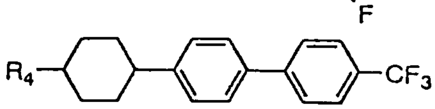

(4-11)

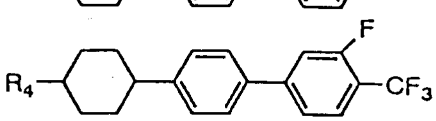

(4-12)

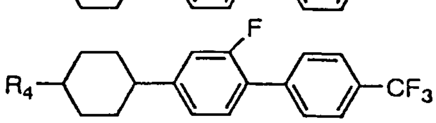

(4-13)

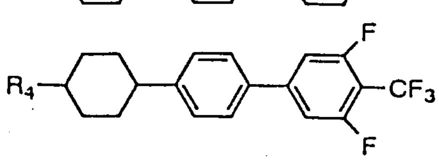

(4-14)

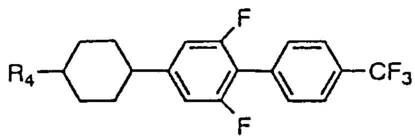
(4-15)

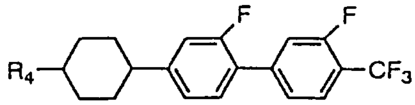
(4-16)

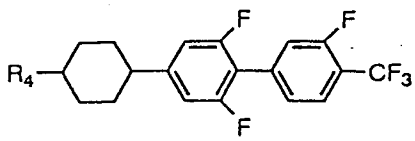
(4-17)

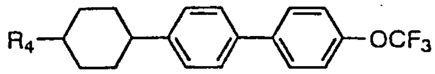
(4-18)

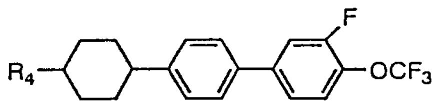
(4-19)

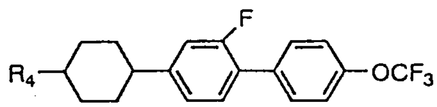
(4-20)

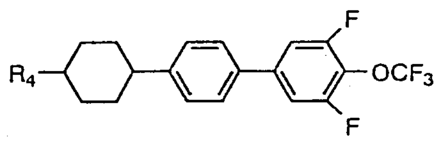
(4-21)

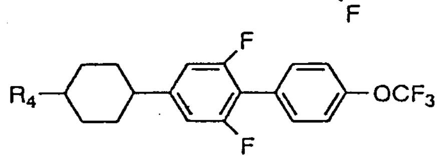
(4-22)

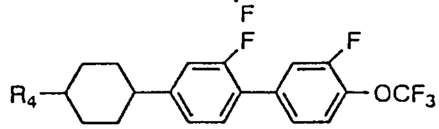
(4-23)

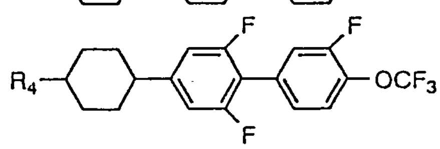
(4-24)

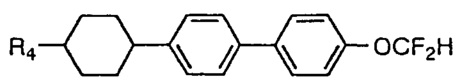
(4-25)

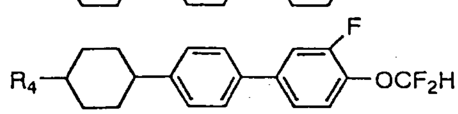
(4-26)

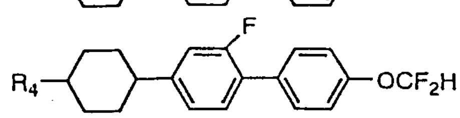
(4-27)

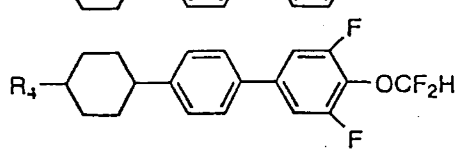
(4-28)

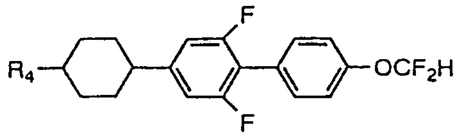
(4-29)

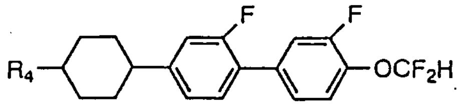
(4-30)

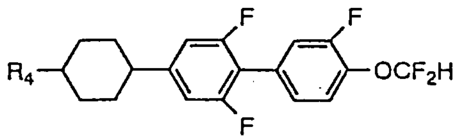
(4-31)

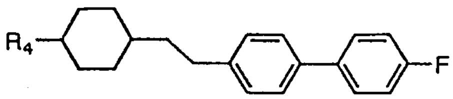
(4-32)

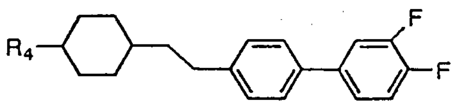
(4-33)

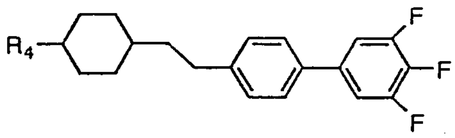
(4-34)

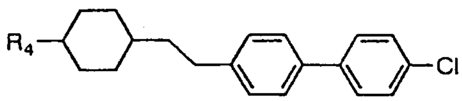
(4-35)

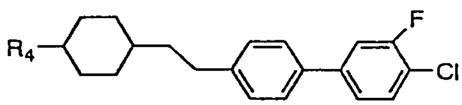
(4-36)

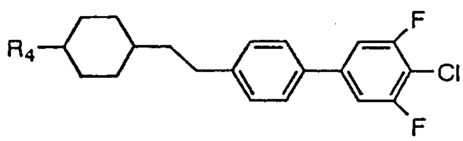
(4-37)

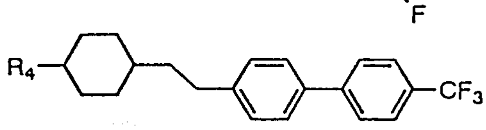
(4-38)

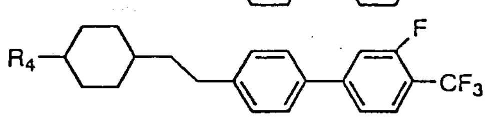
(4-39)

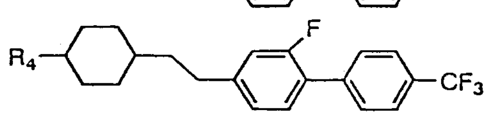
(4-40)

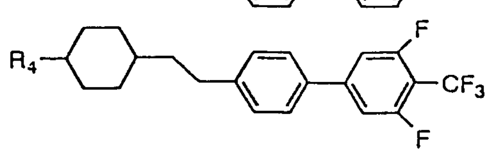
(4-41)

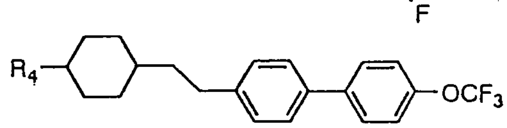
(4-42)

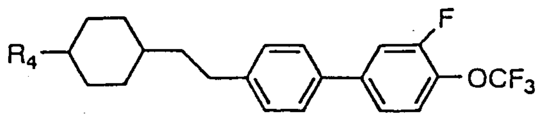

(4-43)

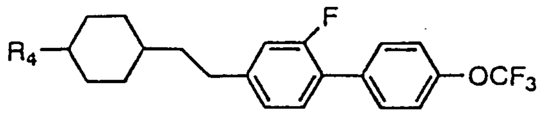

(4-44)

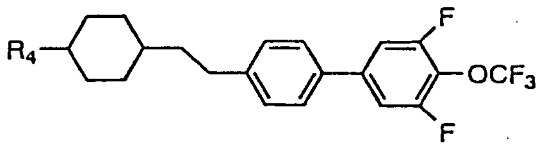

(4-45)

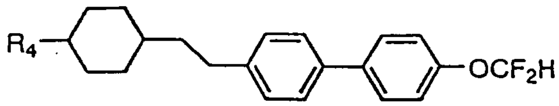

(4-46)

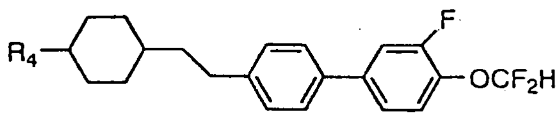

(4-47)

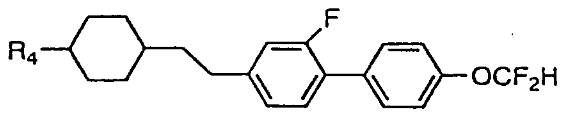

(4-48)

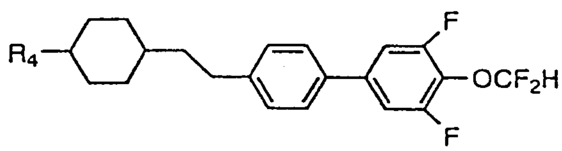

(4-49)

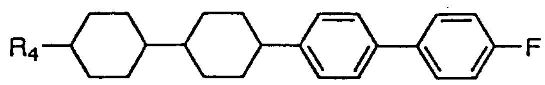

(4-50)

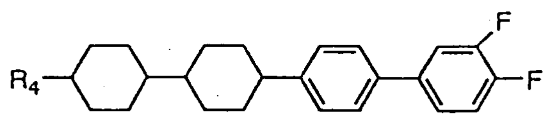

(4-51)

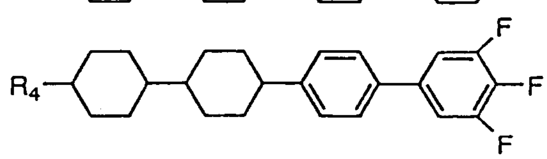

(4-52)

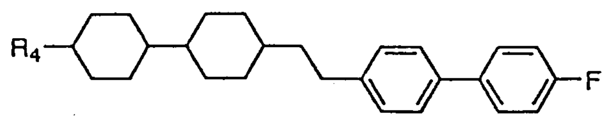

(4-53)

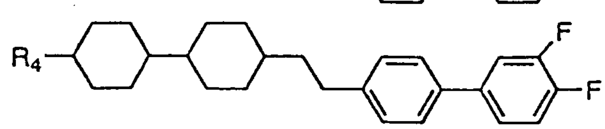

(4-54)

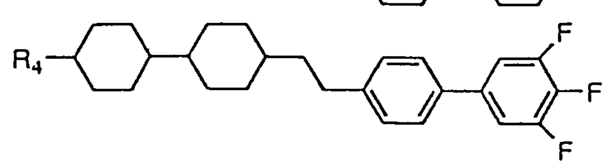

(4-55)

wherein $R_4$ represents an alkyl group or alkoxy group.

Since the compounds expressed by one of the general formulas (2) to (4) have a positive dielectric anisotropy and are considerably excellent in thermal stability and chemical stability, they are useful when liquid crystal compositions for TN display mode are produced. Also, the compounds are useful particularly when liquid crystal compositions for TFT

(AM-LCD) display mode are produced since a high voltage holding ratio and large specific resistance are required of such compositions.

When liquid crystal compositions for TFT are produced, at least one compound expressed by one of the general formulas (2) to (4) can be used in the range of 1 to 99 % by weight based on the total amount of the liquid crystal composition, and its preferable amount is in the range of 10 to 97 % by weight and more desirable amount is in the range of 40 to 95 % by weight. At that time, the compound expressed by one of the general formulas (5) to (9) can be added.

As preferable examples of the compound expressed by one of the general formulas (5) to (7) and used in the liquid crystal compositions of the present invention, the following compounds can be mentioned:

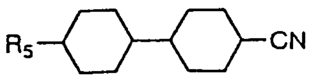 (5-1)

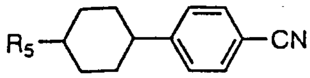 (5-2)

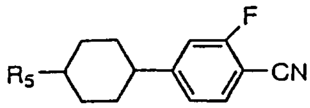 (5-3)

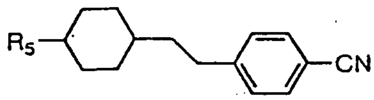 (5-4)

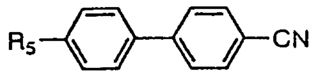 (5-5)

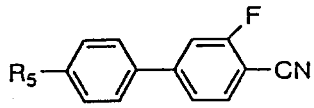 (5-6)

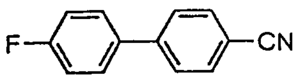 (5-7)

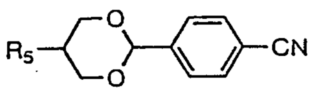 (5-8)

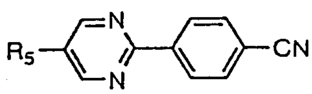 (5-9)

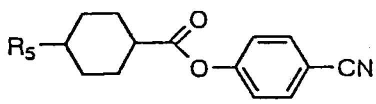 (5-10)

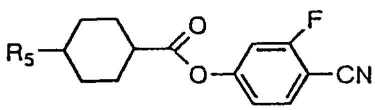 (5-11)

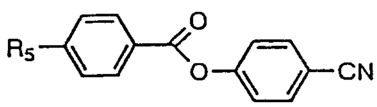 (5-12)

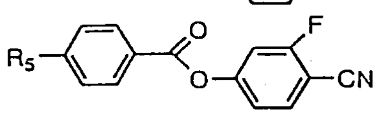 (5-13)

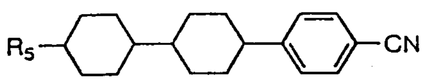 (5-14)

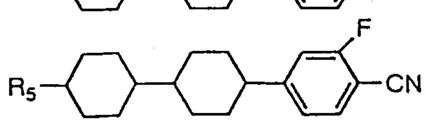 (5-15)

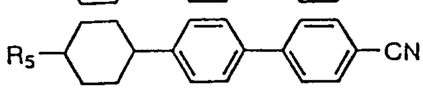 (5-16)

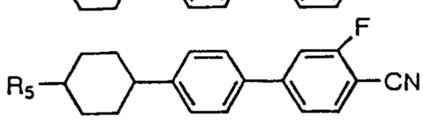 (5-17)

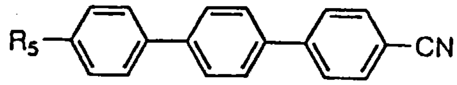

(5-18)

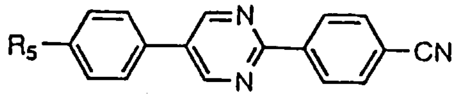

(5-19)

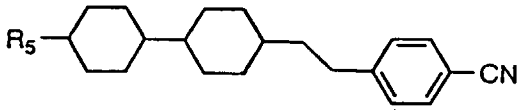

(5-20)

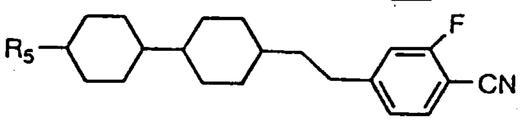

(5-21)

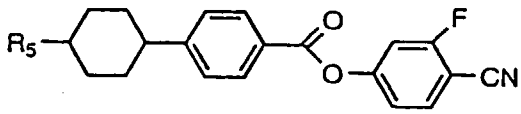

(5-22)

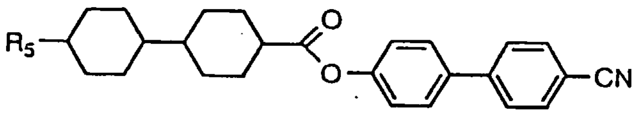

(5-23)

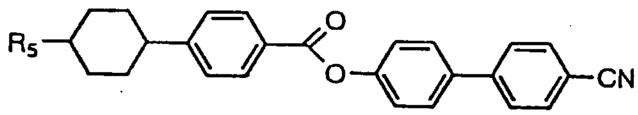

(5-24)

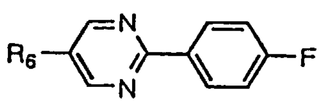

(6-1)

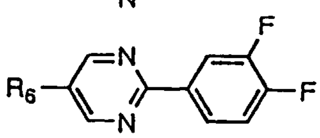

(6-2)

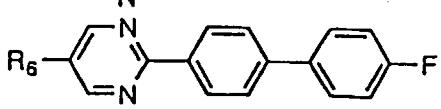

(6-3)

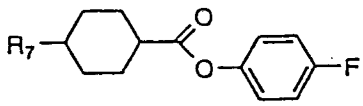

(7-1)

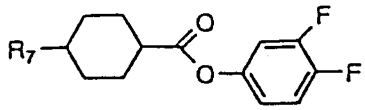

(7-2)

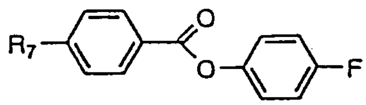

(7-3)

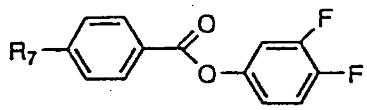

(7-4)

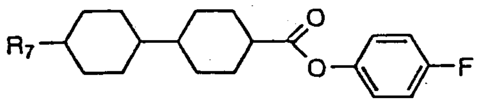

(7-5)

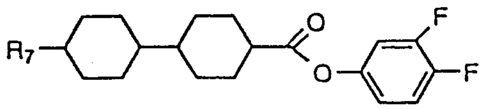

(7-6)

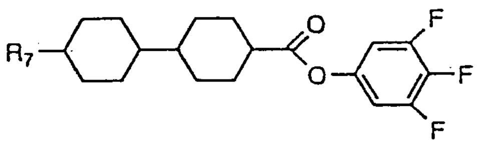

(7-7)

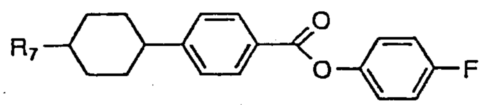

(7-8)

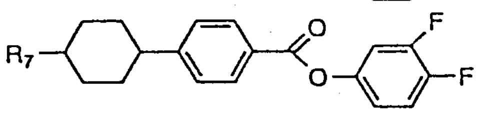

(7-9)

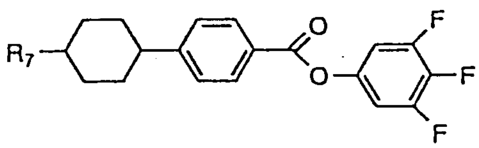

(7-10)

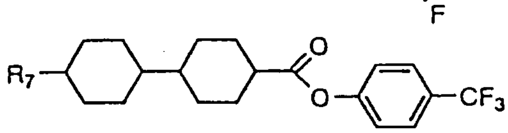

(7-11)

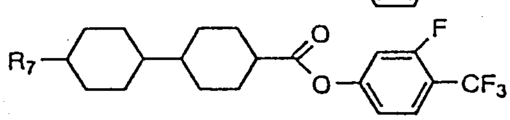

(7-12)

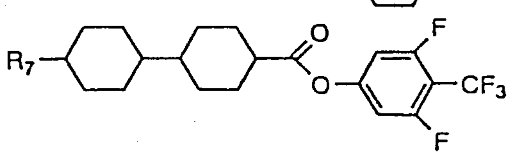

(7-13)

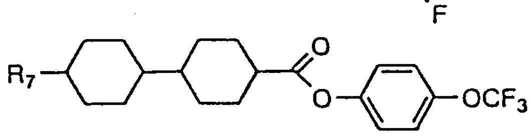

(7-14)

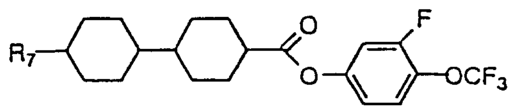
(7-15)

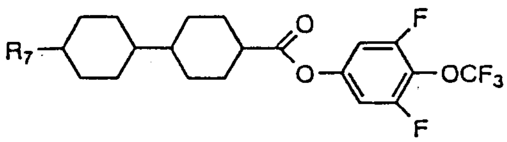
(7-16)

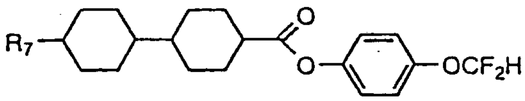
(7-17)

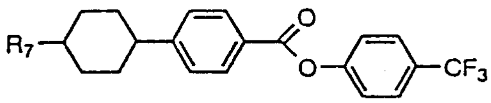
(7-18)

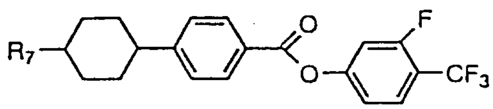
(7-19)

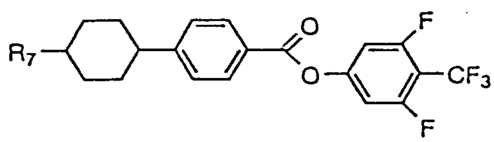
(7-20)

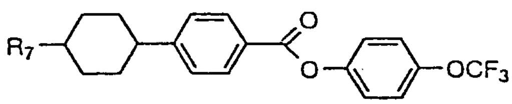
(7-21)

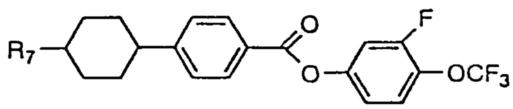
(7-22)

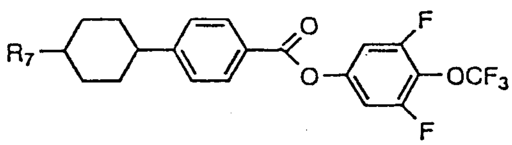
(7-23)

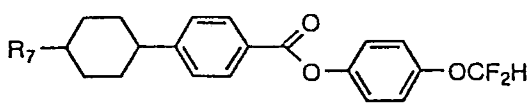
(7-24)

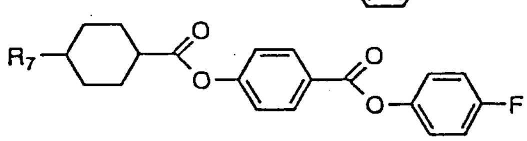
(7-25)

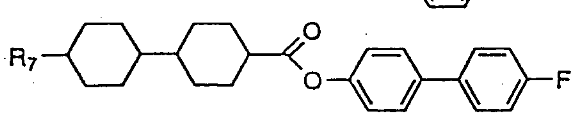
(7-26)

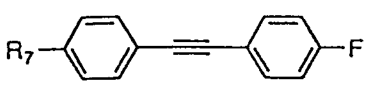
(7-27)

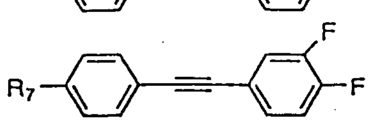
(7-28)

wherein $R_5$, $R_6$, and $R_7$ represent an alkyl group or alkenyl group.

Compounds expressed by one of the general formulas (5) to (7) have a large positive dielectric anisotropy, and are used for the purpose of lowering threshold voltage of liquid crystal compositions. Also, the compounds are used for the

purpose of adjusting viscosity, adjusting optical anisotropy, and widening nematic range such as raising clearing point. Besides, the compounds are used for the purpose of improving the steepness of electrooptical characteristics around threshold value.

As preferable examples of the compounds expressed by the general formula (8) or (9) and used in the liquid crystal compositions of the present invention, the following compounds can be mentioned:

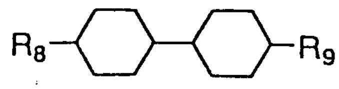

(8-1)

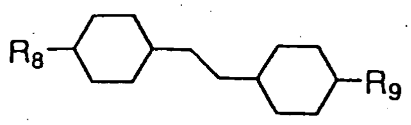

(8-2)

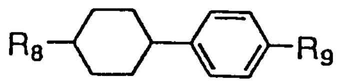

(8-3)

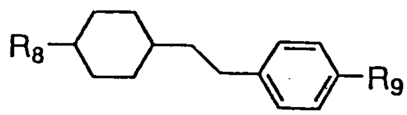

(8-4)

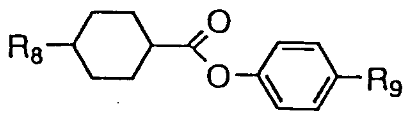

(8-5)

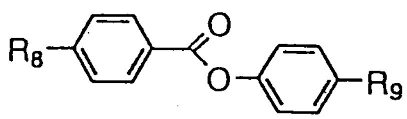

(8-6)

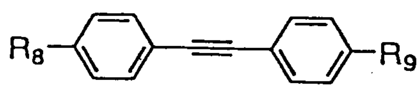

(8-7)

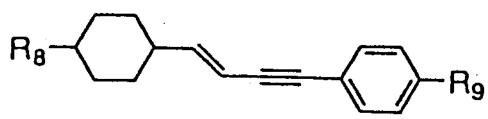

(8-8)

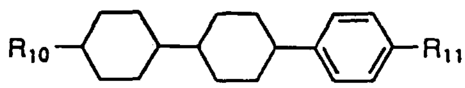
(9-1)

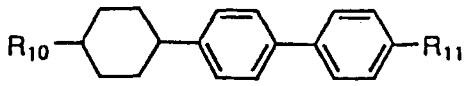
(9-2)

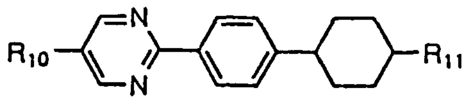
(9-3)

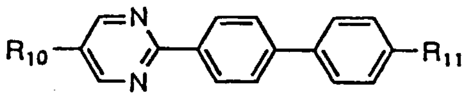
(9-4)

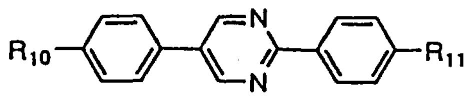
(9-5)

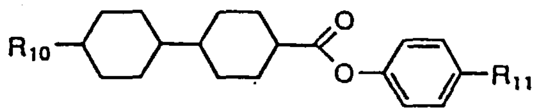
(9-6)

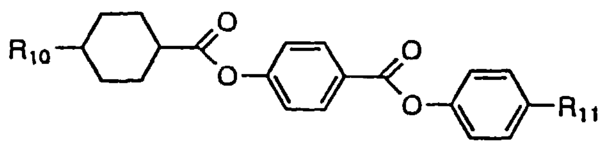
(9-7)

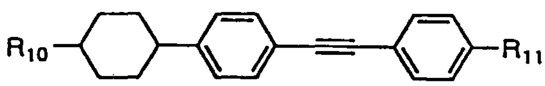
(9-8)

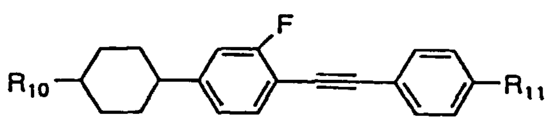
(9-9)

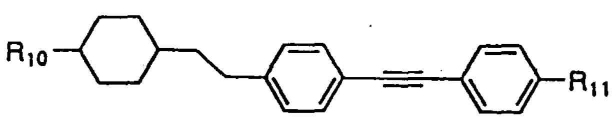
(9-10)

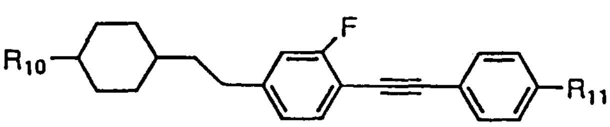
(9-11)

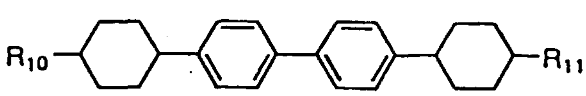
(9-12)

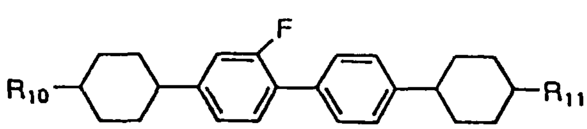
(9-13)

wherein $R_8$, $R_9$, $R_{10}$, and $R_{11}$ represent an alkyl group or alkenyl group.

Compounds expressed by the general formula (8) or (9) have a negative or slightly positive dielectric anisotropy. Compounds expressed by the general formula (8) are used mainly for the purpose of reducing viscosity and/or adjusting optical anisotropy of liquid crystal compositions. Compounds expressed by the general formula (9) are used for the purpose of widening nematic range such as raising clearing point and/or adjusting optical anisotropy of liquid crystal compositions.

When liquid crystal compositions for TN display mode or STN display mode are produced, at least one compound expressed by one of the general formulas (5) to (9) can be used in the range of 1 to 99 % by weight based on the total

amount of liquid crystal composition, and its preferable amount is in the range of 10 to 97 % by weight and more desirable amount is in the range of 40 to 95 % by weight. At that time, the compound expressed by one of the general formulas (2) to (4) can be added.

Since the compounds expressed by the general formula (1) have a low viscosity, response speed of liquid crystal display devices utilizing the compound can be improved.

Liquid crystal compositions used according to the present invention can be produced by methods which are conventional by themselves. Generally, a method is adopted wherein various components are solved each other at a high temperature. Further, liquid crystal materials of the present invention can be improved according to intended uses by adding suitable additives, and optimized. Such additives are well known in the art and are described in detail in the literature. Usually, a chiral dopant or the like is added to induce a helical structure of liquid crystals to adjust a required twisting angle, and to avoid reverse-twist.

Liquid crystal compositions used according to the present invention can also be used as liquid crystal compositions for guest-host (GH) mode by adding a dichroic dye such as merocyanine type, styryl type, azo type, azomethine type, azoxy type, quinophthalone type, anthraquinone type, or tetrazine type. Alternatively, they can also be used as liquid crystal compositions for polymer dispersed liquid crystal display devices (PDLCDs) represented by NCAP which is prepared by the microencapsulation of a nematic liquid crystal or represented by polymer net work liquid crystal display devices (PNLCDs) which are prepared by forming a polymer of three-dimensional reticulated structure in a liquid crystal. Further, the liquid crystal compositions of the present invention can be used as ones for electrically controlled birefringence (ECB) mode or dynamic scattering (DS) mode.

As the nematic liquid crystal compositions produced by such methods and comprising the liquid crystalline compound of the present invention, the following examples of composition can be mentioned. In the followings, compounds in the compositions are indicated by abbreviation according to the rules for abbreviating shown in Table 1.

Table 1

$R_{15}$-(-$A_1$)-)-$Z_{15}$- ...... -$Z_n$-(-$A_n$-)-$X_3$

| 1) Left side terminal group $R_{15}$- | Symbol |
|---|---|
| $C_sH_{2s+1}$- | s- |
| $C_sH_{2s+1}O$- | sO- |
| $C_sH_{2s+1}OC_tH_{2t}$- | sOt- |
| $CH_2$=$CHC_sH_{2s}$- | Vs- |
| $C_sH_{2s+1}CH$=$CHC_tH_{2t}$- | sVt- |
| $C_sH_{2s+1}CH$=$CHC_tH_{2t}CH$=$CHC_kH_{2k}$- | sVtVk- |

| 3) Bonding group -$Z_{15}$-, -$Z_n$- | Symbol |
|---|---|
| -$CH_2CH_2$- | 2 |
| -COO- | E |
| -C≡C- | T |
| -CH=CH- | V |
| -$CF_2O$- | CF2O |

| 2) Ring structure -(-A1-)-, -(-An-)- | Symbol |
|---|---|
| 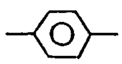 | B |
| 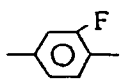 | B(F) |
| 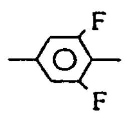 | B(F,F) |
| 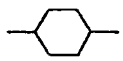 | H |
| 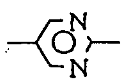 | Py |
| 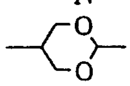 | D |
| 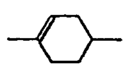 | Ch |

| 4) Right side terminal group -X3 | Symbol |
|---|---|
| -F | -F |
| -Cl | -CL |
| -CN | -C |
| -$CF_3$ | -CF3 |
| -$OCF_3$ | -OCF3 |
| -OCF2H | -OCF2H |
| -$C_qH_{2q+1}$ | -q |
| -$OC_qH_{2q+1}$ | -Oq |
| -$COOCH_3$ | -EMe |

| Composition Example 1 | | |
|---|---|---|
| V2-HB-1O3 | Compound No. 1 | 4.0 % |
| V-HHB-1O1V | Compound No. 637 | 5.0 % |
| V1-HHB-1O2 | Compound No. 638 | 5.0 % |
| 7-HB-(F)-F | | 10.0 % |
| 2-HHB(F)-F | | 10.0 % |
| 3-HHB(F)-F | | 10.0 % |

(continued)

| Composition Example 1 | | |
|---|---|---|
| 5-HHB(F)-F | | 10.0 % |
| 2-H2HB(F)-F | | 6.4 % |
| 3-H2HB(F)-F | | 3.2 % |
| 5-H2HB(F)-F | | 6.4 % |
| 2-HBB(F)-F | | 7.5 % |
| 3-HBB(F)-F | | 7.5 % |
| 5-HBB(F)-F | | 15.0 % |

| Composition Example 2 | | |
|---|---|---|
| V-HHB-1O3 | Compound No. 636 | 3.0 % |
| V-HBB-1O3 | Compound No. 287 | 5.0 % |
| 1V-HHBB-1O3 | Compound No. 942 | 2.0 % |
| 1V2-H2BB-1O2 | Compound No. 492 | 3.0 % |
| 7-HB(F,F)-F | | 5.0 % |
| 3-HHB(F,F)-F | | 4.0 % |
| 3-H2HB(F,F)-F | | 6.0 % |
| 3-HBB(F,F)-F | | 10.0 % |
| 5-HBB(F,F)-F | | 15.0 % |
| 3-H2BB(F,F)-F | | 5.0 % |
| 4-H2BB(F,F)-F | | 5.0 % |
| 5-H2BB(F,F)-F | | 5.0 % |
| 3-HBEB(F,F)-F | | 3.0 % |
| 4-HBEB(F,F)-F | | 3.0 % |
| 5-HBEB(F,F)-F | | 3.0 % |
| 3-HHEB(F,F)-F | | 11.0 % |
| 4-HHEB(F,F)-F | | 5.0 % |
| 5-HHEB(F,F)-F | | 5.0 % |
| 3-HHBB(F,F) | | 2.0 % |

| Composition Example 3 | | |
|---|---|---|
| V-H2HB-1O1V | Compound No. 759 | 5.0 % |
| 1V2-H2HB-1O3 | Compound No. 493 | 4.0 % |
| 7-HB(F,F)-F | | 4.0 % |

(continued)

| Composition Example 3 | | |
|---|---|---|
| 7-HB(F)-F | | 4.0 % |
| 5-H2B(F)-F | | 3.0 % |
| 2-HHB(F)-F | | 13.4 % |
| 3-HHB(F)-F | | 13.3 % |
| 5-HHB(F)-F | | 13.3 % |
| 2-H2HB(F)-F | | 2.0 % |
| 3-H2HB(F)-F | | 1.0 % |
| 5-H2HB(F)-F | | 2.0 % |
| 3-H2HB(F,F)-F | | 4.0 % |
| 4-H2HB(F,F)-F | | 4.0 % |
| 5-H2HB(F,F)-F | | 4.0 % |
| 3-HHB(F,F)-F | | 8.0 % |
| 3-HH2B(F,F)-F | | 8.0 % |
| 5-HH2B(F,F)-F | | 7.0 % |

| Composition Example 4 | | |
|---|---|---|
| 3-HBB-1O1V | | 7.0 % |
| V-HBB-1O3 | Compound No. 287 | 5.0 % |
| 3-HB-CL | | 7.0 % |
| 7-HB(F,F)-F | | 10.0 % |
| 2-HBB(F)-F | | 6.0 % |
| 3-HBB(F)-F | | 6.0 % |
| 5-HBB(F)-F | | 12.0 % |
| 2-HHB-CL | | 5.0 % |
| 4-HHB-CL | | 10.0 % |
| 5-HHB-CL | | 5.0 % |
| 3-HBB(F,F)-F | | 9.0 % |
| 5-HBB(F,F)-F | | 8.0 % |
| 3-HB(F)TB-2 | | 2.0 % |
| 3-HB(F)TB-3 | | 3.0 % |
| 3-HB(F)VB-2 | | 2.0 % |
| 3-HB(F)VB-3 | | 3.0 % |

| Composition Example 5 | | |
|---|---|---|
| V-HHB-1O1V | Compound No. 759 | 10.0 % |
| V-HHB-1O3 | Compound No. 636 | 10.0 % |
| V2-HHB-1O1V | Compound No. 789 | 5.0 % |
| 1V2-HBBB-1O1 | Compound No. 906 | 2.0 % |
| 7-HB(F)-F | | 4.0 % |
| 2-HHB(F)-F | | 5.0 % |
| 3-HHB(F)-F | | 5.0 % |
| 5-HHB(F)-F | | 5.0 % |
| 2-H2HB(F)-F | | 7.0 % |
| 3-H2HB(F)-F | | 3.0 % |
| 5-H2HB(F)-F | | 7.0 % |
| 2-HBB(F)-F | | 3.0 % |
| 3-HBB(F)-F | | 3.0 % |
| 5-HBB(F)-F | | 6.0 % |
| 2-HBB-F | | 4.0 % |
| 3-HBB-F | | 4.0 % |
| 3-HHB-F | | 4.0 % |
| 3-HB-O2 | | 6.0 % |
| 3-HHB-1 | | 5.0 % |
| 1O1-HBBH-3 | | 2.0 % |

| Composition Example 6 | | |
|---|---|---|
| V2-HB-1O3 | Compound No. 1 | 3.0 % |
| 3-HB-1O1V | | 2.0 % |
| 1V2-BEB(F,F)-C | | 12.0 % |
| 3O1-BEB(F)-C | | 12.0 % |
| 2-HB-C | | 12.0 % |
| 3-HB-C | | 12.0 % |
| 1O1-HB-C | | 7.0 % |
| 2-HHB-C | | 4.0 % |
| 3-HHB-C | | 4.0 % |
| 4-HHB-C | | 3.0 % |
| 5-HHB-C | | 3.0 % |
| 3-HHB(F)-C | | 3.0 % |

(continued)

| Composition Example 6 | | |
|---|---|---|
| 3-HHB-1 | | 7.0 % |
| 3-HHB-O1 | | 4.0 % |
| 3-HB-O2 | | 2.0 % |
| 3-H2BTB-2 | | 4.0 % |
| 3-H2BTB-3 | | 3.0 % |
| 3-H2BTB-4 | | 3.0 % |

| Composition Example 7 | | |
|---|---|---|
| 2V-HB-1O3 | Compound No. 1 | 5.0 % |
| 3-H2H2B-1O1 | | 2.0 % |
| 3-H2H2B-1O3 | | 2.0 % |
| V-HHB-1O1V | Compound No. 759 | 5.0 % |
| V-HHB-1O2 | Compound No. 638 | 5.0 % |
| 2-HB(F)-C | | 15.0 % |
| 3-HB(F)-C | | 15.0 % |
| 3-HHB-F | | 4.0 % |
| 3-HB-O2 | | 5.0 % |
| 3-HH-4 | | 6.0 % |
| 1O1-HH-5 | | 4.0 % |
| 2-BTB-O1 | | 7.0 % |
| 4-BTB-4 | | 3.0 % |
| 3-HHB-1 | | 8.0 % |
| 3-HHB-3 | | 6.0 % |
| 3-HHB-O1 | | 4.0 % |
| 3-H2BTB-2 | | 2.0 % |
| 3-H2BTB-3 | | 2.0 % |

| Composition Example 8 | | |
|---|---|---|
| V2-HB-1O3 | Compound No. 1 | 10.0 % |
| 1V2-HB-1O1V | Compound No. 61 | 5.0 % |
| 3-HB-1O1 | | 5.0 % |
| 5-BB-C | | 3.0 % |

(continued)

| Composition Example 8 | | |
|---|---|---|
| 3O-BB-C | | 2.0 % |
| 2O2O-BB-C | | 2.0 % |
| 3-HHB-F | | 4.0 % |
| 3-HB-O2 | | 4.0 % |
| 3-HB-O4 | | 3.0 % |
| 3-PyB-4 | | 3.1 % |
| 4-PyB-4 | | 3.1 % |
| 6-PyB-4 | | 3.2 % |
| 3-PyB-5 | | 3.2 % |
| 4-PyB-5 | | 3.2 % |
| 6-PyB-5 | | 3.2 % |
| 6-PyB-O5 | | 4.0 % |
| 6-PyB-O6 | | 4.0 % |
| 6-PyB-O7 | | 4.0 % |
| 6-PyB-O8 | | 4.0 % |
| 2-HHB-1 | | 4.0 % |
| 3-HHB-1 | | 8.0 % |
| 3-HHB-3 | | 10.0 % |
| 3-HHB-O1 | | 5.0 % |

| Composition Example 9 | | |
|---|---|---|
| V-HHB-1O3 | Compound No. 636 | 5.0 % |
| 3-HHB-1O3 | | 5.0 % |
| 3-DB-C | | 10.0 % |
| 4-DB-C | | 12.0 % |
| 5-DB-C | | 8.0 % |
| 2-BEB-C | | 4.0 % |
| 3-BEB-C | | 3.0 % |
| 4-BEB-C | | 3.0 % |
| 2-PyB-F | | 2.0 % |
| 5-PyB(F)-F | | 8.0 % |
| 2-PyB-2 | | 1.4 % |
| 3-PyB-2 | | 1.3 % |
| 4-PyB-2 | | 1.3 % |
| 3-HEB-O4 | | 5.0 % |
| 4-HEB-O2 | | 3.7 % |

(continued)

| Composition Example 9 | | |
| --- | --- | --- |
| 3-HEB-O2 | | 3.1 % |
| 1O-BEB-2 | | 2.5 % |
| 5-HEB-1 | | 3.7 % |
| 4-HEB-4 | | 5.0 % |
| 3-HHB-3 | | 3.0 % |
| 3-HHB-O1 | | 4.0 % |
| 2-PyBH-3 | | 3.0 % |
| 3-PyBB-2 | | 3.0 % |

| Composition Example 10 | | |
| --- | --- | --- |
| V2-HB-1O3 | Compound No. 1 | 3.0 % |
| V-HBB-1O3 | Compound No. 287 | 3.0 5 |
| 3-PyB(F)-F | | 16.0 % |
| 3-PyB-4 | | 2.0 % |
| 4-PyB-4 | | 2.0 % |
| 6-PyB-4 | | 2.0 % |
| 3-PyB-5 | | 2.0 % |
| 4-PyB-5 | | 2.0 % |
| 6-PyB-5 | | 2.0 % |
| 4-PyB-O2 | | 7.0 % |
| 6-PyB-O2 | | 8.0 % |
| 2-PyBB-F | | 8.0 % |
| 3-PyBB-F | | 8.0 % |
| 4-PyBB-F | | 8.0 % |
| 2-PyBH-2 | | 4.0 % |
| 3-PyBH-2 | | 4.0 % |
| 4-PyBH-2 | | 4.0 % |
| 2-PyBH-3 | | 5.0 % |
| 3-PyBH-3 | | 5.0 % |
| 4-PyBH-3 | | 5.0 % |

| Composition Example 11 | | |
|---|---|---|
| V-HBBB-1O3 | Compound No. 863 | 2.0 % |
| 2V1-HHBB-1O1V | Compound No. 945 | 2.0 % |
| V-HHB-1O3 | Compound No. 636 | 3.0 % |
| 3-DB-C | | 10.0 % |
| 4-DB-C | | 10.0 % |
| 2-BEB-C | | 12.0 % |
| 3-BEB-C | | 4.0 % |
| 3-HHEBB-C | | 3.0 % |
| 5-HBEBB-C | | 2.0 % |
| 3-PyB(F)-F | | 6.0 % |
| 3-HEB-O4 | | 11.9 % |
| 4-HEB-O2 | | 8.9 % |
| 5-HEB-O1 | | 8.9 % |
| 3-HEB-O2 | | 7.4 % |
| 5-HEB-O2 | | 5.9 % |
| 3-HHB-1 | | 3.0 % |

| Composition Example 12 | | |
|---|---|---|
| V2-HB-1O3 | Compound No. 1 | 5.0 % |
| V-HHB-1O1V | Compound No. 759 | 5.0 % |
| V-HBB-1O3 | Compound No. 287 | 3.0 % |
| 5-HB-F | | 9.0 % |
| 6-HB-F | | 7.0 % |
| 7-HB-F | | 7.0 % |
| 5-HB-3 | | 3.0 % |
| 3-HB-O1 | | 2.0 % |
| 3-HHB-OCF3 | | 5.0 % |
| 4-HHB-OCF3 | | 5.0 % |
| 5-HHB-OCF3 | | 7.0 % |
| 3-HH2B-OCF3 | | 2.0 % |
| 5-HH2B-OCF3 | | 3.0 % |
| 3-HH2B-F | | 3.0 % |
| 5-HH2B-F | | 3.0 % |
| 3-HBB(F)-F | | 3.0 % |

(continued)

| Composition Example 12 | | |
|---|---|---|
| 5-HBB(F)-F | | 5.0 % |
| 3-HH2B(F)-F | | 7.0 % |
| 5-HH2B(F)-F | | 9.0 % |
| 3-HB(F)BH-3 | | 3.0 % |
| 5-HB(F)BH-3 | | 2.0 % |
| 5-HB(F)BH-5 | | 2.0 % |

| Composition Example 13 | | |
|---|---|---|
| V-HHB-1O3 | Compound No. 636 | 6.0 % |
| V2-H2H2B-1O2 | Compound No. 740 | 6.0 % |
| 5-HB-F | | 7.0 % |
| 3-HH-O1 | | 7.0 % |
| 3-HH-O3 | | 6.0 % |
| 5-HH-O1 | | 5.0 % |
| 3-HHB-OCHF2 | | 3.0 % |
| 5-HHB-OCHF2 | | 4.0 % |
| 3-HHB(F,F)-OCHF2 | | 10.0 % |
| 5-HHB(F,F)-OCHF2 | | 11.0 % |
| 2-HHB-OCF3 | | 3.0 % |
| 3-HHB-OCF3 | | 4.0 % |
| 4-HHB-OCF3 | | 6.0 % |
| 5-HHB-OCF3 | | 6.0 % |
| 3-HH2B(F)-F | | 5.0 % |
| 5-HH2B(F)-F | | 5.0 % |
| 3-HHEB(F)-F | | 6.0 % |

| Composition Example 14 | | |
|---|---|---|
| 1V2-H2BB-1O3 | Compound No. 493 | 2.0 % |
| V2-HBBB-1O1V | Compound No. 945 | 2.0 % |
| V-HBB-1O3 | Compound No. 287 | 3.0 % |
| V-HB-C | | 10.0 % |
| 1V-HB-C | | 5.0 % |

(continued)

| Composition Example 14 | | |
|---|---|---|
| 3-BB-C | | 5.0 % |
| 5-BB-C | | 5.0 % |
| 2-HB(F)-C | | 5.0 % |
| 4-BB-3 | | 3.0 % |
| 3-H2B-O2 | | 5.0 % |
| 5-H2B-O2 | | 8.0 % |
| 3-BEB-C | | 5.0 % |
| 5-HEB-O1 | | 8.0 % |
| 5-HEB-O3 | | 8.0 % |
| 5-BBB-C | | 5.0 % |
| 4-BPyB-C | | 4.0 % |
| 4-BPyB-5 | | 4.0 % |
| 5-HB2B-4 | | 3.0 % |
| 5-HBB2B-3 | | 3.0 % |
| 1V-HH-1O1 | | 5.0 % |
| 1V2-HBB-3 | | 2.0 % |

| Composition Example 15 | | |
|---|---|---|
| V-HBB-1O3 | Compound No. 287 | 5.0 % |
| 1V2-HB-1O1V | Compound No. 94 | 4.0 % |
| 5-HEB-F | | 3.0 % |
| 7-HEB-F | | 3.0 % |
| 4-HEB(F)-F | | 5.0 % |
| 5-HEB(F)-F | | 5.0 % |
| 2-BEB(F)-C | | 5.0 % |
| 3-BEB(F)-C | | 5.0 % |
| 4-BEB(F)-C | | 8.0 % |
| 5-BEB(F)-C | | 8.0 % |
| 1O3-HB(F)-C | | 6.0 % |
| 3-HHEB-F | | 5.0 % |
| 5-HHEB(F)-F | | 5.0 % |
| 2-HBEB(F)-C | | 4.0 % |
| 3-HBEB(F)-C | | 4.0 % |
| 4-HBEB(F)-C | | 4.0 % |
| 5-HBEB(F)-C | | 4.0 % |

(continued)

| Composition Example 15 | | |
|---|---|---|
| 3-HBEB-F | | 4.0 % |
| 3-HBTB-2 | | 5.0 % |
| V2-HH-3 | | 4.0 % |
| V2-HHB-1 | | 4.0 % |

Compounds of the present invention expressed by the general formula (1) can readily be produced by freely using ordinary chemical procedures of organic synthesis. The compounds can readily be synthesized by selecting proper procedures described, for example, in Organic Synthesis, Organic Reactions, or Jikken Kagaku Kouza (Course of Chemical Experiment) and using the procedures in combination.

First, right side terminal group $R_3$ of the compounds expressed by the general formula (1) can be introduced by reacting compound (16) obtained according to a method described in Japanese Patent Application No. Hei 7-026029 with phosphonium salt (17) obtained according to a method described in G. Wittig et al., Org. Synth. Col. V, 249 (1973).

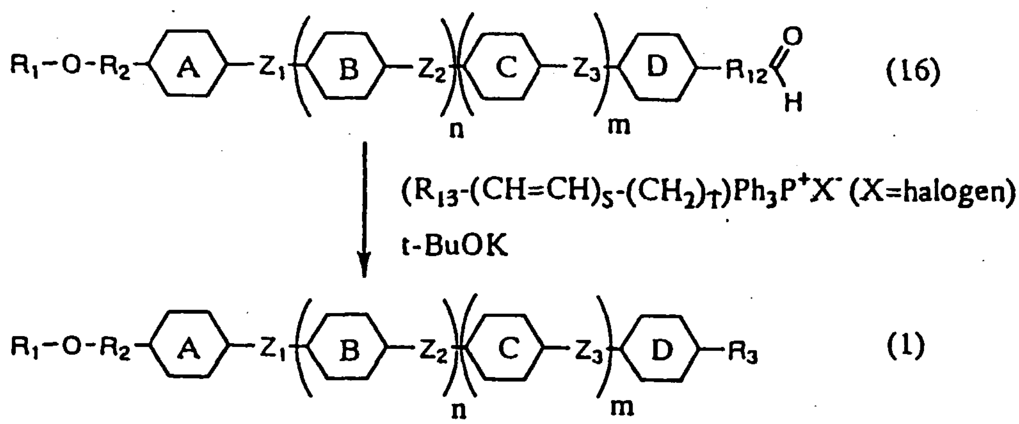


(16)

(17)

(1)

wherein $R_{12}$ represents single bond or an alkylene group having 1 to 14 carbon atoms; $R_{13}$ represents hydrogen atom or an alkyl group having 1 to 14 carbon atoms; and S and T are independently an integer of 0 to 14 provided that $S + T \neq 0$.

Left side terminal group $R_1OR_2$ in the general formula (1) is introduced by the method as follows:

First, carboxylic acid (18) which can be produced by a method described in M. E. Neubert et al., Mol. Cryst. Liq. Cryst., 76, 48 (1981) is subjected to carbon homologation reaction to convert the carboxylic acid into aldehyde (19), and then converted into alcohol derivative (20) by using a reducing agent such as sodium boron hydride. Subsequently, the alcohol derivative is reacted with both sodium hydride and $R_1X$ to form an objective compound expressed by the general formula (1).

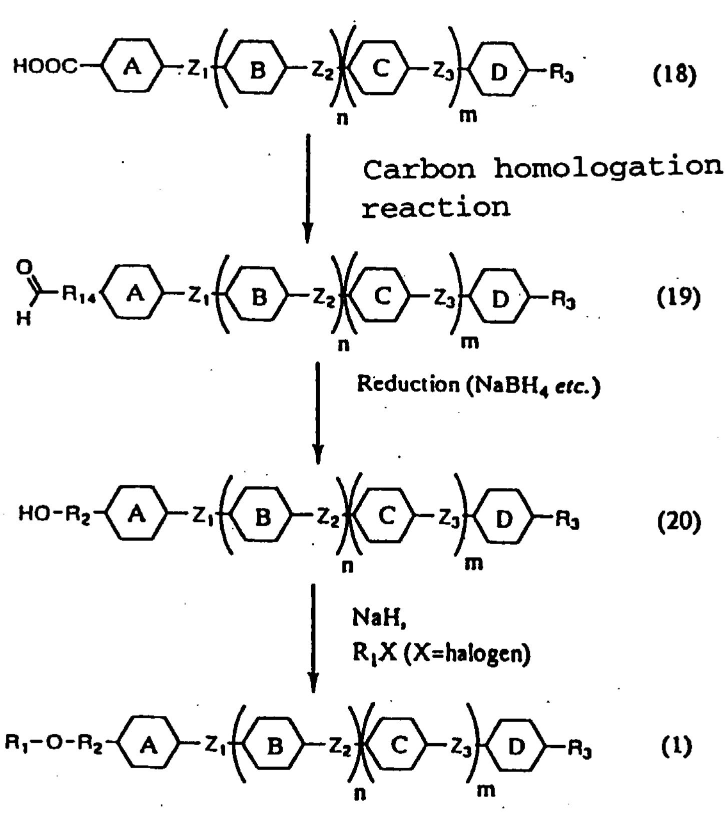


(18)

(19)

(20)

(1)

wherein $R_{14}$ represents single bond or an alkylene group having 1 to 4 carbon atoms.

In the case of the compounds expressed by the general formula (1) wherein any of $Z_1$, $Z_2$, and $Z_3$ represents $-CH_2-CH_2-$, a main skeleton can be constructed according to a method described in Laid-open Japanese Patent Publication No. Hei 5-140015, and in the case wherein any of the $Z_1$ to $Z_3$ represents $-(CH_2)_4-$, a main skeleton can be constructed according to a method described in Laid-open Japanese Patent Publication No. Hei 5-310605, respectively. Compounds expressed by the general formula (1) wherein the position of $Z_1$, $Z_2$, and $Z_3$ are -CH=CH- or -C≡C- can be produced according to a method described in Laid-open Japanese Patent Publication No. Hei 6-92924 or C. E. Castro et al., J. Org. Chem., 28, 2163, 3313 (1963). When the compounds contain $-CH_2O-$ or $-OCH_2-$, the compounds can be produced according to a method described in Japanese Patent Publication No. Hei 2-6743. Compounds expressed by the general formula (1) wherein the position of $Z_1$, $Z_2$, and $Z_3$ are -COO- or -OCO- can be produced according to a method described in B. K. Sadashiva, Mol. Cryst., 55, 135 (1979).

Further, the compounds expressed by the general formula (1) wherein $Z_1$, $Z_2$, and $Z_3$ represent $-CF_2O-$ or $-OCF_2-$ can be produced according to a method described in Lid-open Japanese Patent Publication No. Hei 5-112778 or 5-255165.

BEST MODE FOR CARRYING OUT THE INVENTION

Now, the method for producing or using the compounds of the present invention will be described in more detail with reference to Examples. In the Examples, C indicates crystal, N nematic phase, S smectic phase, and I indicates isotropic liquid, and the unit of all phase transition temperature is °C.

Example 1

Synthesis of 4-(trans-4-(3-butenyl)cyclohexyl)-1-propyloxymethylbenzene (Compound expressed by the general formula (1) wherein $R_1$ is $C_3H_7$, $R_2$ is $-CH_2-$, $R_3$ is vinyl group, ring A is 1,4-phenylene group, ring D is 1,4-cyclohexylene group, $Z_1$ is single bond, and m and n are 0; Compound No. 1)

(First stage)

In 15 ml of toluene was dissolved 2.4 g (0.01 mol) of the 4-(trans-4-(3-butenyl)cyclohexyl)-1-cyanobenzene

obtained according to a method described in Japanese Patent Publication No. 4-30382, cooled down to -60°C, and 360 ml of diisobutyl aluminum hydride (1M toluene solution) was added dropwise thereto while stirring at the same temperature, and then the solution was stirred at the same temperature for 2 hours. After finishing of the reaction, 15 ml of saturated aqueous solution of ammonium chloride was added thereto at a temperature lower than 0°C in 30 min, and then 30 ml of 10 % aqueous sulfuric acid solution to make the reaction solution acidic. Precipitates thus separated were filtered off by using a Celite.

The filtrate was extracted with toluene (twice, each time 200 ml), the extract was washed with saturated aqueous sodium chloride (thrice, each time 100 ml) and dried over anhydrous magnesium sulfate, and then the solvent was distilled off to obtain 3.0 g of 4-(trans-4-(3-butenyl)cyclohexyl) benzaldehyde.

(Second stage)

Solution prepared by dissolving 3.0 g of the 4-(trans-4-(3-butenyl)cyclohexyl)benzaldehyde obtained by the procedures in the first stage in 20 ml of 2-propanol was added dropwise while stirring in 1 hour to a solution prepared by adding 10 ml of 2-propanol to 0.57 g (0.015 mol) of sodium boron hydride and cooling down to a temperature lower than 0°C, and then stirred at the same temperature for 1 hour. After finishing of the reaction, 50 ml of saturated aqueous sodium chloride solution was added in 30 min, 2-propanol was distilled off, and then the reaction product was extracted with toluene (thrice, each time 100 ml). The extract was washed with saturated aqueous sodium chloride solution (thrice, each time 100 ml) and dried over anhydrous magnesium sulfate, and then the solvent was distilled off to obtain 2.7 g of 4-(trans-4-(3-butenyl)cyclohexyl)benzyl alcohol.

(Third stage)

Solution prepared by dissolving 2.7 g of the 4-(trans-4-(3-butenyl)cyclohexyl)benzyl alcohol obtained by the second stage in 10 ml of dimethylformamide (hereinafter abbreviated as DMF) and 0.05 g of potassium iodide were added while stirring at room temperature to a solution prepared by dissolving 0.29 g (0.012 mol) of sodium hydride to 10 ml of DMF. After 1.86 g (0.015 mol) of n-propyl bromide was added dropwise to this solution in 15 min, the solution thus obtained was stirred at room temperature for 24 hours. After 10 ml of water was added dropwise to the reaction solution, the reaction product was extracted with toluene (thrice, each time 100 ml). The extract was washed with saturated aqueous sodium chloride solution (thrice, each time 100 ml) and dried over anhydrous magnesium sulfate, and then the solvent was distilled off to obtain a crude product. The crude product thus obtained was purified by silica gel column chromatography (eluent: toluene/ethyl acetate = 5/1) to obtain 0.87 g of 4-(trans-4-(3-butenyl)cyclohexyl)-1-n-propyloxymethyl benzene. Melting point of this compound was lower than room temperature.

$^1$H-NMR ($CDCl_3$) $\delta$ (ppm): 7.345-7.131 (m, 4H), 6.095-5.646 (m, 1H), 5.110-4.916 (m, 2H), 4.481 (s, 2H), 3.450 (t, 2H), 2.613-0.678 (m, 21H)
MS: 286 ($M^+$)

According to the synthesis method described above, the following compounds can be produced:

Compound No. 2
4-(trans-4-vinylcyclohexyl)-1-methyloxymethylbenzene
Compound No. 3
4-(trans-4-vinylcyclohexyl)-1-ethyloxymethylbenzene
Compound No. 4
4-(trans-4-vinylcyclohexyl)-1-n-propyloxymethylbenzene
Compound No. 5
4-(trans-4-vinylcyclohexyl)-1-n-butyloxymethylbenzene
Compound No. 6
3-fluoro-4-(trans-4-vinylcyclohexyl)-1-n-pentyloxymethylbenzene
Compound No. 7
4-(trans-4-vinylcyclohexyl)-1-(2-propenyl)oxymethyl)benzene
Compound No. 8
2-fluoro-4-(trans-4-vinylcyclohexyl)-1-(2-butenyl)oxymethylbenzene
Compound No. 9
4-(trans-4-vinylcyclohexyl)-1-(3-butenyl)oxymethylbenzene
Compound No. 10
4-(trans-4-vinylcyclohexyl)-1-(2-pentenyl)oxymethylbenzene

Compound No. 11
2-fluoro-4-(trans-4-vinylcyclohexyl)-1-(3-pentenyl)oxymethylbenzene
Compound No. 12
4-(trans-4-vinylcyclohexyl)-1-(4-pentenyl)oxymethylbenzene
Compound No. 13
4-(trans-4-(1-propenyl)cyclohexyl)-1-methyloxymethylbenzene
Compound No. 14
4-(trans-4-(1-propenyl)cyclohexyl)-1-ethyloxymethylbenzene
Compound No. 15
4-(trans-4-(1-propenyl)cyclohexyl)-1-n-propyloxymethylbenzene
Compound No. 16
4-(trans-4-(1-propenyl)cyclohexyl)-1-n-butyloxymethylbenzene
Compound No. 17
4-(trans-4-(1-propenyl)cyclohexyl)-1-n-pentyloxymethylbenzene
Compound No. 18
4-(trans-4-(1-propenyl)cyclohexyl)-1-(2-propenyl)oxymethylbenzene
Compound No. 19
4-(trans-4-(1-propenyl)cyclohexyl)-1-(2-butenyl)oxymethylbenzene
Compound No. 20
4-(trans-4-(1-propenyl)cyclohexyl)-1-(3-butenyl)oxymethylbenzene
Compound No. 21
4-(trans-4-(1-propenyl)cyclohexyl)-1-(2-pentenyl)oxymethylbenzene
Compound No. 22
4-(trans-4-(1-propenyl)cyclohexyl)-1-(3-pentenyl)oxymethylbenzene
Compound No. 23
4-(trans-4-(1-propenyl)cyclohexyl)-1-(4-pentenyl)oxymethylbenzene
Compound No. 24
4-(trans-4-(2-propenyl)cyclohexyl)-1-methyloxymethylbenzene
Compound No. 25
4-(trans-4-(2-propenyl)cyclohexyl)-1-ethyloxymethylbenzene
Compound No. 26
4-(trans-4-(2-propenyl)cyclohexyl)-1-n-propyloxymethylbenzene
Compound No. 27
4-(trans-4-(2-propenyl)cyclohexyl)-1-n-butyloxymethylbenzene
Compound No. 28
4-(trans-4-(2-propenyl)cyclohexyl)-1-n-pentyloxymethylbenzene
Compound No. 29
4-(trans-4-(2-propenyl)cyclohexyl)-1-(2-propenyl)oxymethylbenzene
Compound No. 30
4-(trans-4-(2-propenyl)cyclohexyl)-1-(2-butenyl)oxymethylbenzene
Compound No. 31
4-(trans-4-(2-propenyl)cyclohexyl)-1-(3-butenyl)oxymethylbenzene
Compound No. 32
4-(trans-4-(2-propenyl)cyclohexyl)-1-(2-pentenyl)oxymethylbenzene
Compound No. 33
4-(trans-4-(2-propenyl)cyclohexyl)-1-(3-pentenyl)oxymethylbenzene
Compound No. 34
4-(trans-4-(2-propenyl)cyclohexyl)-1-(4-pentenyl)oxymethylbenzene
Compound No. 35
4-(trans-4-(1-butenyl)cyclohexyl)-1-methyloxymethylbenzene
Compound No. 36
4-(trans-4-(1-butenyl)cyclohexyl)-1-ethyloxymethylbenzene
Compound No. 37
3-fluoro-4-(trans-4-(1-butenyl)cyclohexyl)-1-n-propyloxymethylbenzene
Compound No. 38
4-(trans-4-(1-butenyl)cyclohexyl)-1-n-butyloxymethylbenzene
Compound No. 39
4-(trans-4-(1-butenyl)cyclohexyl)-1-n-pentyloxymethylbenzene

Compound No. 40
4-(trans-4-(1-butenyl)cyclohexyl)-1-(2-propenyl)oxymethylbenzene
Compound No. 41
4-(trans-4-(1-butenyl)cyclohexyl)-1-(2-butenyl)oxymethylbenzene
Compound No. 42
4-(trans-4-(1-butenyl)cyclohexyl)-1-(3-butenyl)oxymethylbenzene
Compound No. 43
4-(trans-4-(1-butenyl)cyclohexyl)-1-(2-pentenyl)oxymethylbenzene
Compound No. 44
4-(trans-4-(1-butenyl)cyclohexyl)-1-(3-pentenyl)oxymethylbenzene
Compound No. 45
4-(trans-4-(1-butenyl)cyclohexyl)-1-(4-pentenyl)oxymethylbenzene
Compound No. 46
4-(trans-4-(2-butenyl)cyclohexyl)-1-methyloxymethylbenzene
Compound No. 47
4-(trans-4-(2-butenyl)cyclohexyl)-1-ethyloxymethylbenzene
Compound No. 48
4-(trans-4-(2-butenyl)cyclohexyl)-1-n-propyloxymethylbenzene
Compound No. 49
4-(trans-4-(2-butenyl)cyclohexyl)-1-n-butyloxymethylbenzene
Compound No. 50
4-(trans-4-(2-butenyl)cyclohexyl)-1-n-pentyloxymethylbenzene
Compound No. 51
4-(trans-4-(2-butenyl)cyclohexyl)-1-(2-propenyl)oxymethylbenzene
Compound No. 52
4-(trans-4-(2-butenyl)cyclohexyl)-1-(2-butenyl)oxymethylbenzene
Compound No. 53
4-(trans-4-(2-butenyl)cyclohexyl)-1-(3-butenyl)oxymethylbenzene
Compound No. 54
4-(trans-4-(2-butenyl)cyclohexyl)-1-(2-pentenyl)oxymethylbenzene
Compound No. 55
4-(trans-4-(2-butenyl)cyclohexyl)-1-(3-pentenyl)oxymethylbenzene
Compound No. 56
4-(trans-4-(2-butenyl)cyclohexyl)-1-(4-pentenyl)oxymethylbenzene
Compound No. 57
4-(trans-4-(3-butenyl)cyclohexyl)-1-methyloxymethylbenzene
Compound No. 58
4-(trans-4-(3-butenyl)cyclohexyl)-1-ethyloxymethylbenzene
Compound No. 59
4-(trans-4-(3-butenyl)cyclohexyl)-1-n-butyloxymethylbenzene
Compound No. 60
4-(trans-4-(3-butenyl)cyclohexyl)-1-n-pentyloxymethylbenzene
Compound No. 61
4-(trans-4-(3-butenyl)cyclohexyl)-1-(2-propenyl)oxymethylbenzene
Compound No. 62
4-(trans-4-(3-butenyl)cyclohexyl)-1-(2-butenyl)oxymethylbenzene
Compound No. 63
4-(trans-4-(3-butenyl)cyclohexyl)-1-(3-butenyl)oxymethylbenzene
Compound No. 64
4-(trans-4-(3-butenyl)cyclohexyl)-1-(2-pentenyl)oxymethylbenzene
Compound No. 65
4-(trans-4-(3-butenyl)cyclohexyl)-1-(3-pentenyl)oxymethylbenzene
Compound No. 66
4-(trans-4-(3-butenyl)cyclohexyl)-1-(4-pentenyl)oxymethylbenzene
Compound No. 67
4-(trans-4-(1-pentenyl)cyclohexyl)-1-methyloxymethylbenzene
Compound No. 68
4-(trans-4-(1-pentenyl)cyclohexyl)-1-ethyloxymethylbenzene

Compound No. 69
4-(trans-4-(1-pentenyl)cyclohexyl)-1-n-propyloxymethylbenzene
Compound No. 70
4-(trans-4-(1-pentenyl)cyclohexyl)-1-n-butyloxymethylbenzene
Compound No. 71
4-(trans-4-(1-pentenyl)cyclohexyl)-1-n-pentyloxymethylbenzene
Compound No. 72
4-(trans-4-(1-pentenyl)cyclohexyl)-1-(2-propenyl)oxymethylbenzene
Compound No. 73
4-(trans-4-(1-pentenyl)cyclohexyl)-1-(2-butenyl)oxymethylbenzene
Compound No. 74
4-(trans-4-(1-pentenyl)cyclohexyl)-1-(3-butenyl)oxymethylbenzene
Compound No. 75
4-(trans-4-(1-pentenyl)cyclohexyl)-1-(2-pentenyl)oxymethylbenzene
Compound No. 76
4-(trans-4-(1-pentenyl)cyclohexyl)-1-(3-pentenyl)oxymethylbenzene
Compound No. 77
4-(trans-4-(1-pentenyl)cyclohexyl)-1-(4-pentenyl)oxymethylbenzene
Compound No. 78
4-(trans-4-(2-pentenyl)cyclohexyl)-1-methyloxymethylbenzene
Compound No. 79
4-(trans-4-(2-pentenyl)cyclohexyl)-1-ethyloxymethylbenzene
Compound No. 80
4-(trans-4-(2-pentenyl)cyclohexyl)-1-n-propyloxymethylbenzene
Compound No. 81
4-(trans-4-(2-pentenyl)cyclohexyl)-1-n-butyloxymethylbenzene
Compound No. 82
4-(trans-4-(2-pentenyl)cyclohexyl)-1-n-pentyloxymethylbenzene
Compound No. 83
4-(trans-4-(2-pentenyl)cyclohexyl)-1-(2-propenyl)oxymethylbenzene
Compound No. 84
4-(trans-4-(2-pentenyl)cyclohexyl)-1-(2-butenyl)oxymethylbenzene
Compound No. 85
4-(trans-4-(2-pentenyl)cyclohexyl)-1-(3-butenyl)oxymethylbenzene
Compound No. 86
4-(trans-4-(2-pentenyl)cyclohexyl)-1-(2-pentenyl)oxymethylbenzene
Compound No. 87
4-(trans-4-(2-pentenyl)cyclohexyl)-1-(3-pentenyl)oxymethylbenzene
Compound No. 88
3-fluoro-4-(trans-4-(2-pentenyl)cyclohexyl)-1-(4-pentenyl)oxymethylbenzene
Compound No. 89
4-(trans-4-(3-pentenyl)cyclohexyl)-1-methyloxymethylbenzene
Compound No. 90
4-(trans-4-(3-pentenyl)cyclohexyl)-1-ethyloxymethylbenzene
Compound No. 91
4-(trans-4-(3-pentenyl)cyclohexyl)-1-n-propyloxymethylbenzene
Compound No. 92
4-(trans-4-(3-pentenyl)cyclohexyl)-1-n-butyloxymethylbenzene
Compound No. 93
4-(trans-4-(3-pentenyl)cyclohexyl)-1-n-pentyloxymethylbenzene
Compound No. 94
4-(trans-4-(3-pentenyl)cyclohexyl)-1-(2-propenyl)oxymethylbenzene
Compound No. 95
4-(trans-4-(3-pentenyl)cyclohexyl)-1-(2-butenyl)oxymethylbenzene
Compound No. 96
4-(trans-4-(3-pentenyl)cyclohexyl)-1-(3-butenyl)oxymethylbenzene
Compound No. 97
4-(trans-4-(3-pentenyl)cyclohexyl)-1-(2-pentenyl)oxymethylbenzene

Compound No. 98
4-(trans-4-(3-pentenyl)cyclohexyl)-1-(3-pentenyl)oxymethylbenzene
Compound No. 99
4-(trans-4-(3-pentenyl)cyclohexyl)-1-(4-pentenyl)oxymethylbenzene
Compound No. 100
4-(trans-4-(4-pentenyl)cyclohexyl)-1-methyloxymethylbenzene
Compound No. 101
4-(trans-4-(4-pentenyl)cyclohexyl)-1-ethyloxymethylbenzene
Compound No. 102
4-(trans-4-(4-pentenyl)cyclohexyl)-1-n-propyloxymethylbenzene
Compound No. 103
4-(trans-4-(4-pentenyl)cyclohexyl)-1-n-butyloxymethylbenzene
Compound No. 104
4-(trans-4-(4-pentenyl)cyclohexyl)-1-n-pentyloxymethylbenzene
Compound No. 105
4-(trans-4-(4-pentenyl)cyclohexyl)-1-(2-propenyl)oxymethylbenzene
Compound No. 106
4-(trans-4-(4-pentenyl)cyclohexyl)-1-(2-butenyl)oxymethylbenzene
Compound No. 107
4-(trans-4-(4-pentenyl)cyclohexyl)-1-(3-butenyl)oxymethylbenzene
Compound No. 108
4-(trans-4-(4-pentenyl)cyclohexyl)-1-(2-pentenyl)oxymethylbenzene
Compound No. 109
4-(trans-4-(4-pentenyl)cyclohexyl)-1-(3-pentenyl)oxymethylbenzene
Compound No. 110
4-(trans-4-(4-pentenyl)cyclohexyl)-1-(4-pentenyl)oxymethylbenzene
Compound No. 111
4-(trans-4-vinylcyclohexyl)-1-methyloxy-(1-propyl)benzene
Compound No. 112
4-(trans-4-vinylcyclohexyl)-1-ethyloxy-(1-propyl)benzene
Compound No. 113
4-(trans-4-vinylcyclohexyl)-1-n-propyloxy-(1-propyl)benzene
Compound No. 114
4-(trans-4-vinylcyclohexyl)-1-(2-propenyl)oxy-(1-propyl)benzene
Compound No. 115
4-(trans-4-(1-propenyl)cyclohexyl)-1-methyloxy-(1-propyl)benzene
Compound No. 116
4-(trans-4-(1-propenyl)cyclohexyl)-1-ethyloxy-(1-propyl)benzene
Compound No. 117
4-(trans-4-(1-propenyl)cyclohexyl)-1-n-propyloxy-(1-propyl)benzene
Compound No. 118
4-(trans-4-(1-propenyl)cyclohexyl)-1-(2-propenyl)oxy-(1-propyl)benzene
Compound No. 119
4-(trans-4-(2-propenyl)cyclohexyl)-1-methyloxy-(1-propyl)benzene
Compound No. 120
4-(trans-4-(2-propenyl)cyclohexyl)-1-ethyloxy-(1-propyl)benzene
Compound No. 121
4-(trans-4-(2-propenyl)cyclohexyl)-1-n-propyloxy-(1-propyl)benzene
Compound No. 122
4-(trans-4-(2-propenyl)cyclohexyl)-1-(2-propenyl)oxy-(1-propyl)benzene
Compound No. 123
4-(trans-4-(1-butenyl)cyclohexyl)-1-methyloxy-(1-propyl)benzene
Compound No. 124
4-(trans-4-(1-butenyl)cyclohexyl)-1-ethyloxy-(1-propyl)benzene
Compound No. 125
4-(trans-4-(1-butenyl)cyclohexyl)-1-n-propyloxy-(1-propyl)benzene
Compound No. 126
4-(trans-4-(1-butenyl)cyclohexyl)-1-(2-propenyl)oxy-(1-propyl)benzene

Compound No. 127
4-(trans-4-(2-butenyl)cyclohexyl)-1-methyloxy-(1-propyl)benzene
Compound No. 128
4-(trans-4-(2-butenyl)cyclohexyl)-1-ethyloxy-(1-propyl)benzene
Compound No. 129
4-(trans-4-(2-butenyl)cyclohexyl)-1-n-propyloxy-(1-propyl)benzene
Compound No. 130
4-(trans-4-(2-butenyl)cyclohexyl)-1-(2-propenyl)oxy-(1-propyl)benzene
Compound No. 131
4-(trans-4-(3-butenyl)cyclohexyl)-1-methyloxy-(1-propyl)benzene
Compound No. 132
4-(trans-4-(3-butenyl)cyclohexyl)-1-ethyloxy-(1-propyl)benzene
Compound No. 133
4-(trans-4-(3-butenyl)cyclohexyl)-1-n-propyloxy-(1-propyl)benzene
Compound No. 134
4-(trans-4-(3-butenyl)cyclohexyl)-1-(2-propenyl)oxy-(1-propyl)benzene
Compound No. 135
4-(trans-4-(1-pentenyl)cyclohexyl)-1-methyloxy-(1-propyl)benzene
Compound No. 136
4-(trans-4-(1-pentenyl)cyclohexyl)-1-ethyloxy-(1-propyl)benzene
Compound No. 137
4-(trans-4-(1-pentenyl)cyclohexyl)-1-n-propyloxy-(1-propyl)benzene
Compound No. 138
2-fluoro-4-(trans-4-(1-pentenyl)cyclohexyl)-1-(2-propenyl)oxy-(1-propyl)benzene
Compound No. 139
4-(trans-4-(2-pentenyl)cyclohexyl)-1-methyloxy-(1-propyl)benzene
Compound No. 140
4-(trans-4-(2-pentenyl)cyclohexyl)-1-ethyloxy-(1-propyl)benzene
Compound No. 141
4-(trans-4-(2-pentenyl)cyclohexyl)-1-n-propyloxy-(1-propyl)benzene
Compound No. 142
4-(trans-4-(2-pentenyl)cyclohexyl)-1-(2-propenyl)oxy-(1-propyl)benzene
Compound No. 143
4-(trans-4-(3-pentenyl)cyclohexyl)-1-methyloxy-(1-propyl)benzene
Compound No. 144
4-(trans-4-(3-pentenyl)cyclohexyl)-1-ethyloxy-(1-propyl)benzene
Compound No. 145
4-(trans-4-(3-pentenyl)cyclohexyl)-1-n-propyloxy-(1-propyl)benzene
Compound No. 146
4-(trans-4-(3-pentenyl)cyclohexyl)-1-(2-propenyl)oxy-(1-propyl)benzene
Compound No. 147
4-(trans-4-(4-pentenyl)cyclohexyl)-1-methyloxy-(1-propyl)benzene
Compound No. 148
4-(trans-4-(4-pentenyl)cyclohexyl)-1-ethyloxy(1-propyl)benzene
Compound No. 149
4-(trans-4-(4-pentenyl)cyclohexyl)-1-n-propyloxy-(1-propyl)benzene
Compound No. 150
4-(trans-4-(4-pentenyl)cyclhexyl)-1-(2-propenyl)oxy-(1-propyl)benzene
Compound No. 151
4-(trans-4-vinylcyclohexyl)-1-methyloxy-(1-pentyl)benzene
Compound No. 152
4-(trans-4-(1-propyl)cyclohexyl)-1-methyloxy(1-pentyl)benzene
Compound No. 153
4-(trans-4-(2-propyl)cyclohexyl)-1-methyloxy(1-pentyl)benzene
Compound No. 154
4-(trans-4-(1-butenyl)cyclohexyl)-1-methyloxy-(1-pentyl)benzene
Compound No. 155
4-(trans-4-(2-butenyl)cyclohexyl)-1-methyloxy-(1-pentyl)benzene

Compound No. 156
4'-vinyl-4-methyloxymethylbiphenyl
Compound No. 157
4'-vinyl-4-ethyloxymethylbiphenyl
Compound No. 158
4'-vinyl-4-n-propyloxymethylbiphenyl
Compound No. 159
4'-vinyl-4-n-butyloxymethylbiphenyl
Compound No. 160
4'-vinyl-4-n-pentyloxymethylbiphenyl
Compound No. 161
4'-vinyl-4-(2-propenyl)oxymethylbiphenyl
Compound No. 162
4'-vinyl-4-(2-butenyl)oxymethylbiphenyl
Compound No. 163
4'-vinyl-4-(3-butenyl)oxymethylbiphenyl
Compound No. 164
4'-vinyl-2-fluoro-4-(2-pentenyl)oxymethylbiphenyl
Compound No. 165
4'-vinyl-4-(3-pentenyl)oxymethylbiphenyl
Compound No. 166
4'-vinyl-4-(4-pentenyl)oxymethylbiphenyl
Compound No. 167
4'-(1-propenyl)-4-methyloxymethylbiphenyl
Compound No. 168
4'-(1-propenyl)-4-ethyloxymethylbiphenyl
Compound No. 169
4'-(1-propenyl)-4-n-propyloxymethylbiphenyl
Compound No. 170
4'-(1-propenyl)-4-n-butyloxymethylbiphenyl
Compound No. 171
4'-(1-propenyl)-4-n-pentyloxymethylbiphenyl
Compound No. 172
4'-(1-propenyl)-4-(2-propenyl)oxymethylbiphenyl
Compound No. 173
4'-(1-propenyl)-4-(2-butenyl)oxymethylbiphenyl
Compound No. 174
4'-(1-propenyl)-4-(3-butenyl)oxymethylbiphenyl
Compound No. 175
4'-(1-propenyl)-4-(2-pentenyl)oxymethylbiphenyl
Compound No. 176
4'-(1-propenyl)-4-(3-pentenyl)oxymethylbiphenyl
Compound No. 177
4'-(1-propenyl)-4-(4-pentenyl)oxymethylbiphenyl
Compound No. 178
3'-fluoro-4'-(2-propenyl)-4-methyloxymethylbiphenyl
Compound No. 179
4'-(2-propenyl)-4-ethyloxymethylbiphenyl
Compound No. 180
4'-(2-propenyl)-4-n-propyloxymethylbiphenyl
Compound No. 181
4'-(2-propenyl)-4-n-butyloxymethylbiphenyl
Compound No. 182
4'-(2-propenyl)-4-n-pentyloxymethylbiphenyl
Compound No. 183
4'-(2-propenyl)-4-(2-propenyl)oxymethylbiphenyl
Compound No. 184
4'-(2-propenyl)-4-(2-butenyl)oxymethylbiphenyl

Compound No. 185
4'-(2-propenyl)-4-(3-butenyl)oxymethylbiphenyl
Compound No. 186
4'-(2-propenyl)-4-(2-pentenyl)oxymethylbiphenyl
Compound No. 187
4'-(2-propenyl)-4-(3-pentenyl)oxymethylbiphenyl
Compound No. 188
4'-(2-propenyl)-4-(4-pentenyl)oxymethylbiphenyl
Compound No. 189
4-(2-(trans-4-vinylcyclohexyl)ethyl)-1-methyloxymethylbenzene
Compound No. 190
4-(2-(trans-4-vinylcyclohexyl)ethenyl)-1-ethyloxymethylbenzene
Compound No. 191
4-(2-(trans-4-vinylcyclohexyl)ethyl)-1-n-propyloxymethylbenzene
Compound No. 192
4-(2-(trans-4-vinylcyclohexyl)ethenyl)-1-n-butyloxymethylbenzene
Compound No. 193
4-(2-(trans-4-vinylcyclohexyl)ethyl)-1-n-pentyloxymethylbenzene
Compound No. 194
4-(2-(trans-4-vinylcyclohexyl)ethyl)-1-(2-propenyl)oxymethylbenzene
Compound No. 195
2,3-difluoro-4-(2-(trans-4-vinylcyclohexyl)ethyl)-1-(2-butenyl)oxymethylbenzene
Compound No. 196
4-(2-(trans-4-vinylcyclohexyl)ethyl)-1-(3-butenyl)oxymethylbenzene
Compound No. 197
4-(2-(trans-4-vinylcyclohexyl)ethyl)-1-(2-pentenyl)oxymethylbenzene
Compound No. 198
4-(2-(trans-4-vinylcyclohexyl)ethyl)-1-(3-pentenyl)oxymethylbenzene
Compound No. 199
4-(2-(trans-4-vinylcyclohexyl)ethyl)-1-(4-pentenyl)oxymethylbenzene
Compound No. 200
4-(1-(4-(trans-4-vinylcyclohexyl)butyl)-1-methyloxymethylbenzene
Compound No. 201
4-(1-(4-(trans-4-vinylcyclohexyl)butyl)-1-ethyloxymethylbenzene
Compound No. 202
4-(1-(4-(trans-4-vinylcyclohexyl)butyl)-(1,4-dibutenyl)-1-n-propyloxymethylbenzene
Compound No. 203
4-(1-(4-(trans-4-vinylcyclohexyl)butyl)-1-n-butyloxymethylbenzene
Compound No. 204
4-(1-(4-(trans-4-vinylcyclohexyl)butyl)-1-n-pentyloxymethylbenzene
Compound No. 205
4-(1-(4-(trans-4-vinylcyclohexyl)butyl)-1-(2-propenyl)oxymethylbenzene
Compound No. 206
4-(1-(4-(trans-4-vinylcyclohexyl)butyl)-1-(2-butenyl)oxymethylbenzene
Compound No. 207
4-(1-(4-(trans-4-vinylcyclohexyl)butyl)-1-(3-butenyl)oxymethylbenzene
Compound No. 208
4-(1-(4-(trans-4-vinylcyclohexyl)butyl)-1-(2-pentenyl)oxymethylbenzene
Compound No. 209
4-(1-(4-(trans-4-vinylcyclohexyl)butyl)-1-(3-pentenyl)oxymethylbenzene
Compound No. 210
4-(1-(4-(trans-4-vinylcyclohexyl)butyl)-1-(4-pentenyl)oxymethylbenzene
Compound No. 211
4-(2-(trans-4-(1-propenyl)cyclohexyl)ethyl)-1-methyloxymethylbenzene
Compound No. 212
4-(2-(trans-4-(1-propenyl)cyclohexyl)ethyl)-1-ethyloxymethylbenzene
Compound No. 213
2-fluoro-4-(2-(trans-4-(1-propenyl)cyclohexyl)ethyl)-1-n-propyloxymethylbenzene

Compound No. 214
4-(2-(trans-4-(1-propenyl)cyclohexyl)ethyl)-1-n-butyloxymethylbenzene
Compound No. 215
4-(2-(trans-4-(1-propenyl)cyclohexyl)ethyl)-1-n-pentyloxymethylbenzene
Compound No. 216
4-(2-(trans-4-(1-propenyl)cyclohexyl)ethyl)-1-(2-propenyl)oxymethylbenzene
Compound No. 217
4-(2-(trans-4-(1-propenyl)cyclohexyl)ethyl)-1-(2-butenyl)oxymethylbenzene
Compound No. 218
4-(2-(trans-4-(1-propenyl)cyclohexyl)ethyl)-1-(3-butenyl)oxymethylbenzene
Compound No. 219
4-(2-(trans-4-(1-propenyl)cyclohexyl)ethynyl)-1-(2-pentenyl)oxymethylbenzene
Compound No. 220
4-(2-(trans-4-(1-propenyl)cyclohexyl)ethyl)-1-(3-pentenyl)oxymethylbenzene
Compound No. 221
4-(2-(trans-4-(1-propenyl)cyclohexyl)ethyl)-1-(4-pentenyl)oxymethylbenzene
Compound No. 222
2-fluoro-4-(1-(4-(trans-4-(1-propenyl)cyclohexyl)butyl)-1-methyloxymethylbenzene
Compound No. 223
4-(1-(4-(trans-4-(1-propenyl)cyclohexyl)butyl)-1-ethyloxymethylbenzene
Compound No. 224
4-(1-(4-(trans-4-(1-propenyl)cyclohexyl)butyl)-1-n-propyloxymethylbenzene
Compound No. 225
4-(1-(4-(trans-4-(1-propenyl)cyclohexyl)butyl)-1-n-butyloxymethylbenzene
Compound No. 226
4-(1-(4-(trans-4-(1-propenyl)cyclohexyl)butyl)-1-n-pentyloxymethylbenzene
Compound No. 227
4-(1-(4-(trans-4-(1-propenyl)cyclohexyl)butyl)-1-(2-propenyl)oxymethylbenzene
Compound No. 228
4-(1-(4-(trans-4-(1-propenyl)cyclohexyl)butyl)-1-(2-butenyl)oxymethylbenzene
Compound No. 229
4-(1-(4-(trans-4-(1-propenyl)cyclohexyl)butyl)-1-(3-butenyl)oxymethylbenzene
Compound No. 230
4-(1-(4-(trans-4-(1-propenyl)cyclohexyl)butyl)-1-(2-pentenyl)oxymethylbenzene
Compound No. 231
4-(1-(4-(trans-4-(1-propenyl)cyclohexyl)butyl)-1-(3-pentenyl)oxymethylbenzene
Compound No. 232
4-(1-(4-(trans-4-(1-propenyl)cyclohexyl)butyl)-1-(4-pentenyl)oxymethylbenzene
Compound No. 233
4-(2-(trans-4-(2-propenyl)cyclohexyl)ethyl)-1-methyloxymethylbenzene
Compound No. 234
4-(2-(trans-4-(2-propenyl)cyclohexyl)ethyl)-1-ethyloxymethylbenzene
Compound No. 235
4-(2-(trans-4-(2-propenyl)cycloheyl)ethyl)-1-n-propyloxymethylbenzene
Compound No. 236
4-(2-(trans-4-(2-propenyl)cyclohexyl)ethyl)-1-n-butyloxymethylbenzene
Compound No. 237
4-(2-(trans-4-(2-propenyl)cyclohexyl)ethyl)-1-n-pentyloxymethylbenzene
Compound No. 238
4-(2-(trans-4-(2-propenyl)cyclohexyl)ethyl)-1-(2-propenyl)oxymethylbenzene
Compound No. 239
4-(2-(trans-4-(2-propenyl)cyclohexyl)ethyl)-1-(2-butenyl)oxymethylbenzene
Compound No. 240
3-fluoro-4-(2-(trans-4-(2-propenyl)cyclohexyl)ethyl)-1-(3-butenyl)oxymethylbenzene
Compound No. 241
4-(2-(trans-4-(2-propenyl)cyclohexyl)ethyl)-1-(2-pentenyl)oxymethylbenzene
Compound No. 242
4-(2-(trans-4-(2-propenyl)cyclohexyl)ethyl)-1-(3-pentenyl)oxymethylbenzene

Compound No. 243
4-(2-(trans-4-(2-propenyl)cyclohexyl)ethyl)-1-(4-pentenyl)oxymethylbenzene
Compound No. 244
4-(1-(4-(trans-4-(2-propenyl)cyclohexyl)butyl)-1-methyloxymethylbenzene
Compound No. 245
4-(1-(4-(trans-4-(2-propenyl)cyclohexyl)butyl)-1-ethyloxymethylbenzene
Compound No. 246
4-(1-(4-(trans-4-(2-propenyl)cyclohexyl)butyl)-1-n-propyloxymethylbenzene
Compound No. 247
4-(1-(4-(trans-4-(2-propenyl)cyclohexyl)butyl)-1-n-butyloxymethylbenzene
Compound No. 248
4-(1-(4-(trans-4-(2-propenyl)cyclohexyl)butyl)-1-n-pentyloxymethylbenzene
Compound No. 249
4-(1-(4-(trans-4-(2-propenyl)cyclohexyl)butyl)-1-(2-propenyl)oxymethylbenzene
Compound No. 250
4-(1-(4-(trans-4-(2-propenyl)cyclohexyl)butyl)-1-(2-butenyl)oxymethylbenzene
Compound No. 251
4-(1-(4-(trans-4-(2-propenyl)cyclohexyl)butyl)-1-(3-butenyl)oxymethylbenzene
Compound No. 252
4-(1-(4-(trans-4-(2-propenyl)cyclohexyl)butyl)-1-(2-pentenyl)oxymethylbenzene
Compound No. 253
4-(1-(4-(trans-4-(2-propenyl)cyclohexyl)butyl)-1-(3-pentenyl)oxymethylbenzene
Compound No. 254
4-(1-(4-(trans-4-(2-propenyl)cyclohexyl)butyl)-1-(4-pentenyl)oxymethylbenzene
Compound No. 255
4-(2-(4-vinylphenyl)ethyl)-1-methyloxymethylbenzene
Compound No. 256
4-(2-(4-vinylphenyl)methyloxy)-1-ethyloxymethylbenzene
Compound No. 257
4-(2-(4-vinylphenyl)ethyl)-1-n-propyloxymethylbenzene
Compound No. 258
4-(2-(4-vinylphenyl)ethyl)-1-n-butyloxymethylbenzene
Compound No. 259
4-(2-(4-vinylphenyl)oxymethyl)-1-n-pentyloxymethylbenzene
Compound No. 260
4-(2-(4-vinylphenyl)ethyl)-1-(2-propenyl)oxymethylbenzene
Compound No. 261
4-(2-(4-vinylphenyl)ethyl)-1-(2-butenyl)oxymethylbenzene
Compound No. 262
4-(2-(4-vinylphenyl)ethyl)-1-(3-butenyl)oxymethylbenzene
Compound No. 263
4-(2-(4-vinylphenyl)ethenyl)-1-(2-pentenyl)oxymethylbenzene
Compound No. 264
4-(2-(4-vinylphenyl)ethyl)-1-(3-pentenyl)oxymethylbenzene
Compound No. 265
4-(2-(4-vinylphenyl)ethyl)-1-(4-pentenyl)oxymethylbenzene
Compound No. 266
4-(1-(4-(trans-4-vinylphenyl)butyl)-1-methyloxymethylbenzene
Compound No. 267
4-(1-(4-(trans-4-vinylphenyl)butyl)-1-ethyloxymethylbenzene
Compound No. 268
4-(1-(4-(4-vinylphenyl)butyl)-1-n-propyloxymethylbenzene
Compound No. 269
4-(1-(4-(4-vinylphenyl)butyl)-1-n-butyloxymethylbenzene
Compound No. 270
4-(1-(4-(4-vinylphenyl)-2-butenyl)-1-n-pentyloxymethylbenzene
Compound No. 271
4-(1-(4-(4-vinylphenyl)butyl)-1-(2-propenyl)oxymethylbenzene

Compound No. 272
4-(1-(4-(4-vinylphenyl)butyl)-1-(2-butenyl)oxymethylbenzene
Compound No. 273
4-(1-(4-(4-vinylphenyl)butyl)-1-(3-butenyl)oxymethylbenzene
Compound No. 274
4-(1-(4-(4-vinylphenyl)butyl)-1-(2-pentenyl)oxymethylbenzene
Compound No. 275
4-(1-(4-(4-vinylphenyl)butyl)-1-(3-pentenyl)oxymethylbenzene
Compound No. 276
4-(1-(4-(4-vinylphenyl)butyl)-1-(4-pentenyl)oxymethylbenzene
Compound No. 277
4-(4-(2-propenyl)phenyl)difluoromethyloxy-1-methyloxymethylbenzene
Compound No. 278
4-(4-vinylphenyl)oxydifluoromethyl-1-n-propyloxymethylbenzene
Compound No. 279
4-(4-(2-propenyl)benzoyloxy)-1-methyloxymethylbenzene
Compound No. 280
4-(4-vinylbenzoxycarbonyl)-1-propyloxymethylbenzene
Compound No. 281
4-(trans-4-vinylcyclohexyl)methoxy-1-n-propyloxymethylbenzene
Compound No. 282
4-(trans-4-(2-propenyl)cyclohexyl)methyloxy-1-methyloxymethylbenzene
Compound No. 283
4-(trans-4-(4-pentenyl)cyclohexyl)difluoromethoxy-1-methyloxymethylbenzene
Compound No. 284
4-(trans-4-vinylcycloheyxl)oxydifluoromethyl-1-n-propyloxymethylbenzene
Compound No. 285
4-(trans-4-vinylcyclohexylcarbonyloxy)-1-methyloxymethylbenzene
Compound No. 286
4-(trans-4-vinylcyclohexyloxycarbonyl)-1-n-pentyloxymethylbenzene

Example 2

Synthesis of 4'-(trans-4-vinylcyclohexyl)-4-n-propyloxymethylbiphenyl (Compound expressed by the general formula (1) wherein $R_1$ is n-$C_3H_7$, $R_2$ is -$CH_2$-, $R_3$ is vinyl group, n is 1, m is 0, rings A and B are 1,4-phenylene group, ring D is 1,4-cyclohexylene group, and $Z_1$ and $Z_2$ are single bond; Compound No. 287)

(First stage)

Solution prepared by dissolving 141.3 g (0.9 mol) of bromobenzene in 500 ml of tetrahydrofuran (THF) was added dropwise while stirring at room temperature in 1 hour to a liquid prepared by suspending 22.6 g (0.93 mol) of magnesium in 30 ml of THF, and then stirred at the same temperature to prepare a Grignard reagent.

(Second stage)

Palladium chloride in an amount of 1.0 g was added to a solution prepared by dissolving 198.1 g (0.6 mol) of the 4-(trans-4-methoxymethylcyclohexyl)iodo benzene obtained by a method described in Japanese Patent Publication No. Hei 1-41621 in 1 ℓ of THF, and then heated to 60°C. Subsequently, to the solution was added dropwise the Grignard reagent obtained by the first stage, while stirring in 1 hour. After finishing of the dropping, the solution was stirred at the same temperature for further 3 hours. After 600 ml of water was added to the reaction solution under a condition cooled with ice, the reaction product was extracted with toluene (thrice, each time 1 ℓ). The extract was washed with 300 ml of saturated aqueous solution of sodium thiosulfate and saturated aqueous sodium chloride solution (thrice, each time 500 ml) in turn and dried over anhydrous magnesium sulfate, and then the solvent was distilled off to obtain a crude product. The crude product thus obtained was purified by silica gel column chromatography (eluent: toluene) and then recrystallized from 200 ml of heptane to obtain 65.3 g of 4-(trans-4-methoxymethylcyclohexyl)biphenyl.

(Third stage)

Acetic acid in an amount of 300 ml, 24.6 g (0.14 mol) of iodic acid, 32.5 g (0.13 mol) of iodine, 85 ml of water, and 11.4 g (0.12 mol) of a concentrated sulfuric acid were added in turn to a solution prepared by dissolving 65.3 g (0.23 mol) of the 4-(trans-4-methoxymethylcyclohexyl)biphenyl obtained by the second stage in turn, and heated to reflux while stirring for 5 hours. The reaction solution was allowed to cool down to room temperature, and poured into 300 ml of an ice water, and the reaction product was extracted with toluene (once in an amount of 1 ℓ; and twice, each time 300 ml). The extract was washed with 300 ml of saturated aqueous solution of sodium thiosulfate and saturated aqueous sodium chloride solution (thrice, each time 300 ml) in turn, and dried over anhydrous magnesium sulfate, and then the solvent was distilled off to obtain a crude product. The crude product thus obtained was purified by silica gel column chromatography (eluent: n-heptane) to obtain 93.1 g of 4'-(trans-4-methoxymethylcyclohexyl)-4-iodobiphenyl.

(Fourth stage)

Solution prepared by dissolving 93.1 g (0.23 mol) of the 4'-(trans-4-methoxymethylcyclohexyl)-4-iodobiphenyl obtained by the third stage in 300 ml of dimethylformamide (DMF) was added to a solution prepared by dissolving 30.8 g (0.34 mol) of cuprous cyanide in 200 ml of DMF, and heated to reflux for 6 hours. The reaction solution was allowed to cool. Subsequently, a solution prepared by dissolving 21.7 g (0.08 mol) of ferric chloride hexahydrate in 200 mol of 6N hydrochloric acid was added to the reaction solution, heated to 50°C, and then stirred for 2 hours. Toluene in an amount of 1 ℓ was added to the reaction solution, and the precipitates thus produced were filtered off by using a Celite. The organic layer separated from the filtrate was washed with 300 ml of saturated aqueous solution of sodium thiosulfate and saturated aqueous sodium chloride solution (thrice, each time 500 ml) in turn and dried over anhydrous magnesium sulfate, and then the solvent was distilled off to obtain a crude product. The crude product thus obtained was recrystallized from 360 ml of ethyl acetate to obtain 58.2 g of 4'-(trans-4-methoxymethylcyclohexyl)-4-cyanobiphenyl.

(Fifth stage)

In a mixed solvent of 300 ml of chloroform and 300 ml of acetonitrile was dissolved 58.2 g (0.21 mol) of the 4'-(trans-4-methoxymethylcyclohexyl)-4-cyanobiphenyl obtained by the fourth stage, 62.5 g (3.1 mol) of trimethylsilyl iodide was added dropwise to the solution while stirring at 40°C, and the solution was further heated while stirring at the same temperature for 1 hour. After 500 ml of water was added to the reaction solution which was allowed to cool down to room temperature, the reaction product was extracted with methylene chloride (thrice, each time 500 ml). The extract was washed with 300 ml of saturated aqueous solution of sodium thiosulfate and saturated aqueous sodium chloride solution (thrice, each time 300 ml) in turn and dried over anhydrous magnesium sulfate, and then the solvent was distilled off to obtain a crude product. The crude product thus obtained was recrystallized from 50 ml of toluene to obtain 14.3 g of 4'-(trans-4-hydroxymethylcyclohexyl)-4-cyanobiphenyl.

(Sixth stage)

Pyridinium chlorochromate (PCC) in an amount of 30.8 g (0.14 mol), 35 g of silica gel, and 300 ml of methylene chloride were mixed, and a solution prepared by dissolving 37.9 g of the 4'-(trans-4-hydroxymethylcyclohexyl)-4-cyanobiphenyl obtained by the fifth stage in 500 ml of methylene chloride was added to the mixture and then stirred at room temperature for 24 hours. The silica gel was separated by filtration from the reaction solution, and then the solvent was distilled off from the filtrate to obtain a crude product. The crude product thus obtained was purified by silica gel column chromatography (eluent: toluene/ethyl acetate = 2/1) to obtain 28.5 g of 4'-(trans-4-formylcyclohexyl)-4-cyanobiphenyl.

(Seventh stage)

After 10.8 g (0.095 mol) of potassium t-butoxide was added to a solution prepared by dissolving 34.9 g (0.086 mol) of methyltriphenylphosphonium iodide in 100 ml of THF, the solution was stirred at -50°C for 2 hours. To this solution was added dropwise at the same temperature a solution prepared by dissolving 20.0 g of the 4'-(trans-4-formylcyclohexyl)-4-cyanobiphenyl obtained by the sixth stage, and they were further stirred for 3 hours. After the reaction solution was warmed up to 0°C, 150 ml of water was added thereto to terminate the reaction. The reaction solution was extracted with toluene (once in an amount of 600 ml; and twice, each time 200 ml). The extract was washed with saturated aqueous sodium chloride solution (thrice, each time 300 ml), saturated aqueous solution of sodium bicarbonate (twice, each time 300 ml), and saturated aqueous sodium chloride solution (thrice, each time 300 ml) in turn and dried over anhydrous magnesium sulfate, and then the solvent was distilled off to obtain a crude product. The crude product thus obtained was purified by silica gel column chromatography (eluent: toluene) to obtain 17.6 g of 4'-(trans-4-vinylcyclohexyl)-4-cyanobiphenyl.

(Eighth stage)

After 16.7 g (0.058 mol) of the 4'-(trans-4-vinylcyclohexyl)-4-cyanobiphenyl obtained by the seventh stage was dissolved in 150 ml of toluene and cooled down to -73°C, 87 ml (0.087 mol) of diisobutylaluminum hydride was added dropwise while stirring, and then stirred for 2 hours. Saturated aqueous solution of ammonium chloride in an amount of 100 ml was added to the reaction solution to terminate the reaction, and then 300 ml of 6N hydrochloric acid was added to make the solution acidic. The precipitates thus formed were filtered off by using a Celite. The filtrate was extracted with toluene (twice, each time 300 ml), the extract was washed with saturated aqueous sodium chloride solution (thrice, each time 300 ml) and dried over anhydrous magnesium sulfate, and then the solvent was distilled off under a reduced pressure to obtain 17.3 g of 4'-(trans-4-vinylcyclohexyl)-4-biphenylcarbaldehyde.

(Ninth stage)

Solution prepared by dissolving 17.3 g of the 4'-(trans-4-vinylcyclohexyl)-4-biphenylcarbaldehyde obtained by the eighth stage in a mixed solvent of 300 ml of isopropanol and 200 ml of toluene was added dropwise to a solution prepared by dissolving 3.4 g (0.089 mol) of sodium boron hydride to 20 ml of isopropanol, under a condition cooled with ice in 1 hour, and stirred at the same temperature for 2 hours. To the reaction solution was added dropwise 400 ml of 2N hydrochloric acid to terminate the reaction. From the reaction solution, the organic solvent was distilled off, and the remaining water layer was extracted with toluene (thrice, each time 700 ml). The extract was washed with saturated aqueous sodium chloride solution (thrice, each time 300 ml), and dried over anhydrous magnesium sulfate, and then the solvent was distilled off under a reduced pressure to obtain a crude product. The crude product was recrystallized from 20 ml of a mixed solvent (heptane/ethyl acetate = 3/1) to obtain 10.2 g of 4'-(trans-4-vinylcyclohexyl)-4-hydroxymethylbiphenyl.

(Tenth stage)

The 4'-(trans-4-vinylcyclohexyl)-4-hydroxymethylbiphenyl obtained by the ninth stage in an amount of 2.9 g (0.010 mol) and 5.0 g (0.04 mol) of n-propyl bromide were dissolved in 50 ml of DMF, and 0.01 g of potassium iodide was added to the solution and stirred at room temperature. Sodium hydride in an amount of 0.60 g (0.025 mol) was added to the solution in 30 min, and then the solution was stirred for 24 hours. Water in an amount of 100 ml was added to the reaction solution, and the reaction product was extracted with toluene (thrice, each time 100 ml). The extract was washed with saturated aqueous sodium chloride solution (fifth times, each time 300 ml) and dried over anhydrous magnesium sulfate, and then the solvent was distilled off to obtain a crude product. The crude product thus obtained was purified by silica gel column chromatography (eluent: heptane/ethyl acetate = 4/1) and then recrystallized from 5 ml of a mixed solvent (ethanol/ethyl acetate = 5/1) to obtain 1.9 g of 4'-(trans-4-vinylcyclohexyl)-4-n-propyloxymethylbiphenyl which is the objective compound of this Example. This compound exhibited liquid crystallinity, and had a S-I point of 112.4~ 113.2°C and a S-N point of 124.7~125.5°C.

$^1$H-NMR ($CDCl_3$) δ (ppm): 7.588-6.942 (m, 8H), 6.009-5.633 (m, 1H), 5.073-4.860 (m, 2H), 4.506 (s, 2H), 3.435 (t, 2H), 2.497-0.525 (m, 17H)
MS: 334 ($M^+$)

According to the production method described above, the following compounds can be produced:

Compound No. 288
4'-(trans-4-vinylcyclohexyl)-4-methyloxymethylbiphenyl
Compound No. 289
4'-(trans-4-vinylcyclohexyl)-4-methyloxy-n-propylbiphenyl
Compound No. 290
4'-(trans-4-vinylcyclohexyl)-4-methyloxy-n-pentylbiphenyl
Compound No. 291
4'-(trans-4-vinylcyclohexyl)-4-ethyloxymethylbiphenyl
Compound No. 292
4'-(trans-4-vinylcyclohexyl)-4-n-propyloxymethylbiphenyl
Compound No. 293
4'-(trans-4-vinylcyclohexyl)-4-n-butyloxymethylbiphenyl

Compound No. 294
4'-(trans-4-vinylcyclohexyl)-4-n-pentyloxymethylbiphenyl
Compound No. 295
4'-(trans-4-vinylcyclohexyl)-4-(2-propenyl)oxymethylbiphenyl
Compound No. 296
4'-(trans-4-vinylcyclohexyl)-4-(2-butenyl)oxymethylbiphenyl
Compound No. 297
4'-(trans-4-vinylcyclohexyl)-4-(3-butenyl)oxymethylbiphenyl
Compound No. 298
4'-(trans-4-vinylcyclohexyl)-4-(2-pentenyl)oxymethylbiphenyl
Compound No. 299
4'-(trans-4-vinylcyclohexyl)-4-(3-pentenyl)oxymethylbiphenyl
Compound No. 300
4'-(trans-4-vinylcyclohexyl)-4-(4-pentenyl)oxymethylbiphenyl
Compound No. 301
2'-fluoro-4'-(trans-4-(1-propenyl)cyclohexyl)-4-methyloxymethylbiphenyl
Compound No. 302
4'-(trans-4-(1-propenyl)cyclohexyl)-4-ethyloxymethylbiphenyl
Compound No. 303
4'-(trans-4-(1-propenyl)cyclohexyl)-4-n-propyloxymethylbiphenyl
Compound No. 304
4'-(trans-4-(1-propenyl)cyclohexyl)-4-n-butyloxymethylbiphenyl
Compound No. 305
4'-(trans-4-(1-propenyl)cyclohexyl)-2',6'-difluoro-4-n-pentyloxymethylbiphenyl
Compound No. 306
4'-(trans-4-(1-propenyl)cyclohexyl)-4-(2-propenyl)oxymethylbiphenyl
Compound No. 307
2,3'-difluoro-4'-(trans-4-(1-propenyl)cyclohexyl)-4-(2-butenyl)oxymethylbiphenyl
Compound No. 308
4'-(trans-4-(1-propenyl)cyclohexyl)-4-(3-butynyl)oxymethylbiphenyl
Compound No. 309
4'-(trans-4-(1-propenyl)cyclohexyl)-4-(2-pentynyl)oxymethylbiphenyl
Compound No. 310
4'-(trans-4-(1-propenyl)cyclohexyl)-4-(3-pentynyl)oxymethylbiphenyl
Compound No. 311
4'-(trans-4-(1-propenyl)cyclohexyl)-4-(4-pentynyl)oxymethylbiphenyl
Compound No. 312
4'-(trans-4-(2-propenyl)cyclohexyl)-4-methyloxymethylbiphenyl
Compound No. 313
4'-(trans-4-(2-propenyl)cyclohexyl)-3',5'-difluoro-4-ethyloxymethylbiphenyl
Compound No. 314
4'-(trans-4-(2-propenyl)cyclohexyl)-4-n-propyloxymethylbiphenyl
Compound No. 315
4'-(trans-4-(2-propenyl)cyclohexyl)-4-n-butyloxymethylbiphenyl
Compound No. 316
4'-(trans-4-(2-propenyl)cyclohexyl)-4-n-pentyloxymethylbiphenyl
Compound No. 317
2',3-difluoro-4'-(trans-4-(2-propenyl)cyclohexyl)-4-(2-propenyl)oxymethyl)biphenyl
Compound No. 318
4'-(trans-4-(2-propenyl)cyclohexyl)-4-(2-butenyl)oxymethylbiphenyl
Compound No. 319
4'-(trans-4-(2-propenyl)cyclohexyl)-4-(3-butenyl)oxymethylbiphenyl
Compound No. 320
4'-(trans-4-(2-propenyl)cyclohexyl)-4-(2-pentenyl)oxymethylbiphenyl
Compound No. 321
4'-(trans-4-(2-propenyl)cyclohexyl)-4-(3-pentenyl)oxymethylbiphenyl
Compound No. 322
4'-(trans-4-(2-propenyl)cyclohexyl)-4-(4-pentenyl)oxymethylbiphenyl

Compound No. 323
4'-(trans-4-(1-butenyl)cycloheyxl)-4-methyloxymethylbiphenyl
Compound No. 324
4'-(trans-4-(1-butenyl)cycloheyxl)-4-ethyloxymethylbiphenyl
Compound No. 325
4'-(trans-4-(1-butenyl)cycloheyxl)-4-n-propyloxymethylbiphenyl
Compound No. 326
4'-(trans-4-(1-butenyl)cycloheyxl)-4-n-butyloxymethylbiphenyl
Compound No. 327
4'-(trans-4-(1-butenyl)cycloheyxl)-4-n-pentyloxymethylbiphenyl
Compound No. 328
4'-(trans-4-(1-butenyl)cycloheyxl)-4-(2-propenyl)oxymethylbiphenyl
Compound No. 329
4'-(trans-4-(1-butenyl)cycloheyxl)-4-(2-butenyl)oxymethylbiphenyl
Compound No. 330
4'-(trans-4-(1-butenyl)cycloheyxl)-4-(3-butenyl)oxymethylbiphenyl
Compound No. 331
4'-(trans-4-(1-butenyl)cycloheyxl)-4-(2-pentenyl)oxymethylbiphenyl
Compound No. 332
4'-(trans-4-(1-butenyl)cycloheyxl)-4-(3-pentenyl)oxymethylbiphenyl
Compound No. 333
4'-(trans-4-(1-butenyl)cycloheyxl)-4-(4-pentenyl)oxymethylbiphenyl
Compound No. 334
4'-(trans-4-(2-butenyl)cyclohexyl)-4-methyloxymethylbiphenyl
Compound No. 335
4'-(trans-4-(2-butenyl)cyclohexyl)-4-ethyloxymethylbiphenyl
Compound No. 336
4'-(trans-4-(2-butenyl)cyclohexyl)-4-n-propyloxymethylbiphenyl
Compound No. 337
4'-(trans-4-(2-butenyl)cyclohexyl)-4-n-butyloxymethylbiphenyl
Compound No. 338
4'-(trans-4-(2-butenyl)cyclohexyl)-4-n-pentyloxymethylbiphenyl
Compound No. 339
4'-(trans-4-(2-butenyl)cyclohexyl)-4-(2-propenyl)oxymethylbiphenyl
Compound No. 340
4'-(trans-4-(2-butenyl)cyclohexyl)-4-(2-butenyl)oxymethylbiphenyl
Compound No. 341
4'-(trans-4-(2-butenyl)cyclohexyl)-4-(3-butenyl)oxymethylbiphenyl
Compound No. 342
4'-(trans-4-(2-butenyl)cyclohexyl)-4-(2-pentenyl)oxymethylbiphenyl
Compound No. 343
4'-(trans-4-(2-butenyl)cyclohexyl)-4-(3-pentenyl)oxymethylbiphenyl
Compound No. 344
4'-(trans-4-(2-butenyl)cyclohexyl)-4-(4-pentenyl)oxymethylbiphenyl
Compound No. 345
4'-(trans-4-(3-butenyl)cyclohexyl)-4-methyloxymethylbiphenyl
Compound No. 346
4'-(trans-4-(3-butenyl)cyclohexyl)-4-ethyloxymethylbiphenyl
Compound No. 347
4'-(trans-4-(3-butenyl)cyclohexyl)-4-n-propyloxymethylbiphenyl
Compound No. 348
4'-(trans-4-(3-butenyl)cyclohexyl)-4-n-butyloxymethylbiphenyl
Compound No. 349
4'-(trans-4-(3-butenyl)cyclohexyl)-4-n-pentyloxymethylbiphenyl
Compound No. 350
4'-(trans-4-(3-butenyl)cyclohexyl)-4-(2-propenyl)oxymethylbiphenyl
Compound No. 351
4'-(trans-4-(3-butenyl)cyclohexyl)-4-(2-butenyl)oxymethylbiphenyl

Compound No. 352
4'-(trans-4-(3-butenyl)cyclohexyl)-4-(3-butenyl)oxymethylbiphenyl
Compound No. 353
4'-(trans-4-(3-butenyl)cyclohexyl)-4-(2-pentenyl)oxymethylbiphenyl
Compound No. 354
4'-(trans-4-(3-butenyl)cyclohexyl)-4-(3-pentenyl)oxymethylbiphenyl
Compound No. 355
4'-(trans-4-(3-butenyl)cyclohexyl)-4-(4-pentenyl)oxymethylbiphenyl
Compound No. 356
4'-(trans-4-(1-pentenyl)cycloheyxl)-4-methyloxymethylbiphenyl
Compound No. 357
4'-(trans-4-(1-pentenyl)cycloheyxl)-4-ethyloxymethylbiphenyl
Compound No. 358
4'-(trans-4-(1-pentenyl)cycloheyxl)-4-n-propyloxymethylbiphenyl
Compound No. 359
4'-(trans-4-(1-pentenyl)cycloheyxl)-4-n-butyloxymethylbiphenyl
Compound No. 360
4'-(trans-4-(1-pentenyl)cycloheyxl)-4-n-pentyloxymethylbiphenyl
Compound No. 361
4'-(trans-4-(1-pentenyl)cycloheyxl)-4-(2-propenyl)oxymethylbiphenyl
Compound No. 362
4'-(trans-4-(1-pentenyl)cycloheyxl)-4-(2-butenyl)oxymethylbiphenyl
Compound No. 363
4'-(trans-4-(1-pentenyl)cycloheyxl)-4-(3-butenyl)oxymethylbiphenyl
Compound No. 364
4'-(trans-4-(1-pentenyl)cycloheyxl)-4-(2-pentenyl)oxymethylbiphenyl
Compound No. 365
4'-(trans-4-(1-pentenyl)cycloheyxl)-4-(3-pentenyl)oxymethylbiphenyl
Compound No. 366
4'-(trans-4-(1-pentenyl)cycloheyxl)-4-(4-pentenyl)oxymethylbiphenyl
Compound No. 367
4'-(trans-4-(2-pentenyl)cyclohexyl)-4-methyloxymethylbiphenyl
Compound No. 368
4'-(trans-4-(2-pentenyl)cyclohexyl)-4-ethyloxymethylbiphenyl
Compound No. 369
4'-(trans-4-(2-pentenyl)cyclohexyl)-4-n-propyloxymethylbiphenyl
Compound No. 370
4'-(trans-4-(2-pentenyl)cyclohexyl)-4-n-butyloxymethylbiphenyl
Compound No. 371
4'-(trans-4-(2-pentenyl)cyclohexyl)-4-n-pentyloxymethylbiphenyl
Compound No. 372
4'-(trans-4-(2-pentenyl)cyclohexyl)-4-(2-propenyl)oxymethylbiphenyl
Compound No. 273
4'-(trans-4-(2-pentenyl)cyclohexyl)-4-(2-butenyl)oxymethylbiphenyl
Compound No. 374
4'-(trans-4-(2-pentenyl)cyclohexyl)-4-(3-butenyl)oxymethylbiphenyl
Compound No. 375
4'-(trans-4-(2-pentenyl)cyclohexyl)-4-(2-pentenyl)oxymethylbiphenyl
Compound No. 376
4'-(trans-4-(2-pentenyl)cyclohexyl)-4-(3-pentenyl)oxymethylbiphenyl
Compound No. 377
4'-(trans-4-(2-pentenyl)cyclohexyl)-4-(4-pentenyl)oxymethylbiphenyl
Compound No. 378
4'-(trans-4-(3-pentenyl)cyclohexyl)-4-methyloxymethylbiphenyl
Compound No. 379
4'-(trans-4-(3-pentenyl)cyclohexyl)-4-ethyloxymethylbiphenyl
Compound No. 380
4'-(trans-4-(3-pentenyl)cyclohexyl)-4-n-propyloxymethylbiphenyl

Compound No. 381
4'-(trans-4-(3-pentenyl)cyclohexyl)-4-n-butyloxymethylbiphenyl
Compound No. 382
4'-(trans-4-(3-pentenyl)cyclohexyl)-4-n-pentyloxymethylbiphenyl
Compound No. 383
4'-(trans-4-(3-pentenyl)cyclohexyl)-4-(2-propenyl)oxymethylbiphenyl
Compound No. 384
4'-(trans-4-(3-pentenyl)cyclohexyl)-4-(2-butenyl)oxymethylbiphenyl
Compound No. 385
4'-(trans-4-(3-pentenyl)cyclohexyl)-4-(3-butenyl)oxymethylbiphenyl
Compound No. 386
4'-(trans-4-(3-pentenyl)cyclohexyl)-4-(2-pentenyl)oxymethylbiphenyl
Compound No. 387
4'-(trans-4-(3-pentenyl)cyclohexyl)-4-(3-pentenyl)oxymethylbiphenyl
Compound No. 388
4'-(trans-4-(3-pentenyl)cyclohexyl)-4-(4-pentenyl)oxymethylbiphenyl
Compound No. 389
4'-(trans-4-(4-pentenyl)cyclohexyl)-4-methyloxymethylbiphenyl
Compound No. 390
4'-(trans-4-(4-pentenyl)cyclohexyl)-4-ethyloxymethylbiphenyl
Compound No. 391
4'-(trans-4-(4-pentenyl)cyclohexyl)-4-n-propyloxymethylbiphenyl
Compound No. 392
4'-(trans-4-(4-pentenyl)cyclohexyl)-4-n-butyloxymethylbiphenyl
Compound No. 393
4'-(trans-4-(4-pentenyl)cyclohexyl)-4-n-pentyloxymethylbiphenyl
Compound No. 394
4'-(trans-4-(4-pentenyl)cyclohexyl)-4-(2-propenyl)oxymethylbiphenyl
Compound No. 395
4'-(trans-4-(4-pentenyl)cyclohexyl)-4-(2-butenyl)oxymethylbiphenyl
Compound No. 396
4'-(trans-4-(4-pentenyl)cyclohexyl)-4-(3-butenyl)oxymethylbiphenyl
Compound No. 397
4'-(trans-4-(4-pentenyl)cyclohexyl)-4-(2-pentenyl)oxymethylbiphenyl
Compound No. 398
4'-(trans-4-(4-pentenyl)cyclohexyl)-4-(3-pentenyl)oxymethylbiphenyl
Compound No. 399
4'-(trans-4-(4-pentenyl)cyclohexyl)-4-(4-pentenyl)oxymethylbiphenyl
Compound No. 400
4"-vinyl-4-methyloxymethyl-p-terphenyl
Compound No. 401
4"-vinyl-4-methyloxy-n-propyl-p-terphenyl
Compound No. 402
4"-vinyl-4-methyloxy-n-pentyl-p-terphenyl
Compound No. 403
4"-vinyl-2'-fluoro-4-ethyloxymethyl-p-terphenyl
Compound No. 404
4"-vinyl-4-n-propyloxymethyl-p-terphenyl
Compound No. 405
4"-vinyl-4-n-butyloxymethyl-p-terphenyl
Compound No. 406
4"-vinyl-4-n-pentyloxymethyl-p-terphenyl
Compound No. 407
4"-vinyl-4-(2-propenyl)oxymethyl-p-terphenyl
Compound No. 408
4"-vinyl-4-(2-butenyl)oxymethyl-p-terphenyl
Compound No. 409
4"-vinyl-2",2'-difluoro-4-(3-butenyl)oxymethyl-p-terphenyl

Compound No. 410
4"-vinyl-4-(2-pentenyl)oxymethyl-p-terphenyl
Compound No. 411
4"-vinyl-4-(3-pentenyl)oxymethyl-p-terphenyl
Compound No. 412
4"-vinyl-4-(4-pentenyl)oxymethyl-p-terphenyl
Compound No. 413
4"-(1-propenyl)-4-methyloxymethyl-p-terphenyl
Compound No. 414
4"-(1-propenyl)-4-ethyloxymethyl-p-terphenyl
Compound No. 415
4"-(1-propenyl)-4-n-propyloxymethyl-p-terphenyl
Compound No. 416
4"-(1-propenyl)-4-n-butyloxymethyl-p-terphenyl
Compound No. 417
4"-(1-propenyl)-4-n-pentyloxymethyl-p-terphenyl
Compound No. 418
4"-(1-propenyl)-4-(2-propenyl)oxymethyl-p-terphenyl
Compound No. 419
4"-(1-propenyl)-4-(2-butenyl)oxymethyl-p-terphenyl
Compound No. 420
4"-(1-propenyl)-4-(3-butenyl)oxymethyl-p-terphenyl
Compound No. 421
4"-(1-propenyl)-4-(2-pentenyl)oxymethyl-p-terphenyl
Compound No. 422
4"-(1-propenyl)-4-(3-pentenyl)oxymethyl-p-terphenyl
Compound No. 423
4"-(1-propenyl)-4-(4-pentenyl)oxymethyl-p-terphenyl
Compound No. 424
4"-(2-propenyl)-4-methyloxymethyl-p-terphenyl
Compound No. 425
4"-(2-propenyl)-4-ethyloxymethyl-p-terphenyl
Compound No. 426
4"-(2-propenyl)-4-n-propyloxymethyl-p-terphenyl
Compound No. 427
4"-(2-propenyl)-2'-fluoro-4-n-butyloxymethyl-p-terphenyl
Compound No. 428
4"-(2-propenyl)-4-n-pentyloxymethyl-p-terphenyl
Compound No. 429
4"-(2-propenyl)-4-(2-propenyl)oxymethyl-p-terphenyl
Compound No. 430
4"-(2-propenyl)-2-fluoro-4-(2-butenyl)oxymethyl-p-terphenyl
Compound No. 431
4"-(2-propenyl)-4-(3-butenyl)oxymethyl-p-terphenyl
Compound No. 432
4"-(2-propenyl)-4-(2-pentenyl)oxymethyl-p-terphenyl
Compound No. 433
4"-(2-propenyl)-4-(3-pentenyl)oxymethyl-p-terphenyl
Compound No. 434
4"-(2-propenyl)-4-(4-pentenyl)oxymethyl-p-terphenyl
Compound No. 435
4-(trans-4-(4-vinylphenyl)cyclohexyl)-1-methyloxymethylbenzene
Compound No. 436
4-(trans-4-(4-vinylphenyl)cyclohexyl)-1-methyloxy-n-propylbenzene
Compound No. 437
2-fluoro-4-(trans-4-(4-vinylphenyl)cyclohexyl)-1-methyloxy-n-pentylbenzene
Compound No. 438
4-(trans-4-(4-vinylphenyl)cyclohexyl)-1-ethyloxymethylbenzene

Compound No. 439
4-(trans-4-(4-vinylphenyl)cyclohexyl)-1-n-propyloxymethylbenzene
Compound No. 440
4-(trans-4-(4-vinylphenyl)cyclohexyl)-1-n-butyloxymethylbenzene
Compound No. 441
4-(trans-4-(4-vinylphenyl)cyclohexyl)-1-n-pentyloxymethylbenzene
Compound No. 442
4-(trans-4-(4-vinylphenyl)cyclohexyl)-1-(2-propenyl)oxymethylbenzene
Compound No. 443
4-(trans-4-(4-vinylphenyl)cyclohexyl)-1-(2-butenyl)oxymethylbenzene
Compound No. 444
4-(trans-4-(4-vinylphenyl)cyclohexyl)-1-(3-butenyl)oxymethylbenzene
Compound No. 445
4-(trans-4-(4-vinylphenyl)cyclohexyl)-1-(2-pentenyl)oxymethylbenzene
Compound No. 446
4-(trans-4-(4-vinylphenyl)cyclohexyl)-1-(3-pentenyl)oxymethylbenzene
Compound No. 447
4-(trans-4-(4-vinylphenyl)cyclohexyl)-1-(4-pentenyl)oxymethylbenzene
Compound No. 448
4-(trans-4-(4-(1-propenyl)phenyl)cyclohexyl)-1-methyloxymethylbenzene
Compound No. 449
4-(trans-4-(4-(1-propenyl)phenyl)cyclohexyl)-1-methyloxy-n-propylbenzene
Compound No. 450
4-(trans-4-(4-(1-propenyl)phenyl)cyclohexyl)-1-ethyloxymethylbenzene
Compound No. 451
4-(trans-4-(4-(1-propenyl)phenyl)cyclohexyl)-1-n-propyloxymethylbenzene
Compound No. 452
4-(trans-4-(4-(1-propenyl)phenyl)cyclohexyl)-1-n-butyloxymethylbenzene
Compound No. 453
4-(trans-4-(4-(1-propenyl)phenyl)cyclohexyl)-1-n-pentyloxymethylbenzene
Compound No. 454
4-(trans-4-(4-(1-propenyl)phenyl)cyclohexyl)-1-(2-propenyl)oxymethylbenzene
Compound No. 455
4-(trans-4-(4-(1-propenyl)phenyl)cyclohexyl)-1-(2-butenyl)oxymethylbenzene
Compound No. 456
4-(trans-4-(4-(1-propenyl)phenyl)cyclohexyl)-1-(3-butenyl)oxymethylbenzene
Compound No. 457
4-(trans-4-(4-(1-propenyl)phenyl)cyclohexyl)-1-(2-pentenyl)oxymethylbenzene
Compound No. 458
4-(trans-4-(4-(1-propenyl)phenyl)cyclohexyl)-1-(3-pentenyl)oxymethylbenzene
Compound No. 459
4-(trans-4-(4-(1-propenyl)phenyl)cyclohexyl)-1-(4-pentenyl)oxymethylbenzene
Compound No. 460
4-(trans-4-(4-(2-propenyl)phenyl)cyclohexyl)-1-methyloxymethylbenzene
Compound No. 461
4-(trans-4-(4-(2-propenyl)phenyl)cyclohexyl)-1-ethyloxymethylbenzene
Compound No. 462
4-(trans-4-(4-(2-propenyl)phenyl)cyclohexyl)-1-n-propyloxymethylbenzene
Compound No. 463
4-(trans-4-(4-(2-propenyl)phenyl)cyclohexyl)-1-n-butyloxymethylbenzene
Compound No. 464
4-(trans-4-(4-(2-propenyl)phenyl)cyclohexyl)-1-n-pentyloxymethylbenzene
Compound No. 465
4-(trans-4-(4-(2-propenyl)phenyl)cyclohexyl)-1-(2-propenyl)oxymethylbenzene
Compound No. 466
4-(trans-4-(4-(2-propenyl)phenyl)cyclohexyl)-1-(2-butenyl)oxymethylbenzene
Compound No. 467
4-(trans-4-(4-(2-propenyl)phenyl)cyclohexyl)-1-(3-butenyl)oxymethylbenzene

Compound No. 468
4-(trans-4-(4-(2-propenyl)phenyl)cyclohexyl)-1-(2-pentenyl)oxymethylbenzene
Compound No. 469
4-(trans-4-(4-(2-propenyl)phenyl)cyclohexyl)-1-(3-pentenyl)oxymethylbenzene
Compound No. 470
4-(trans-4-(4-(2-propenyl)phenyl)cyclohexyl)-1-(4-pentenyl)oxymethylbenzene
Compound No. 471
4'-(2-(trans-4-vinylcyclohexyl)ethyl)-4-methyloxymethylbiphenyl
Compound No. 472
4'-(2-(trans-4-vinylcyclohexyl)ethyl)-4-methyloxy-n-propylbiphenyl
Compound No. 473
4'-(2-(trans-4-vinylcyclohexyl)ethyl)-4-n-propyloxymethylbiphenyl
Compound No. 474
4'-(2-(trans-4-vinylcyclohexyl)ethyl)-4-n-pentyloxymethylbiphenyl
Compound No. 475
4'-(2-(trans-4-vinylcyclohexyl)ethyl)-4-(2-propenyl)oxymethylbiphenyl
Compound No. 476
4'-(2-(trans-4-vinylcyclohexyl)ethenyl)-4-(2-butenyl)oxymethylbiphenyl
Compound No. 477
4'-(2-(trans-4-vinylcyclohexyl)ethyl)-4-(3-butenyl)oxymethylbiphenyl
Compound No. 478
4'-(2-(trans-4-(1-propenyl)cyclohexyl)ethyl)-4-methyloxymethylbiphenyl
Compound No. 479
4'-(2-(trans-4-(1-propenyl)cyclohexyl)ethyl)-1-n-propyloxymethylbiphenyl
Compound No. 480
4'-(2-(trans-4-(1-propenyl)cyclohexyl)ethyl)-4-n-pentyloxymethylbiphenyl
Compound No. 481
4'-(2-(trans-4-(1-propenyl)cyclohexyl)ethyl)-4-(2-propenyl)oxymethylbiphenyl
Compound No. 482
4'-(2-(trans-4-(1-propenyl)cyclohexyl)ethynyl)-2'-fluoro-4-(2-butenyl)oxymethylbiphenyl
Compound No. 483
4'-(2-(trans-4-(1-propenyl)cyclohexyl)ethyl)-4-(3-butenyl)oxymethylbiphenyl
Compound No. 484
4'-(2-(trans-4-(2-propenyl)cyclohexyl)ethyl)-4-methyloxymethylbiphenyl
Compound No. 485
4'-(2-(trans-4-(2-propenyl)cyclohexyl)ethyl)-4-n-propyloxymethylbiphenyl
Compound No. 486
4'-(2-(trans-4-(2-propenyl)cyclohexyl)ethyl)-4-n-pentyloxymethylbiphenyl
Compound No. 487
4'-(2-(trans-4-(2-propenyl)cyclohexyl)ethyl)-4-(2-propenyl)oxymethylbiphenyl
Compound No. 488
4'-(2-(trans-4-(2-propenyl)cyclohexyl)ethyl)-4-(2-butenyl)oxymethylbiphenyl
Compound No. 489
4'-(2-(trans-4-(2-propenyl)cyclohexyl)ethyl)-4-(3-butenyl)oxymethylbiphenyl
Compound No. 490
4'-(trans-4-(2-propenyl)cyclohexylmethoxymethyl)-4-(2-propenyl)oxymethylbiphenyl
Compound No. 491
4'-(2-(trans-4-(2-propenyl)cyclohexyloxymethyl)-4-(2-propenyl)oxymethylbiphenyl
Compound No. 492
4'-(2-(trans-4-(3-butenyl)cyclohexyl)ethyl)-4-ethyloxymethylbiphenyl
Compound No. 493
4'-(2-(trans-4-(3-butenyl)cyclohexyl)ethyl)-4-n-propyloxymethylbiphenyl
Compound No. 494
4'-(4-(trans-4-vinylcyclohexyl)butyl)-4-methyloxymethylbiphenyl
Compound No. 495
4'-(4-(trans-4-vinylcyclohexyl)butyl)-4-methyloxy-n-pentylbiphenyl
Compound No. 496
4'-(4-(trans-4-vinylcyclohexyl)butyl)-4-n-propyloxymethylbiphenyl

Compound No. 497
4'-(4-(trans-4-vinylcyclohexyl)butyl)-4-n-pentyloxymethylbiphenyl
Compound No. 498
4'-(4-(trans-4-vinylcyclohexyl)-2-butenyl)-4-(2-propenyl)oxymethylbiphenyl
Compound No. 499
4'-(4-(trans-4-vinylcyclohexyl)butyl)-4-(2-butenyl)oxymethylbiphenyl
Compound No. 500
4'-(4-(trans-4-vinylcyclohexyl)butyl)-4-(3-butenyl)oxymethylbiphenyl
Compound No. 501
4'-(4-(trans-4-(1-propenyl)cyclohexyl)butyl)-4-methyloxymethylbiphenyl
Compound No. 502
4'-(4-(trans-4-(1-propenyl)cyclohexyl)butyl)-4-n-propyloxymethylbiphenyl
Compound No. 503
4'-(4-(trans-4-(1-propenyl)cyclohexyl)butyl)-4-n-pentyloxymethylbiphenyl
Compound No. 504
4'-(4-(trans-4-(1-propenyl)cyclohexyl)butyl)-4-(2-propenyl)oxymethylbiphenyl
Compound No. 505
4'-(4-(trans-4-(1-propenyl)cyclohexyl)butyl)-4-(2-butenyl)oxymethylbiphenyl
Compound No. 506
4'-(4-(trans-4-(1-propenyl)cyclohexyl)-3-butenyl)-4-(3-butenyl)oxymethylbiphenyl
Compound No. 507
4'-(4-(trans-4-(2-propenyl)cyclohexyl)butyl)-4-methyloxymethylbiphenyl
Compound No. 508
4'-(4-(trans-4-(2-propenyl)cyclohexyl)butyl)-4-n-propyloxymethylbiphenyl
Compound No. 509
4'-(4-(trans-4-(2-propenyl)cyclohexyl)butyl)-4-n-pentyloxymethylbiphenyl
Compound No. 510
4'-(4-(trans-4-(2-propenyl)cyclohexyl)butyl)-4-(2-propenyl)oxymethylbiphenyl
Compound No. 511
4'-(4-(trans-4-(2-propenyl)cyclohexyl)butyl)-4-(2-butenyl)oxymethylbiphenyl
Compound No. 512
4'-(4-(trans-4-(2-propenyl)cyclohexyl)butyl)-4-(3-butenyl)oxymethylbiphenyl
Compound No. 513
4-(4-(2-(trans-4-vinylcyclohexyl)ethyl)phenyl)-1-methyloxymethylbenzene
Compound No. 514
4-(2-(4-(trans-4-(1-propenyl)cyclohexyl)phenyl)ethyl)-4-n-propyloxymethylbenzene
Compound No. 515
4-(2-(4-(trans-4-(2-propenyl)cyclohexyl)phenyl)ethyl)-1-n-pentyloxymethylbenzene
Compound No. 516
4-(2-(4-(trans-4-(1-butenyl)cyclohexyl)phenyl)oxymethyl)-1-(2-propenyl)oxymethylbenzene
Compound No. 517
4-(2-(4-(trans-4-(2-butenyl)cyclohexyl)phenyl)ethyl)-1-(2-butenyl)oxymethylbenzene
Compound No. 518
4-(2-(4-(trans-4-(1-pentenyl)cyclohexyl)phenyl)difluoromethoxy) 1-(3-butenyl)oxymethylbenzene
Compound No. 519
4-(4-(4-(trans-4-(2-pentenyl)cyclohexyl)phenyl)butyl)-1-methyloxymethylbenzene
Compound No. 520
4-(4-(4-(trans-4-(3-pentenyl)cyclohexyl)phenyl)butyl)-1-n-propyloxymethylbenzene
Compound No. 521
4-(4-(4-(trans-4-(4-pentenyl)cyclohexyl)phenyl)butyl)-1-n-pentyloxymethylbenzene
Compound No. 522
4-(4-(4-(trans-4-vinylcyclohexyl)phenyl)butyl)-4-(2-propenyl)oxymethylbenzene
Compound No. 523
4-(4-(4-(trans-4-vinylcyclohexyl)phenyl)butyl)-1-(2-propenyl)oxy-n-propylbenzene
Compound No. 524
4-(4-(4-(trans-4-(1-propenyl)cyclohexyl)phenyl)butyl)-1-(2-butenyl)oxymethylbenzene
Compound No. 525
4-(4-(4-(trans-4-(2-propenyl)cyclohexyl)phenyl)butyl)-1-(3-butenyl)oxymethylbenzene

Compound No. 526
4-(2-(4-(2-(trans-4-(1-butenyl)cyclohexyl)ethyl)phenyl)ethyl)-1-methyloxymethylbenzene
Compound No. 527
4-(2-(4-(2-(trans-4-(2-butenyl)cyclohexyl)ethyl)phenyl)ethyl)-1-n-propyloxymethylbenzene
Compound No. 528
4-(2-(4-(2-(trans-4-(3-butenyl)cyclohexyl)ethyl)phenyl)ethyl)-1-n-pentyloxymethylbenzene
Compound No. 529
4-(2-(4-(2-(trans-4-(1-pentenyl)cyclohexyl)ethyl) phenyl)oxydifluoromethyl)-1-(2-propenyl)oxymethylbenzene
Compound No. 530
4-(2-(4-(2-(trans-4-(1-pentenyl)cyclohexyl)ethyl) phenyl)difluoromethyloxy)-1-(2-propenyl)oxymethylbenzene
Compound No. 531
4-(2-(4-(3-fluoro-2-(trans-4-(2-pentenyl)cyclohexyl)ethyl) phenyl)ethyl)-1-(2-butenyl)oxymethylbenzene
Compound No. 532
4-(2-(4-(2-(trans-4-(3-pentenyl)cycloheyxl)ethyl)phenyl)ethyl)-1-(3-butenyl)oxymethylbenzene
Compound No. 533
4-(2-(4-(2-(trans-4-(4-pentenyl)cycloheyxl)ethyl)phenyl)ethyl)-1-methyloxymethylbenzene
Compound No. 534
4-(2-(4-(2-(trans-4-(4-pentenyl)cycloheyxl)ethyl)phenyl)ethyl)-1-methyloxy-n-pentylbenzene
Compound No. 535
4-(2-(4-(4-(trans-4-vinylcycloheyxl)butyl)phenyl)ethyl)-1-n-propyloxymethylbenzene
Compound No. 536
4-(2-(4-(4-(trans-4-(1-propenyl)cyclohexyl)butyl)phenyl)ethyl)-1-n-pentyloxymethylbenzene
Compound No. 537
4-(2-(4-(4-(trans-4-(2-propenyl)cyclohexyl)butyl)phenyl)ethyl)-1-(2-propenyl)oxymethylbenzene
Compound No. 538
4-(2-(4-(4-(trans-4-(1-butenyl)cyclohexyl)butyl)phenyl)ethyl)-1-(2-butenyl)oxymethylbenzene
Compound No. 539
4-(2-(4-(4-(trans-4-(2-butenyl)cyclohexyl)-2-butenyl)phenyl)ethynyl)-1-(3-butenyl)oxymethylbenzene
Compound No. 540
4-(4-(4-(2-(trans-4-(3-butenyl)cyclohexyl)ethyl)phenyl)butyl)-1-methyloxymethylbenzene
Compound No. 541
4-(4-(4-(2-(trans-4-(1-pentenyl)cyclohexyl)ethyl)phenyl)butyl)-1-n-propyloxymethylbenzene
Compound No. 542
4-(4-(4-(2-(trans-4-(2-pentenyl)cyclohexyl)ethyl)phenyl)butyl)-1-n-pentyloxymethylbenzene
Compound No. 543
4-(4-(4-(2-(trans-4-(3-pentenyl)cyclohexyl)ethyl)phenyl)butyl)-1-(2-propenyl)oxymethylbenzene
Compound No. 544
4-(4-(4-(2-(trans-4-(4-pentenyl)cyclohexyl)ethyl)phenyl)butyl)-1-(2-butenyl)oxymethylbenzene
Compound No. 545
4-(4-(4-(2-(trans-4-vinylcyclohexyl)ethyl)phenyl)butyl)-1-(3-butenyl)oxymethylbenzene
Compound No. 546
4-(4-(4-(4-(trans-4-(1-propenyl)cyclohexyl)-2-butenyl)phenyl)-2-butenyl)-1-methyloxymethylbenzene
Compound No. 547
4-(4-(4-(4-(trans-4-(2-propenyl)cyclohexyl)butyl)phenyl)butyl)-1-n-propyloxymethylbenzene
Compound No. 548
4-(4-(4-(4-(trans-4-(1-butenyl)cyclohexyl)butyl)phenyl)butyl)-1-n-pentyloxymethylbenzene
Compound No. 549
4-(4-(4-(4-(trans-4-(2-butenyl)cyclohexyl)butyl)phenyl)butyl)-1-(2-propenyl)oxymethylbenzene
Compound No. 550
4-(4-(4-(4-(trans-4-(3-butenyl)cyclohexyl)butyl)phenyl)butyl)-1-(2-butenyl)oxymethylbenzene
Compound No. 551
4-(4-(4-(4-(trans-4-vinylcyclohexyl)butyl)phenyl)butyl)-1-(3-butenyl)oxymethylbenzene
Compound No. 552
4'-(2-(4-vinylphenyl)ethyl)-4-methyloxymethylbiphenyl
Compound No. 553
4'-(2-(4-(1-propenyl)phenyl)ethyl)-4-ethyloxymethylbiphenyl
Compound No. 554
4'-(2-(4-(2-propenyl)phenyl)ethyl)-4-n-propyloxymethylbiphenyl

Compound No. 555
4'-(2-(4-(1-butenyl)phenyl)ethyl)-4-n-butyloxymethylbiphenyl
Compound No. 556
4'-(2-(4-(2-butenyl)phenyl)ethyl)-4-n-pentyloxymethylbiphenyl
Compound No. 557
4'-(2-(4-(1-pentenyl)phenyl)ethyl)-4-(2-propenyl)oxymethylbiphenyl
Compound No. 558
3'-fluoro-4'-(2-(4-(2-pentenyl)phenyl)ethyl)-4-(2-butenyl)oxymethylbiphenyl
Compound No. 559
4'-(2-(4-(3-pentenyl)phenyl)ethyl)-4-(3-butenyl)oxymethylbiphenyl
Compound No. 560
4'-(2-(4-(4-pentenyl)phenyl)ethyl)-4-(2-pentenyl)oxymethylbiphenyl
Compound No. 561
4'-(2-(4-vinylphenyl)methoxy)-4-(3-pentenyl)oxymethylbiphenyl
Compound No. 562
4'-(2-(4-vinylphenyl)ethyl)-4-(4-pentenyl)oxymethylbiphenyl
Compound No. 563
2-(4-(2-(4-(4-pentenyl)phenyl)ethenyl)phenyl)ethyl-4-methyloxymethylphenyl
Compound No. 564
2-(4-(2-(4-(3-pentenyl)phenyl)ethyl)phenyl)ethyl-4-ethyloxymethylbiphenyl
Compound No. 565
2-(4-(2-(4-(2-pentenyl)phenyl)ethyl)phenyl)ethyl-1-n-propyloxymethylbenzene
Compound No. 566
2-(4-(2-(4-(1-pentenyl)phenyl)ethyl)phenyl)ethyl-1-n-butyloxymethylbenzene
Compound No. 567
2-(4-(2-(4-(1-butenyl)phenyl)ethyl)phenyl)ethyl-1-n-pentyloxymethylbenzene
Compound No. 568
2-(4-(2-(4-(2-butenyl)phenyl)ethyl)phenyl)ethyl-1-(2-propenyl)oxymethylphenyl
Compound No. 569
2-(4-(2-(4-(3-butenyl)phenyl)ethyl)phenyl)ethyl-1-(2-butenyl)oxymethylbenzene
Compound No. 570
2-(4-(2-(4-(1-propenyl)phenyl)ethyl)phenyl)ethyl-1-(3-butenyl)oxymethylbenzene
Compound No. 571
2-(4-(2-(4-(2-propenyl)phenyl)ethyl)phenyl)ethyl-1-(2-pentenyl)oxymethylbenzene
Compound No. 572
2-(4-(2-(4-vinylphenyl)ethyl)phenyl)ethynyl-1-(3-pentenyl)oxymethylbenzene
Compound No. 573
2-(4-(2-(4-vinylphenyl)ethyl)phenyl)ethyl-1-(4-pentenyl)oxymethylbenzene
Compound No. 574
2-(4-(4-(4-vinylphenyl)butyl)phenyl)ethyl-1-methyloxymethylphenyl
Compound No. 575
2-(4-(4-(4-(1-propenyl)phenyl)butyl)phenyl)ethyl-1-ethyloxymethylbenzene
Compound No. 576
2-(4-(4-(4-(2-propenyl)phenyl)butyl)phenyl)ethyl-1-n-propyloxymethylbenzene
Compound No. 577
4-(4-(4-(4-vinylphenyl)butyl)phenyl)butyl-1-n-butyloxymethylbenzene
Compound No. 578
4-(4-(4-(4-(4-butenyl)phenyl)butyl)phenyl)butyl-1-n-pentyloxymethylbenzene
Compound No. 579
4-(4-(4-(4-(2-butenyl)phenyl)-1-butenyl)phenyl)butyl-1-(2-propenyl)oxymethylphenyl
Compound No. 580
4-(4-(4-(4-(3-butenyl)phenyl)-2-butenyl)phenyl)butyl-1-(2-butenyl)oxymethylbenzene
Compound No. 581
4-(4-(4-(4-(1-propenyl)phenyl)butyl)phenyl)butyl-1-(3-butenyl)oxymethylbenzene
Compound No. 582
4-(4-(4-(4-(2-propenyl)phenyl)butyl)phenyl)ethyl-1-(2-pentenyl)oxymethylbenzene
Compound No. 583
4-(4-(4-(4-vinylphenyl)butyl)phenyl)butyl-1-(3-pentenyl)oxymethylbenzene

Compound No. 584
4-(4-(4-(4-vinylphenyl)butyl)phenyl)butyl-1-(4-pentenyl)oxymethylbenzene
Compound No. 585
4-(trans-4-(2-(4-vinylphenyl)ethenyl)cyclohexyl)-1-methyloxymethylbenzene
Compound No. 586
4-(trans-4-(2-(4-vinylphenyl)ethenyl)cyclohexyl)-1-methyloxy-n-propylbenzene
Compound No. 587
4-(trans-4-(2-(4-vinylphenyl)ethyl)cyclohexyl)-1-methyloxy-n-pentylbenzene
Compound No. 588
4-(trans-4-(2-(4-(1-propenyl)phenyl)ethyl)cyclohexyl)-1-n-propyloxymethylbenzene
Compound No. 589
4-(trans-4-(2-(4-(2-propenyl)phenyl)ethyl)cyclohexyl)-1-n-pentyloxymethylbenzene
Compound No. 590
4-(trans-4-(2-(4-(1-pentenyl)phenyl)ethyl)cyclohexyl)-1-(2-propenyl)oxymethylbenzene
Compound No. 591
4-(trans-4-(2-(4-(2-pentenyl)phenyl)ethyl)cyclohexyl)-1-(2-butenyl)oxymethylbenzene
Compound No. 592
4-(trans-4-(2-(4-(3-pentenyl)phenyl)ethyl)cyclohexyl)-1-(3-butenyl)oxymethylbenzene
Compound No. 593
4-(trans-4-(2-(4-(4-pentenyl)phenyl)ethyl)cyclohexyl)-1-(2-pentenyl)oxymethylbenzene
Compound No. 594
4-(trans-4-(2-(4-vinylphenyl)ethyl)cyclohexyl)-1-(3-pentenyl)oxymethylbenzene
Compound No. 595
4-(trans-4-(2-(4-(1-propenyl)phenyl)ethyl)cyclohexyl)-1-(4-pentenyl)oxymethylbenzene
Compound No. 596
4-(trans-4-(4-(4-(2-propenyl)phenyl)butyl)cyclohexyl)-1-methyloxymethylbenzene
Compound No. 597
4-(trans-4-(4-(4-(1-pentenyl)phenyl)butyl)cyclohexyl)-1-methyloxy-n-propylbenzene
Compound No. 598
4-(trans-4-(4-(4-(2-pentenyl)phenyl)butyl)cyclohexyl)-1-methyloxy-n-pentylbenzene
Compound No. 599
4-(trans-4-(4-(4-(3-pentenyl)phenyl)butyl)cyclohexyl)-1-n-propyloxymethylbenzene
Compound No. 600
4-(trans-4-(4-(4-(4-pentenyl)phenyl)butyl)cyclohexyl)-1-n-pentyloxymethylbenzene
Compound No. 601
4-(trans-4-(4-(4-vinylphenyl)butyl)cyclohexyl)-1-(2-propenyl)oxymethylbenzene
Compound No. 602
4-(trans-4-(4-(4-(1-propenyl)phenyl)butyl)cyclohexyl)-1-(2-butenyl)oxymethylbenzene
Compound No. 603
4-(trans-4-(4-(4-(2-propenyl)phenyl)butyl)cyclohexyl)-1-(3-butenyl)oxymethylbenzene
Compound No. 604
4-(2-(trans-4-(2-(4-(1-propenyl)phenyl)ethynyl) cyclohexyl)ethyl)-1-methyloxymethylbenzene
Compound No. 605
4-(2-(trans-4-(2-(4-(2-pentenyl)phenyl)ethyl)cyclohexyl)ethyl)-1-methyloxy-n-propylbenzene
Compound No. 606
4-(2-(trans-4-(2-(4-(3-pentenyl)phenyl)ethyl)cyclohexyl)ethyl)-1-methyloxy-n-pentylbenzene
Compound No. 607
4-(2-(trans-4-(2-(4-vinylphenyl)ethyl)cyclohexyl)ethyl)-1-n-propyloxymethylbenzene
Compound No. 608
4-(2-(trans-4-(2-(4-(1-butenyl)phenyl)ethyl)cyclohexyl)ethyl)-1-n-pentyloxymethylbenzene
Compound No. 609
4-(2-(trans-4-(2-(4-(2-butenyl)phenyl)ethyl)cyclohexyl)ethyl)-1-(2-propenyl)oxymethylbenzene
Compound No. 610
4-(2-(trans-4-(2-(4-(1-propenyl)phenyl)ethyl)cyclohexyl)ethyl)-1-(2-butenyl)oxymethylbenzene
Compound No. 611
4-(2-(trans-4-(2-(4-(2-propenyl)phenyl)ethenyl) cyclohexyl)ethyl)-1-(3-butenyl)oxymethylbenzene
Compound No. 612
4-(2-(trans-4-(4-(4-(1-pentenyl)phenyl)butyl)cyclohexyl)ethyl)-1-methyloxymethylbenzene

Compound No. 613
4-(2-(trans-4-(4-(4-(2-pentenyl)phenyl)butyl)cyclohexyl)ethyl)-1-methyloxy-n-propylbenzene
Compound No. 614
4-(2-(trans-4-(4-(4-(3-pentenyl)phenyl)butyl)cyclohexyl)ethyl)-1-methyloxy-n-pentylbenzene
Compound No. 615
4-(2-(trans-4-(4-(4-(4-pentenyl)phenyl)butyl)cyclohexyl)ethyl)-1-n-propyloxymethylbenzene
Compound No. 616
4-(2-(trans-4-(4-(4-vinylphenyl)butyl)cyclohexyl)ethyl)-1-n-pentyloxymethylbenzene
Compound No. 617
4-(2-(trans-4-(4-(4-(1-propenyl)phenyl)butyl)cyclohexyl)ethyl)-1-(2-propenyl)oxymethylbenzene
Compound No. 618
4-(2-(trans-4-(4-(4-(2-propenyl)phenyl)butyl)cyclohexyl)ethyl)-1-(2-butenyl)oxymethylbenzene
Compound No. 619
4-(2-(trans-4-(4-(4-(1-pentenyl)phenyl)butyl)cyclohexyl)ethyl)-1-(3-butenyl)oxymethylbenzene
Compound No. 620
4-(4-(trans-4-(2-(4-(2-pentenyl)phenyl)ethyl)cyclohexyl)butyl)-1-methyloxymethylbenzene
Compound No. 621
4-(4-(trans-4-(2-(4-vinylphenyl)ethyl)cyclohexyl)butyl)-1-methyloxy-n-propylbenzene
Compound No. 622
4-(4-(trans-4-(2-(4-(1-propenyl)phenyl)ethyl)cyclohexyl)butyl)-1-methyloxy-n-pentylbenzene
Compound No. 623
4-(4-(trans-4-(2-(4-(2-propenyl)phenyl)ethyl)cyclohexyl)butyl)-1-n-propyloxymethylbenzene
Compound No. 624
4-(4-(trans-4-(2-(4-vinylphenyl)ethyl)cyclohexyl)butyl)-1-n-pentyloxymethylbenzene
Compound No. 625
4-(4-(trans-4-(2-(4-(1-pentenyl)phenyl)ethenyl) cyclohexyl)butyl)-1-(2-propenyl)oxymethylbenzene
Compound No. 626
4-(4-(trans-4-(2-(4-(2-pentenyl)phenyl)ethyl)cyclohexyl)butyl)-1-(2-butenyl)oxymethylbenzene
Compound No. 627
4-(4-(trans-4-(2-(4-(3-pentenyl)phenyl)ethyl)cyclohexyl)butyl)-1-(3-butenyl)oxymethylbenzene
Compound No. 628
4-(4-(trans-4-(4-(4-(4-pentenyl)phenyl)butyl)cyclohexyl)butyl)-1-methyloxymethylbenzene
Compound No. 629
4-(4-(trans-4-(4-(4-vinylphenyl)butyl)cyclohexyl)butyl)-1-methyloxy-n-propylbenzene
Compound No. 630
4-(4-(trans-4-(4-(4-(1-propenyl)phenyl)butyl)cyclohexyl)butyl)-1-methyloxy-n-pentylbenzene
Compound No. 631
4-(4-(trans-4-(4-(4-(2-propenyl)phenyl)butyl)cyclohexyl)butyl)-1-n-propyloxymethylbenzene
Compound No. 632
4-(4-(trans-4-(4-(4-(1-butenyl)phenyl)butyl)cyclohexyl)butyl)-n-pentyloxymethylbenzene
Compound No. 633
4-(4-(trans-4-(4-(4-(2-butenyl)phenyl)butyl)cyclohexyl)butyl)-(4-propenyl)oxymethylbenzene
Compound No. 634
4-(4-(trans-4-(4-(4-(1-pentenyl)phenyl)butyl)cyclohexyl)butyl)-1-(4-butenyl)oxymethylbenzene
Compound No. 635
4-(4-(trans-4-(4-(4-(4-pentenyl)phenyl)-2-butenyl)cyclohexyl)butyl)-1-(3-butenyl)oxymethylbenzene

Example 3

Synthesis of 4-(trans-4-(trans-4-vinylcyclohexyl) cyclohexyl)-1-n-propyloxymethylbenzene (Compound expressed by the general formula (1) wherein $R_1$ is n-$C_3H_7$, $R_2$ is -$CH_2$-, $R_3$ is vinyl group, n is 1, m is 0, ring A is 1,4-phenylene group, ring B and ring D are 1,4-cyclohexylene groups, $Z_1$ and $Z_2$ are single bond; Compound No. 636)

(First stage)

After a solution prepared by dissolving 94.2 g (0.6 mol) of bromobenzene in 100 ml of THF was added dropwise while stirring at room temperature to a liquid prepared by suspending 15.1 g (0.62 mol) of magnesium in 100 ml of THF, 300 ml of THF was added, heated up to 50°C, and then stirred for 1 hour. The reaction solution was cooled down to a

temperature lower than 0°C, and a solution prepared by dissolving 100.9 g (0.4 mol) of 8-(4-oxocyclohexyl)-1,5-dioxy-spiro[3,5]nonane in 300 ml of THF was added dropwise thereto at the same temperature in 30 min. After finishing of the dropping, the solution was raised up to 50°C, stirred for 1 hour, and then 100 ml of saturated aqueous solution of ammonium chloride was added to the reaction solution. A water layer separated from the reaction solution was extracted with isopropyl ether (twice, each time 1 ℓ). The extract was washed with saturated aqueous sodium chloride solution (thrice, each time 500 ml) and dried over anhydrous magnesium sulfate, and then the solvent was distilled off to obtain 151.4 g of 8-(4-hydroxy-4-phenylcyclohexyl)-1,5-dioxy-spiro[3,5]nonane.

(Second stage)

Ion exchange resin (Amberlist 15) in an amount of 7.5 g was added to a solution prepared by dissolving 151.4 g (0.4 mol) of the 8-(4-hydroxy-4-phenylcyclohexyl)-1,5-dioxy-spiro[3,5]nonane obtained by the first stage and heated to reflux while stirring for 4 hours, and then the ion exchange resin was separated from the reaction solution by filtration. The filtrate was washed with saturated aqueous sodium chloride solution (thrice, each time 200 ml), saturated aqueous solution of sodium bicarbonate (twice, each time 200 ml), and saturated aqueous sodium chloride solution (thrice, each time 200 ml) in turn and dried over anhydrous magnesium sulfate, and then the solvent was distilled off to obtain a crude product. The crude product thus obtained was purified by silica gel column chromatography (eluent: toluene) to obtain 97.6 g of 8-(4-phenyl-3-cyclohexenyl)-1,5-dioxy-spiro[3,5]nonane.

(Third stage)

The 8-(4-phenyl-3-cyclohexenyl)-1,5-dioxy-spiro[3,5]nonane obtained by the second stage in an amount of 97.6 g (0.31 mol) was dissolved in a mixed solvent of 500 ml of toluene and 1500 ml of ethanol, 39 g a Raney nickel catalyst was added to the solution, and then the spiro[3,5]nonane was subjected to a hydrogen-reduction at room temperature under a hydrogen pressure of 1 to 2 $Kg/cm^2$. After finishing of the reaction, the Raney nickel catalyst was separated by filtration, and the remaining reaction solution was concentrated to obtain 98.4 g of 8-(4-phenylcyclohexyl)-1,5-dioxy-spiro[3,5]nonane.

(Fourth stage)

The 8-(4-phenylcyclohexyl)-1,5-dioxy-spiro[3,5]nonane obtained by the third stage in an amount of 98.4 g (0.31 mol) was dissolved in 400 ml of toluene, 72.5 g (1.6 mol) of formic acid was added to the solution, and then the solution was heated to reflux for 3 hours. Toluene in an amount of 1 ℓ was added to the reaction solution, the solution was washed with saturated aqueous sodium chloride solution (thrice, each time 300 ml), saturated aqueous solution of sodium bicarbonate (twice, each time 300 ml), and saturated aqueous sodium chloride solution (twice, each time 300 ml) in turn, and dried over anhydrous magnesium, and then the solvent was distilled off to obtain a crude product. The crude product thus obtained was purified by silica gel column chromatography (eluent: toluene) and recrystallized from 80 ml of a mixed solvent (heptane/ethyl acetate = 7/3) to obtain 33.9 g of (4-(trans-4-phenyl)cyclohexyl)cyclohexanone.

(Fifth stage)

Solution prepared by dissolving 73.2 g (0.29 mol) of (4-(trans-4-phenyl)cyclohexyl)cyclohexanone in 500 ml of THF was added dropwise under a condition cooled with ice to a solution prepared by dissolving 3.8 g (0.11 mol) of aluminum lithium hydride in 50 ml of THF, and then stirred at the same temperature for 2 hours. Ethyl acetate in an amount of 100 ml was added dropwise to the reaction solution in 15 min, and then stirred for 30 min. Further, 200 ml of 6N hydrochloric acid was added dropwise to the reaction solution in 1 hour, the precipitates thus produced were filtered off by using a Celite, and the remaining reaction solution was extracted with methylene chloride (thrice, each time 1 ℓ). The extract was washed with saturated aqueous sodium chloride solution (thrice, each time 500 ml) and dried over anhydrous magnesium sulfate, and then the solvent was distilled off to obtain 69,9 g of trans-4-(4-hydroxycyclohexyl)cyclohexylbenzene.

(Sixth stage)

The trans-4-(4-hydroxycyclohexyl)cyclohexylbenzene obtained by the fifth stage in an amount of 69.9 g (0.27 mol) was dissolved in 1 ℓ of toluene, 51.3 g (0.65 mol) of pyridine was added thereto, and then 42.5 g (0.54 mol) of acetyl chloride was added dropwise while heating at 70°C to the solution. Subsequently, the solution was stirred at the same temperature for 5 hours. After finishing of the reaction, the reaction solution was cooled with an ice bath, and 200 ml of water was added dropwise thereto. A water layer separated from the reaction solution was extracted with toluene (twice,

each time 500 ml). The extract was washed with saturated aqueous sodium chloride solution (thrice, each time 300 ml) and dried over anhydrous magnesium sulfate, and then the solvent was distilled off to obtain 84.8 g of trans-4-(4-acetyloxycyclohexyl)cyclohexylbenzene.

(Seventh stage)

To a solution prepared by dissolving 69.9 g (0.27 mol) of trans-4-(4-acetyloxycyclohexyl)cyclohexylbenzene in 120 ml of carbon tetrachloride was added 28.5 g (0.16 mol) of iodic acid, 37.7 g (0.15 mol) of iodine, 13.2 g (0.14 mol) of a concentrated sulfuric acid, 100 ml of water, and 700 ml of acetic acid in turn, and then heated to reflux for 8 hours. After finishing of the reaction, the reaction solution was poured into 1 ℓ of an ice water, and a water layer separated from the solution was extracted with toluene (thrice, each time 1 ℓ). The extract was washed with saturated aqueous sodium chloride solution (twice, each time 300 ml), an aqueous solution of sodium thiosulfate (twice, each time 300 ml), and saturated aqueous sodium chloride solution (thrice, each time 300 ml) in turn and dried over anhydrous magnesium sulfate, and then the solvent was distilled off to obtain a crude product. The crude product thus obtained was purified by silica gel column chromatography (eluent: toluene) to obtain 108.3 g of 4-(trans-4-(4-acetyloxycyclohexyl)cyclohexyl)-1-iodobenzene.

(Eighth stage)

The 4-(trans-4-(4-acetyloxycyclohexyl)cyclohexyl)-1-iodobenzene obtained by the seventh stage in an amount of 108.3 g (0.25 mol) was dissolved in 500 ml of DMF, 34.1 g (0.38 mol) of cuprous cyanide was added thereto, and then they were heated to reflux for 8 hours. Subsequently, 75.5 g (0.28 mol) of ferric chloride hexahydrate was added thereto and stirred at 50°C for 1 hour. Further, 1 ℓ of toluene was added to the reaction solution, and the precipitates were separated by filtration by using a Celite. Remaining filtrate was washed with saturated aqueous sodium chloride solution (twice, each time 300 ml), 300 ml of saturated aqueous solution of sodium thiosulfate, and saturated aqueous sodium chloride solution (thrice, each time 300 ml) in turn and dried over anhydrous magnesium sulfate, and the solvent was distilled off to obtain 76.0 g of 4-(trans-4-(4-acetyloxycyclohexyl)cyclohexyl)-1-cyanobenzene.

(Ninth stage)

Sulfuric acid in an amount of 0.2 ml was added to a solution prepared by dissolving 76.0 g (0.23 mol) of the 4-(trans-4-(4-acetyloxycyclohexyl)cyclohexyl)-1-cyanobenzene obtained by the eighth stage in 500 ml of methanol, and heated to reflux for 7 hours. After finishing of the reaction, the solvent was distilled off under a reduced pressure from the reaction solution, and 2 ℓ of methylene chloride was added thereto to dissolve the residue. This solution was washed with an aqueous solution of sodium bicarbonate (twice, each time 300 ml) and saturated aqueous sodium chloride solution (thrice, each time 300 ml) in turn, and dried over anhydrous magnesium sulfate, and then the solvent was distilled off to obtain 70.1 g of 4-(trans-4-(4-hydroxycyclohexyl) cyclohexyl)-1-cyanobenzene.

(Tenth stage)

Solution prepared by dissolving 70.1 g (0.23 mol) of the 4-(trans-4-(4-hydroxycyclohexyl)cyclohexyl)-1-cyanobenzene obtained by the ninth stage in 1 ℓ of methylene chloride was added while stirring at room temperature to a solution prepared by dissolving a mixture of 50.2 g (0.28 mol) of pyridinium chlorochromate and 60 g of silica gel in 300 ml of methylene chloride, and stirred at the same temperature for 24 hours. The silica gel was separated from the reaction solution by filtration, and from the residual solution, the solvent was distilled off under a reduced pressure to obtain a crude product. The crude product was purified by silica gel column chromatography (eluent: toluene/ethyl acetate = 3/1) to obtain 54.8 g of 4-(trans-4-(4-oxycyclohexyl)cycloheyxl)-1-cyanobenzene.

(Eleventh stage)

Potassium t-butoxide in an amount of 1.5 g (0.28 mol) was added while stirring to a solution prepared by dissolving 86.8 g (0.25 mol) of methoxymethyltriphenylphosphine chloride in 200 ml of THF, cooled down to -50°C, and stirred for 2 hours. To the solution thus obtained, a solution prepared by dissolving 754.8 g (0.20 mol) of the 4-(trans-4-(4-cyanophenyl)cyclohexyl) cyclohexane obtained by the tenth stage in 500 ml of THF was added at the same temperature in 1 hour. The reaction solution was warmed up to room temperature in 3 hours, and 200 ml of water was added thereto. Water layer separated from the reaction solution was extracted with toluene (thrice, each time 300 ml). The extract was washed with saturated aqueous sodium chloride solution (thrice, each time 300 ml) and dried over anhydrous magnesium sulfate, and then the solvent was distilled off to obtain a crude product. The crude product thus obtained was purified by silica gel column chromatography (eluent: toluene) to obtain 56.4 g of 4-(trans-4-(4-methoxymethylenecyclohexyl)cyclohexyl)-1-cyanobenzene.

(Twelfth stage)

Formic acid in an amount of 84.2 g (1.8 mol) was added to a solution prepared by dissolving 56.4 g (0.18 mol) of 4-(trans-4-(4-methoxymethylenecyclohexyl)cyclohexyl)-1-cyanobenzene in 200 ml of toluene, and heated to reflux for 1 hour. After 700 ml of toluene was added to the reaction solution, the solution was washed with saturated aqueous sodium chloride solution (fifth times, each time 300 ml), and dried over anhydrous magnesium sulfate, and then the solvent was distilled off to obtain 52.1 g of 4-(trans-4-(formylcyclohexyl)cyclohexyl)-1-cyanobenzene.

(Thirteenth stage)

Potassium t-butoxide in an amount of 27.2 g (0.18 mol) was added to a solution prepared by dissolving 71.8 g (0.12 mol) of methyltriphenylphosphine iodide in 200 ml of THF, cooled down to -50°C, and then stirred at the same time for 2 hours. Then, a solution prepared by dissolving 35.0 g (0.12 mol) of the 4-(trans-4-(4-formylcyclohexyl)cyclohexyl)-1-cyanobenzene obtained by the twelfth stage in 350 ml of THF was added dropwise thereto in 1 hour. Subsequently, it was gradually warmed up to room temperature while stirring in 3 hours. After 250 ml of water was added to the reaction solution, the reaction product was extracted with toluene (thrice, each time 500 ml). The extract was washed with saturated aqueous sodium chloride solution (thrice, each time 300 ml) and dried over anhydrous magnesium sulfate, and then the solvent was distilled off under a reduced pressure to obtain a crude product. The crude product thus obtained was purified by silica gel column chromatography (eluent: toluene) to obtain 31.6 g of 4-(trans-4-(4-vinylcyclohexyl)cyclohexyl-1-cyanobenzene.

(Fourteenth stage)

After 30.4 g (0.10 mol) of the 4-(trans-4-(4-vinylcyclohexyl)cyclohexyl)-1-cyanobenzene obtained by the thirteenth stage was dissolved in 300 ml of toluene and cooled down to -70°C, 160 ml of diisobutyl aluminum hydride (1M toluene solution) was added dropwise thereto in 1 hour and stirred at the same temperature for 2 hours. After 20 ml of an aqueous solution of ammonium chloride and 100 ml of water were added to the reaction solution to terminate the reaction, 500 ml of toluene and 200 ml of 6N hydrochloric acid were added thereto in turn. Organic layer separated from the reaction solution was washed with saturated aqueous sodium chloride solution (thrice, each time 300 ml) and dried over anhydrous magnesium sulfate, and then the solvent was distilled off to obtain 27.2 g of 4-(trans-4-(4-vinylcyclohexyl)cyclohexyl)-1-benzaldehyde.

(Fifteenth stage)

After 5.2 g of sodium boron hydride was dissolved in 100 ml of isopropanol and cooled down to 0°C, a solution prepared by dissolving 27.2 g (0.092 mol) of the 4-(trans-4-(4-vinylcyclohexyl)cycloheyxl)-1-benzaldehyde in a mixed solvent of 400 ml of isopropanol and 200 ml of toluene was added dropwise thereto at the same temperature in 2 hours and further stirred for 1 hour. Then, 400 ml of 6N hydrochloric acid was added to the reaction solution to terminate the reaction. The reaction solution was concentrated and extracted with toluene (thrice, each time 300 ml). The extract was washed with saturated aqueous sodium chloride solution (thrice, each time 300 ml) and dried over anhydrous magnesium sulfate, and then the solvent was distilled off to obtain a crude product. The crude product thus obtained was purified by silica gel column chromatography (eluent: ethyl acetate) and recrystallized from 100 ml of n-heptane to obtain 9.0 g of 4-(trans-4-(trans-4-vinylcyclohexyl)cyclohexyl)-1-hydroxymethylbenzene.

(Sixteen stage)

n-Propyl bromide in an amount of 5.0 g (0.04 mol) and 0.1 g of potassium iodide were added to a solution prepared by dissolving 3.0 g (0.01 mol) of the 4-(trans-4-(trans-4-vinylcyclohexyl)cyclohexyl)-1-hydroxymethylbenzene in 50 ml of DMF, and 0.6 g (0.015 mol) of sodium hydride was added thereto while stirring at room temperature in 15 min. After 300 ml of toluene was added to the reaction solution, 100 ml of an ice water was poured thereto to terminate the reaction. Water layer separated from the reaction solution was extracted with toluene (thrice, each time 300 ml). After the extract was washed with saturated aqueous sodium chloride solution (thrice, each time 300 ml) and dried over anhydrous magnesium sulfate, the solvent was distilled off to obtain a crude product. The crude product thus obtained was purified by silica gel column chromatography (eluent: toluene) and recrystallized from 10 ml of ethanol to obtain 1.5 g of 4-(trans-4-(trans-4-vinylcyclohexyl)cyclohexyl)-1-n-propyloxymethylbenzene.

This compound exhibited liquid crystallinity, and its $S_B$-I point was 96.1 to 97.5°C. Besides, various kind of spectrum data well supported its structure.

According to the preparation method described above, the following compounds can be prepared.

Compound No. 637
4-(trans-4-(trans-4-vinylcyclohexyl)cyclohexyl)-1-methyloxymethylbenzene
Compound No. 638
4-(trans-4-(trans-4-vinylcyclohexyl)cyclohexyl)-1-ethyloxymethylbenzene
Compound No. 639
4-(trans-4-(trans-4-vinylcyclohexyl)cyclohexyl)-1-methyloxy-n-propylbenzene
Compound No. 640
4-(trans-4-(trans-4-vinylcyclohexyl)cyclohexyl)-4-ethyloxymethylbenzene
Compound No. 641
3-fluoro-4-(trans-4-(trans-4-vinylcyclohexyl)cyclohexyl)-1-n-propyloxymethylbenzene
Compound No. 642
4-(trans-4-(trans-4-vinylcyclohexyl)cycloheyxl)-1-n-butyloxymethylbenzene
Compound No. 643
4-(trans-4-(trans-4-(1-propenyl)cyclohexyl)cyclohexyl)-1-methyloxymethylbenzene
Compound No. 644
4-(trans-4-(trans-4-(1-propenyl)cyclohexyl)cyclohexyl)-1-ethyloxymethylbenzene
Compound No. 645
4-(trans-4-(trans-4-(1-propenyl)cyclohexyl)cyclohexyl)-1-n-propyloxymethylbenzene
Compound No. 646
4-(trans-4-(trans-4-(1-propenyl)cyclohexyl)cyclohexyl)-1-n-butyloxymethylbenzene
Compound No. 647
4-(trans-4-(trans-4-(1-propenyl)cyclohexyl)cyclohexyl)-1-n-pentyloxymethylbenzene
Compound No. 648
4-(trans-4-(trans-4-(2-propenyl)cyclohexyl)cyclohexyl)-1-methyloxymethylbenzene
Compound No. 649
4-(trans-4-(trans-4-(2-propenyl)cyclohexyl)cyclohexyl)-1-ethyloxymethylbenzene
Compound No. 650
4-(trans-4-(trans-4-(2-propenyl)cyclohexyl)cyclohexyl)-1-n-propyloxymethylbenzene
Compound No. 651
4-(trans-4-(trans-4-(2-propenyl)cyclohexyl)cyclohexyl)-1-n-butyloxymethylbenzene
Compound No. 652
4-(trans-4-(trans-4-(2-propenyl)cyclohexyl)cyclohexyl)-1-n-pentyloxymethylbenzene
Compound No. 653
4-(trans-4-(trans-4-(1-butenyl)cycloheyxl)cyclohexyl)-1-methyloxymethylbenzene
Compound No. 654
4-(trans-4-(trans-4-(1-butenyl)cycloheyxl)cyclohexyl)-1-ethyloxymethylbenzene
Compound No. 655
4-(trans-4-(trans-4-(1-butenyl)cycloheyxl)cyclohexyl)-1-n-propyloxymethylbenzene
Compound No. 656
4-(trans-4-(trans-4-(1-butenyl)cycloheyxl)cyclohexyl)-1-n-butyloxymethylbenzene
Compound No. 657
4-(trans-4-(trans-4-(1-butenyl)cycloheyxl)cyclohexyl)-1-n-pentyloxymethylbenzene
Compound No. 658
4-(trans-4-(trans-4-(1-butenyl)cycloheyxl)cyclohexyl)-1-(2-propenyl)oxymethylbenzene
Compound No. 659
4-(trans-4-(trans-4-(1-butenyl)cycloheyxl)cyclohexyl)-1-(2-butenyl)oxymethylbenzene
Compound No. 660
4-(trans-4-(trans-4-(1-butenyl)cycloheyxl)cyclohexyl)-1-(3-butenyl)oxymethylbenzene
Compound No. 661
4-(trans-4-(trans-4-(1-butenyl)cycloheyxl)cyclohexyl)-1-(2-pentenyl)oxymethylbenzene
Compound No. 662
4-(trans-4-(trans-4-(1-butenyl)cycloheyxl)cyclohexyl)-4-(3-pentenyl)oxymethylbenzene
Compound No. 663
4-(trans-1-(trans-4-(1-butenyl)cycloheyxl)cyclohexyl)-4-(4-pentenyl)oxymethylbenzene

Compound No. 664
4-(trans-4-(trans-4-(2-butenyl)cycloheyxl)cyclohexyl)-1-methyloxymethylbenzene
Compound No. 665
4-(trans-4-(trans-4-(2-butenyl)cycloheyxl)cyclohexyl)-1-ethyloxymethylbenzene
Compound No. 666
4-(trans-4-(trans-4-(2-butenyl)cycloheyxl)cyclohexyl)-1-n-propyloxymethylbenzene
Compound No. 667
4-(trans-4-(trans-4-(2-butenyl)cycloheyxl)cyclohexyl)-1-n-butyloxymethylbenzene
Compound No. 668
4-(trans-4-(trans-4-(2-butenyl)cycloheyxl)cyclohexyl)-1-n-pentyloxymethylbenzene
Compound No. 669
4-(trans-4-(trans-4-(2-butenyl)cycloheyxl)cyclohexyl)-1-(2-propenyl)oxymethylbenzene
Compound No. 670
3-fluoro-4-(trans-4-(trans-4-(2-butenyl)cycloheyxl)cyclohexyl)-1-(2-butenyl)oxymethylbenzene
Compound No. 671
4-(trans-4-(trans-4-(2-butenyl)cycloheyxl)cyclohexyl)-1-(3-butenyl)oxymethylbenzene
Compound No. 672
4-(trans-4-(trans-4-(2-butenyl)cycloheyxl)cyclohexyl)-1-(2-pentenyl)oxymethylbenzene
Compound No. 673
4-(trans-4-(trans-4-(2-butenyl)cycloheyxl)cyclohexyl)-1-(3-pentenyl)oxymethylbenzene
Compound No. 674
4-(trans-4-(trans-4-(2-butenyl)cycloheyxl)cyclohexyl)-1-(4-pentenyl)oxymethylbenzene
Compound No. 675
4-(trans-4-(trans-4-(3-butenyl)cycloheyxl)cyclohexyl)-1-methyloxymethylbenzene
Compound No. 676
4-(trans-4-(trans-4-(3-butenyl)cycloheyxl)cyclohexyl)-1-ethyloxymethylbenzene
Compound No. 677
4-(trans-4-(trans-4-(3-butenyl)cycloheyxl)cyclohexyl)-1-n-propyloxymethylbenzene
Compound No. 678
4-(trans-4-(trans-4-(3-butenyl)cycloheyxl)cyclohexyl)-1-n-butyloxymethylbenzene
Compound No. 679
4-(trans-4-(trans-4-(3-butenyl)cycloheyxl)cyclohexyl)-1-n-pentyloxymethylbenzene
Compound No. 680
4-(trans-4-(trans-4-(3-butenyl)cycloheyxl)cyclohexyl)-1-(2-propenyl)oxymethylbenzene
Compound No. 681
2,3-difluoro-4-(trans-4-(trans-4-(3-butenyl)cycloheyxl) cyclohexyl)-1-(2-butenyl)oxymethylbenzene
Compound No. 682
4-(trans-4-(trans-4-(3-butenyl)cycloheyxl)cyclohexyl)-1-(3-butenyl)oxymethylbenzene
Compound No. 683
4-(trans-4-(trans-4-(3-butenyl)cycloheyxl)cyclohexyl)-1-(2-pentenyl)oxymethylbenzene
Compound No. 684
4-(trans-4-(trans-4-(3-butenyl)cycloheyxl)cyclohexyl)-1-(3-pentenyl)oxymethylbenzene
Compound No. 685
4-(trans-4-(trans-4-(3-butenyl)cycloheyxl)cyclohexyl)-1-(4-pentenyl)oxymethylbenzene
Compound No. 686
4-(trans-4-(trans-4-(1-pentenyl)cycloheyxl)cyclohexyl)-1-methyloxymethylbenzene
Compound No. 687
4-(trans-4-(trans-4-(1-pentenyl)cycloheyxl)cyclohexyl)-1-ethyloxymethylbenzene
Compound No. 688
4-(trans-4-(trans-4-(1-pentenyl)cycloheyxl)cyclohexyl)-1-n-propyloxymethylbenzene
Compound No. 689
4-(trans-4-(trans-4-(1-pentenyl)cycloheyxl)cyclohexyl)-1-n-butyloxymethylbenzene
Compound No. 690
4-(trans-4-(trans-4-(1-pentenyl)cycloheyxl)cyclohexyl)-1-n-pentyloxymethylbenzene
Compound No. 691
4-(trans-4-(trans-4-(1-pentenyl)cycloheyxl)cyclohexyl)-1-(2-propenyl)oxymethylbenzene
Compound No. 692
4-(trans-4-(trans-4-(1-pentenyl)cycloheyxl)cyclohexyl)-1-(2-butenyl)oxymethylbenzene

Compound No. 693
4-(trans-4-(trans-4-(1-pentenyl)cycloheyxl)cyclohexyl)-1-(3-butenyl)oxymethylbenzene
Compound No. 694
4-(trans-4-(trans-4-(1-pentenyl)cycloheyxl)cyclohexyl)-1-(2-pentenyl)oxymethylbenzene
Compound No. 695
4-(trans-4-(trans-4-(1-pentenyl)cycloheyxl)cyclohexyl)-1-(3-pentenyl)oxymethylbenzene
Compound No. 696
4-(trans-4-(trans-4-(1-pentenyl)cycloheyxl)cyclohexyl)-1-(4-pentenyl)oxymethylbenzene
Compound No. 697
4-(trans-4-(trans-4-(2-pentenyl)cycloheyxl)cyclohexyl)-1-methyloxymethylbenzene
Compound No. 698
4-(trans-4-(trans-4-(2-pentenyl)cycloheyxl)cyclohexyl)-1-ethyloxymethylbenzene
Compound No. 699
4-(trans-4-(trans-4-(2-pentenyl)cycloheyxl)cyclohexyl)-1-n-propyloxymethylbenzene
Compound No. 700
4-(trans-4-(trans-4-(2-pentenyl)cycloheyxl)cyclohexyl)-1-n-butyloxymethylbenzene
Compound No. 701
4-(trans-4-(trans-4-(2-pentenyl)cycloheyxl)cyclohexyl)-1-n-pentyloxymethylbenzene
Compound No. 702
4-(trans-4-(trans-4-(2-pentenyl)cycloheyxl)cyclohexyl)-1-(2-propenyl)oxymethylbenzene
Compound No. 703
4-(trans-4-(trans-4-(2-pentenyl)cycloheyxl)cyclohexyl)-1-(2-butenyl)oxymethylbenzene
Compound No. 704
4-(trans-4-(trans-4-(2-pentenyl)cycloheyxl)cyclohexyl)-1-(3-butenyl)oxymethylbenzene
Compound No. 705
4-(trans-4-(trans-4-(2-pentenyl)cycloheyxl)cyclohexyl)-1-(2-pentenyl)oxymethylbenzene
Compound No. 706
4-(trans-4-(trans-4-(2-pentenyl)cycloheyxl)cyclohexyl)-1-(3-pentenyl)oxymethylbenzene
Compound No. 707
4-(trans-4-(trans-4-(2-pentenyl)cycloheyxl)cyclohexyl)-1-(4-pentenyl)oxymethylbenzene
Compound No. 708
4-(trans-4-(trans-4-(3-pentenyl)cycloheyxl)cyclohexyl)-1-methyloxymethylbenzene
Compound No. 709
4-(trans-4-(trans-4-(3-pentenyl)cycloheyxl)cyclohexyl)-1-ethyloxymethylbenzene
Compound No. 710
4-(trans-4-(trans-4-(3-pentenyl)cycloheyxl)cyclohexyl)-1-n-propyloxymethylbenzene
Compound No. 711
3-fluoro-4-(trans-4-(trans-4-(3-pentenyl)cycloheyxl)cyclohexyl)-1-n-butyloxymethylbenzene
Compound No. 712
4-(trans-4-(trans-4-(3-pentenyl)cycloheyxl)cyclohexyl)-1-n-pentyloxymethylbenzene
Compound No. 713
4-(trans-4-(trans-4-(4-pentenyl)cycloheyxl)cyclohexyl)-1-methyloxymethylbenzene
Compound No. 714
4-(trans-4-(trans-4-(4-pentenyl)cycloheyxl)cyclohexyl)-1-ethyloxymethylbenzene
Compound No. 715
4-(trans-4-(trans-4-(4-pentenyl)cycloheyxl)cyclohexyl)-1-n-propyloxymethylbenzene
Compound No. 716
4-(trans-4-(trans-4-(4-pentenyl)cycloheyxl)cyclohexyl)-1-n-butyloxymethylbenzene
Compound No. 717
4-(trans-4-(trans-4-(4-pentenyl)cycloheyxl)cyclohexyl)-1-n-pentyloxymethylbenzene
Compound No. 718
4-(trans-4-(2-(trans-4-vinylcyclohexyl)ethyl)cyclohexyl)-1-methoxymethylbenzene
Compound No. 719
4-(trans-4-(2-(trans-4-vinylcyclohexyl)ethyl)cyclohexyl)-1-n-propyloxymethylbenzene
Compound No. 720
4-(trans-4-(2-(trans-4-vinylcyclohexyl)ethyl)cyclohexyl)-1-methoxy-n-propylbenzene
Compound No. 721
4-(trans-4-(2-(trans-4-(1-propenyl)cyclohexyl)ethyl)cyclohexyl)-1-n-propyloxymethylbenzene

Compound No. 722
4-(trans-4-(2-(trans-4-(2-propenyl)cyclohexyl)ethyl)cyclohexyl)-1-n-pentyloxymethylbenzene
Compound No. 723
4-(trans-4-(2-(trans-4-(4-pentenyl)cyclohexyl)ethyl)cyclohexyl)-1-n-propyloxymethylbenzene
Compound No. 724
4-(trans-4-(2-(trans-4-(3-butenyl)cyclohexyl)ethyl)cyclohexyl)-1-n-propyloxymethylbenzene
Compound No. 725
4-(trans-4-(2-(trans-4-(2-butenyl)cyclohexyl)ethyl)cyclohexyl)-1-n-pentyloxymethylbenzene
Compound No. 726
4-(trans-4-(4-(trans-4-(2-propenyl)cyclohexyl)butyl)cyclohexyl)-1-methoxy-n-propylbenzene
Compound No. 727
4-(trans-4-(4-(trans-4-vinylcyclohexyl)butyl)cyclohexyl)-1-methoxy-n-propylbenzene
Compound No. 728
4-(trans-4-(4-(trans-4-(2-propenyl)cyclohexyl)butyl)cyclohexyl)-1-methoxymethylbenzene
Compound No. 729
4-(2-(trans-4-(trans-4-(1-propenyl)cyclohexyl)cycloheyxl)ethyl)-1-methoxymethylbenzene
Compound No. 730
4-(2-(trans-4-(trans-4-vinylcyclohexyl)cyclohexyl)ethyl)-1-methoxy-n-propylbenzene
Compound No. 731
4-(2-(trans-4-(trans-4-(4-pentenyl)cyclohexyl)cyclohexyl)ethyl)-1-methoxy-n-pentylbenzene
Compound No. 732
4-(2-(trans-4-(trans-4-vinylcyclohexyl)cyclohexyl)ethyl)-1-n-propyloxymethylbenzene
Compound No. 733
4-(2-(trans-4-(trans-4-(2-butenyl)cyclohexyl)cyclohexyl)ethyl)-1-n-pentyloxymethylbenzene
Compound No. 734
4-(2-(trans-4-(trans-4-vinylcyclohexyl)cyclohexyl)ethyl)-1-n-propyloxymethylbenzene
Compound No. 735
4-(4-(trans-4-(trans-4-(2-propenyl)cyclohexyl)cyclohexyl)butyl)-1-methoxy-n-propylbenzene
Compound No. 736
4-(4-(trans-4-(trans-4-vinylcyclohexyl)cyclohexyl)butyl)-1-methoxy-n-propylbenzene
Compound No. 737
4-(4-(trans-4-(trans-4-(2-propenyl)cyclohexyl)cyclohexyl)butyl)-1-methoxymethylbenzene
Compound No. 738
4-(2-(trans-4-(2-(trans-4-vinylcyclohexyl)ethyl)cyclohexyl) ethyl)-1-methoxymethylbenzene
Compound No. 739
4-(2-(trans-4-(2-(trans-4-vinylcyclohexyl)ethyl)cyclohexyl) ethyl)-1-n-propyloxymethylbenzene
Compound No. 740
4-(2-(trans-4-(2-(trans-4-(3-butenyl)cyclohexyl)ethyl) cyclohexyl)ethyl)-1-ethyloxymethylbenzene
Compound No. 741
4-(2-(trans-4-(4-(trans-4-vinylcyclohexyl)ethyl)cyclohexyl)-2-butenyl)-1-methoxymethylbenzene
Compound No. 742
4-(2-(trans-4-(4-(trans-4-(2-propenyl)cyclohexyl)ethyl) cyclohexyl)butyl)-1-n-propyloxymethylbenzene
Compound No. 743
2-fluoro-4-(trans-4-(trans-4-vinylcyclohexyl)cyclohexyl)-1-methoxymethylbenzene
Compound No. 744
3-fluoro-4-(trans-4-(trans-4-(3-pentenyl)cyclohexyl)-1-n-propyloxymethylbenzene
Compound No. 745
4-(2-(trans-4-(4-(trans-4-vinylcyclohexyl)ethyl)cyclohexyl) butyl)-1-n-propyloxymethylbenzene
Compound No. 746
4-(4-(trans-4-(2-(trans-4-(3-butenyl)cyclohexyl)butyl) cyclohexyl)ethyl)-1-methoxymethylbenzene
Compound No. 747
4-(4-(trans-4-(2-(trans-4-vinylcyclohexyl)butyl)cyclohexyl) ethyl)-1-n-pentyloxymethylbenzene
Compound No. 748
4-(4-(trans-4-(2-(trans-4-(2-propenyl)cycloheyxl)ethenyl) cyclohexyl)ethyl)-1-methoxymethylbenzene
Compound No. 749
4-(4-(trans-4-(4-(trans-4-vinylcyclohexyl)butyl) cyclohexyl)butyl)-1-n-propyloxymethylbenzene
Compound No. 750
4-(4-(trans-4-(4-(trans-4-(1-pentenyl)cyclohexyl)butyl) cyclohexyl)butyl)-1-methoxymethylbenzene

Compound No. 751
4-(4-(trans-4-(4-(trans-4-vinylcyclohexyl)butyl) cyclohexyl)butyl)-1-methoxy-n-propylbenzene
Compound No. 752
4-(4-(trans-4-(4-(trans-4-(1-propenyl)cyclohexyl)butyl) cyclohexyl)butyl)-1-methoxymethylbenzene
Compound No. 753
4-(trans-4-(trans-4-vinylcyclohexyl)cyclohexyl)difluoromethoxy)-1-methoxymethylbenzene
Compound No. 754
4-(trans-4-(trans-4-vinylcyclohexyl)cyclohexyloxydifluoromethyl) -1-n-propyloxymethylbenzene
Compound No. 755
4-(trans-4-(trans-4-(1-propenyl)cyclohexyloxycarbonyl) cyclohexyl)-1-n-propyloxymethylbenzene
Compound No. 756
4-(trans-4-(trans-4-(2-propenyl)cyclohexylcarbonyloxy) cyclohexyl)-1-methoxymethylbenzene
Compound No. 757
4-(trans-4-(trans-4-vinylcyclohexyl)cyclohexyloxycarbonyl)-1-n-propyloxymethylbenzene
Compound No. 758
4-(trans-4-(trans-4-(2-propenyl)cyclohexyl) cyclohexylcarbonyloxy)-1-methoxymethylbenzene

Example 4

Synthesis of 4-(trans-4-(trans-4-vinylcyclohexyl) cyclohexyl)-1-(2-propenyl)oxymethylbenzene (Compound expressed by the general formula (1) wherein $R_1$ is 1-propenyl group, $R_2$ is $-CH_2-$, $R_3$ is vinyl group, n is 1, m is 0, ring A is 1,4-phenylene group, rings B and D are 1,4-cyclohexylene group, and $Z_1$ and $Z_2$ are single bond; Compound No. 759)

To a solution prepared by dissolving 3.0 g (0.01 mol) of the 4-(trans-4-(trans-4-vinylcyclohexyl)cyclohexyl)-1-hydroxymethylbenzene obtained by the fifteenth stage in Example 3 in 50 ml of DMF were added 4.9 g (0.04 mol) of 1-propenyl bromide and 0.1 g of potassium iodide, and 0.6 g (0.01 mol) of sodium hydride was added thereto while stirring at room temperature in 15 min. After 300 ml of toluene was added to the reaction solution, 100 ml of an ice water was added thereto to terminate the reaction. Water layer separated from the reaction solution was extracted with toluene (thrice, each time 300 ml). After the extract was washed with saturated aqueous sodium chloride solution (thrice, each time 300 ml) and dried over anhydrous magnesium sulfate, the solvent was distilled off to obtain a crude product. The crude product thus obtained was purified by silica gel column chromatography (eluent: toluene) and then recrystallized from 10 ml of ethanol to obtain 0.92 g of 4-(trans-4-(trans-4-vinylcyclohexyl)cyclohexyl)-1-(2-propenyl)oxymethylbenzene.

This compound exhibited liquid crystallinity, and its $S_B$-I point was 110.3 ~ 112.7°C. Besides, various kind of spectrum date well supported its structure.

According to the preparation method described above, the following compounds can be prepared.

Compound No. 760
4-(trans-4-(trans-4-vinylcyclohexyl)cyclohexyl)-1-(2-butenyl)oxymethylbenzene
Compound No. 761
4-(trans-4-(trans-4-vinylcyclohexyl)cyclohexyl)-1-(3-butenyl)oxymethylbenzene
Compound No. 762
4-(trans-4-(trans-4-vinylcyclohexyl)cyclohexyl)-1-(2-pentenyl)oxymethylbenzene
Compound No. 763
4-(trans-4-(trans-4-vinylcyclohexyl)cyclohexyl)-1-(3-pentenyl)oxymethylbenzene
Compound No. 764
4-(trans-4-(trans-4-vinylcyclohexyl)cyclohexyl)-1-(4-pentenyl)oxymethylbenzene
Compound No. 765
4-(trans-4-(trans-4-(1-propenyl)cyclohexyl)cyclohexyl)-1-(2-propenyl)oxymethylbenzene
Compound No. 766
4-(trans-4-(trans-4-(1-propenyl)cyclohexyl)cyclohexyl)-1-(2-butenyl)oxymethylbenzene
Compound No. 767
4-(trans-4-(trans-4-(1-propenyl)cyclohexyl)cyclohexyl)-1-(3-butenyl)oxymethylbenzene
Compound No. 768
2-fluoro-4-(trans-4-(trans-4-(1-propenyl)cyclohexyl)cyclohexyl)-1-(2-pentenyl)oxymethylbenzene
Compound No. 769
4-(trans-4-(trans-4-(1-propenyl)cyclohexyl)cyclohexyl)-1-(3-pentenyl)oxymethylbenzene
Compound No. 770

4-(trans-4-(trans-4-(1-propenyl)cyclohexyl)cyclohexyl)-1-(4-pentenyl)oxymethylbenzene
Compound No. 771
4-(trans-4-(trans-4-(2-propenyl)cyclohexyl)cyclohexyl)-1-(2-propenyl)oxymethylbenzene
Compound No. 772
4-(trans-4-(trans-4-(2-propenyl)cyclohexyl)cyclohexyl)-1-(2-butenyl)oxymethylbenzene
Compound No. 773
4-(trans-4-(trans-4-(2-propenyl)cyclohexyl)cyclohexyl)-1-(3-butenyl)oxymethylbenzene
Compound No. 774
4-(trans-4-(trans-4-(2-propenyl)cyclohexyl)cyclohexyl)-1-(2-pentenyl)oxymethylbenzene
Compound No. 775
4-(trans-4-(trans-4-(2-propenyl)cyclohexyl)cyclohexyl)-1-(3-pentenyl)oxymethylbenzene
Compound No. 776
4-(trans-4-(trans-4-(2-propenyl)cyclohexyl)cyclohexyl)-1-(4-pentenyl)oxymethylbenzene
Compound No. 777
4-(trans-4-(trans-4-(1-butenyl)cyclohexyl)cyclohexyl)-1-(2-propenyl)oxymethylbenzene
Compound No. 778
4-(trans-4-(trans-4-(1-butenyl)cyclohexyl)cyclohexyl)-1-(2-butenyl)oxymethylbenzene
Compound No. 779
4-(trans-4-(trans-4-(1-butenyl)cyclohexyl)cyclohexyl)-1-(3-butenyl)oxymethylbenzene
Compound No. 780
4-(trans-4-(trans-4-(1-butenyl)cyclohexyl)cyclohexyl)-1-(2-pentenyl)oxymethylbenzene
Compound No. 781
4-(trans-4-(trans-4-(1-butenyl)cyclohexyl)cyclohexyl)-1-(3-pentenyl)oxymethylbenzene
Compound No. 782
4-(trans-4-(trans-4-(1-butenyl)cyclohexyl)cyclohexyl)-1-(4-pentenyl)oxymethylbenzene
Compound No. 783
4-(trans-4-(trans-4-(2-butenyl)cyclohexyl)cyclohexyl)-1-(2-propenyl)oxymethylbenzene
Compound No. 784
4-(trans-4-(trans-4-(2-butenyl)cyclohexyl)cyclohexyl)-1-(2-butenyl)oxymethylbenzene
Compound No. 785
4-(trans-4-(trans-4-(2-butenyl)cyclohexyl)cyclohexyl)-1-(3-butenyl)oxymethylbenzene
Compound No. 786
4-(trans-4-(trans-4-(2-butenyl)cyclohexyl)cyclohexyl)-1-(2-pentenyl)oxymethylbenzene
Compound No. 787
4-(trans-4-(trans-4-(2-butenyl)cyclohexyl)cyclohexyl)-1-(3-pentenyl)oxymethylbenzene
Compound No. 788
4-(trans-4-(trans-4-(2-butenyl)cyclohexyl)cyclohexyl)-1-(4-pentenyl)oxymethylbenzene
Compound No. 789
4-(trans-4-(trans-4-(3-butenyl)cyclohexyl)cyclohexyl)-1-(2-propenyl)oxymethylbenzene
Compound No. 790
4-(trans-4-(trans-4-(3-butenyl)cyclohexyl)cyclohexyl)-1-(2-butenyl)oxymethylbenzene
Compound No. 791
4-(trans-4-(trans-4-(3-butenyl)cyclohexyl)cyclohexyl)-1-(3-butenyl)oxymethylbenzene
Compound No. 792
4-(trans-4-(trans-4-(3-butenyl)cyclohexyl)cyclohexyl)-1-(2-pentenyl)oxymethylbenzene
Compound No. 793
4-(trans-4-(trans-4-(3-butenyl)cyclohexyl)cyclohexyl)-1-(3-pentenyl)oxymethylbenzene
Compound No. 794
4-(trans-4-(trans-4-(3-butenyl)cyclohexyl)cyclohexyl)-1-(4-pentenyl)oxymethylbenzene
Compound No. 795
4-(trans-4-(trans-4-(1-pentenyl)cyclohexyl)cyclohexyl)-1-(2-propenyl)oxymethylbenzene
Compound No. 796
4-(trans-4-(trans-4-(1-pentenyl)cyclohexyl)cyclohexyl)-1-(2-butenyl)oxymethylbenzene
Compound No. 797
4-(trans-4-(trans-4-(1-pentenyl)cyclohexyl)cyclohexyl)-1-(3-butenyl)oxymethylbenzene
Compound No. 798
4-(trans-4-(trans-4-(1-pentenyl)cyclohexyl)cyclohexyl)-1-(2-pentenyl)oxymethylbenzene
Compound No. 799

4-(trans-4-(trans-4-(1-pentenyl)cyclohexyl)cyclohexyl)-1-(3-pentenyl)oxymethylbenzene
Compound No. 800
4-(trans-4-(trans-4-(1-pentenyl)cyclohexyl)cyclohexyl)-1-(4-pentenyl)oxymethylbenzene
Compound No. 801
4-(trans-4-(trans-4-(2-pentenyl)cyclohexyl)cyclohexyl)-1-(2-propenyl)oxymethylbenzene
Compound No. 802
4-(trans-4-(trans-4-(2-pentenyl)cyclohexyl)cyclohexyl)-1-(2-butenyl)oxymethylbenzene
Compound No. 803
4-(trans-4-(trans-4-(2-pentenyl)cyclohexyl)cyclohexyl)-1-(3-butenyl)oxymethylbenzene
Compound No. 804
4-(trans-4-(trans-4-(2-pentenyl)cyclohexyl)cyclohexyl)-1-(2-pentenyl)oxymethylbenzene
Compound No. 805
4-(trans-4-(trans-4-(2-pentenyl)cyclohexyl)cyclohexyl)-1-(3-pentenyl)oxymethylbenzene
Compound No. 806
4-(trans-4-(trans-4-(2-pentenyl)cyclohexyl)cyclohexyl)-1-(4-pentenyl)oxymethylbenzene
Compound No. 807
4-(trans-4-(trans-4-(3-pentenyl)cyclohexyl)cyclohexyl)-1-(2-propenyl)oxymethylbenzene
Compound No. 808
4-(trans-4-(trans-4-(3-pentenyl)cyclohexyl)cyclohexyl)-1-(2-butenyl)oxymethylbenzene
Compound No. 809
4-(trans-4-(trans-4-(3-pentenyl)cyclohexyl)cyclohexyl)-1-(3-butenyl)oxymethylbenzene
Compound No. 810
4-(trans-4-(trans-4-(3-pentenyl)cyclohexyl)cyclohexyl)-1-(2-pentenyl)oxymethylbenzene
Compound No. 811
4-(trans-4-(trans-4-(3-pentenyl)cyclohexyl)cyclohexyl)-1-(3-pentenyl)oxymethylbenzene
Compound No. 812
4-(trans-4-(trans-4-(3-pentenyl)cyclohexyl)cyclohexyl)-1-(4-pentenyl)oxymethylbenzene
Compound No. 813
4-(trans-4-(trans-4-(4-pentenyl)cyclohexyl)cyclohexyl)-1-(2-propenyl)oxymethylbenzene
Compound No. 814
4-(trans-4-(trans-4-(4-pentenyl)cyclohexyl)cyclohexyl)-1-(2-butenyl)oxymethylbenzene
Compound No. 815
4-(trans-4-(trans-4-(4-pentenyl)cyclohexyl)cyclohexyl)-1-(3-butenyl)oxymethylbenzene
Compound No. 816
4-(trans-4-(trans-4-(4-pentenyl)cyclohexyl)cyclohexyl)-1-(2-pentenyl)oxymethylbenzene
Compound No. 817
4-(trans-4-(trans-4-(4-pentenyl)cyclohexyl)cyclohexyl)-1-(3-pentenyl)oxymethylbenzene
Compound No. 818
4-(trans-4-(trans-4-(4-pentenyl)cyclohexyl)cyclohexyl)-1-(4-pentenyl)oxymethylbenzene
Compound No. 819
4-(trans-4-(2-(trans-4-vinylcyclohexyl)ethyl)cyclohexyl)-1-(2-propenyl)oxymethylbenzene
Compound No. 820
4-(trans-4-(2-(trans-4-vinylcyclohexyl)ethyl)cyclohexyl)-1-(2-butenyl)oxymethylbenzene
Compound No. 821
4-(trans-4-(2-(trans-4-vinylcyclohexyl)ethyl)cyclohexyl)-1-(3-butenyl)oxymethylbenzene
Compound No. 822
4-(trans-4-(2-(trans-4-vinylcyclohexyl)ethyl)cyclohexyl)-1-(2-pentenyl)oxymethylbenzene
Compound No. 823
4-(trans-4-(2-(trans-4-vinylcyclohexyl)ethyl)cyclohexyl)-1-(3-pentenyl)oxymethylbenzene
Compound No. 824
4-(trans-4-(2-(trans-4-vinylcyclohexyl)ethyl)cyclohexyl)-1-(4-pentenyl)oxymethylbenzene
Compound No. 825
4-(trans-4-(2-(trans-4-(1-propenyl)cyclohexyl)ethyl)cyclohexyl)-1-(2-propenyl)oxymethylbenzene
Compound No. 826
4-(trans-4-(2-(trans-4-(2-propenyl)cyclohexyl)ethyl)cyclohexyl)-1-(2-propenyl)oxymethylbenzene
Compound No. 827
4-(trans-4-(2-(trans-4-(4-pentenyl)cyclohexyl)ethyl)cyclohexyl)-1-(2-butenyl)oxymethylbenzene
Compound No. 828

4-(trans-4-(2-(trans-4-(3-butenyl)cyclohexyl)ethyl)cyclohexyl)-1-(2-propenyl)oxymethylbenzene
Compound No. 829
4-(trans-4-(2-(trans-4-(2-butenyl)cyclohexyl)ethyl)cyclohexyl)-1-(2-butenyl)oxymethylbenzene
Compound No. 830
4-(trans-4-(4-(trans-4-(2-propenyl)cyclohexyl)butyl)cyclohexyl)-1-(2-propenyl)oxy-n-propylbenzene
Compound No. 831
4-(trans-4-(4-(trans-4-vinylcyclohexyl)butyl)cyclohexyl)-1-(2-butenyl)oxy-n-propylbenzene
Compound No. 832
4-(trans-4-(4-(trans-4-(2-propenyl)cyclohexyl)butyl)cyclohexyl)-1-(2-propenyl)oxymethylbenzene
Compound No. 833
4-(2-(trans-4-(trans-4-(1-propenyl)cyclohexyl)cyclohexyl)ethyl)-1-(3-butenyl)oxymethylbenzene
Compound No. 834
4-(2-(trans-4-(trans-4-vinylcyclohexyl)cyclohexyl)ethyl)-1-(4-pentenyl)oxy-n-propylbenzene
Compound No. 835
4-(2-(trans-4-(trans-4-(4-pentenyl)cyclohexyl)cyclohexyl)ethyl)-1-(2-butenyl)oxymethylbenzene
Compound No. 836
4-(2-(trans-4-(trans-4-vinylcyclohexyl)cyclohexyl)ethyl)-1-(2-propenyl)oxymethylbenzene
Compound No. 837
4-(2-(trans-4-(trans-4-(2-butenyl)cyclohexyl)cyclohexyl)ethyl)-1-(2-propenyl)oxymethylbenzene
Compound No. 838
4-(4-(trans-4-(trans-4-(2-propenyl)cyclohexyl)cyclohexyl)butyl)-1-(2-propenyl)oxymethylbenzene
Compound No. 839
4-(4-(trans-4-(trans-4-vinylcyclohexyl)cyclohexyl)butyl)-1-(2-butenyl)oxymethylbenzene
Compound No. 840
4-(4-(trans-4-(trans-4-(2-propenyl)cyclohexyl)cyclohexyl)butyl)-1-(3-butenyl)oxymethylbenzene
Compound No. 841
4-(2-(trans-4-(2-(trans-4-vinylcyclohexyl)ethyl)cyclohexyl) ethyl)-1-(2-propenyl)oxymethylbenzene
Compound No. 842
4-(2-(trans-4-(2-(trans-4-vinylcyclohexyl)ethyl)cyclohexyl) ethyl)-1-(2-butenyl)oxymethylbenzene
Compound No. 843
4-(2-(trans-4-(2-(trans-4-(3-butenyl)cyclohexyl)ethyl) cyclohexyl)ethyl)-1-(3-butenyl)oxymethylbenzene
Compound No. 844
4-(2-(trans-4-(4-(trans-4-vinylcyclohexyl)ethyl)cyclohexyl)-2-butenyl)-1-(2-propenyl)oxymethylbenzene
Compound No. 845
4-(2-(trans-4-(4-(trans-4-(2-propenyl)cyclohexyl)ethyl) cyclohexyl)butyl)-1-(3-butenyl)oxymethylbenzene
Compound No. 846
2-fluoro-4-(trans-4-(trans-4-vinylcyclohexyl)cyclohexyl)-1-(2-propenyl)oxymethylbenzene
Compound No. 847
3-fluoro-4-(trans-4-(trans-4-(3-pentenyl)cyclohexyl)-1-(3-butenyl)oxymethylbenzene
Compound No. 848
4-(2-(trans-4-(4-(trans-4-vinylcyclohexyl)ethyl) cyclohexyl)butyl)-1-(2-pentenyl)oxymethylbenzene
Compound No. 849
4-(4-(trans-4-(2-(trans-4-(3-butenyl)cyclohexyl)butyl) cyclohexyl)ethyl)-1-(2-propenyl)oxymethylbenzene
Compound No. 850
4-(4-(trans-4-(2-(trans-4-vinylcyclohexyl)butyl) cyclohexyl)ethyl)-1-(2-butenyl)oxymethylbenzene
Compound No. 851
4-(4-(trans-4-(2-(trans-4-(2-propenyl)cyclohexyl)ethenyl) cyclohexyl)ethyl)-1-(4-pentenyl)oxymethylbenzene
Compound No. 852
4-(4-(trans-4-(4-(trans-4-vinylcyclohexyl)butyl) cyclohexyl)butyl)-1-(2-propenyl)oxymethylbenzene
Compound No. 853
4-(4-(trans-4-(4-(trans-4-(1-pentenyl)cyclohexyl)butyl) cyclohexyl)butyl)-1-(2-propenyl)oxymethylbenzene
Compound No. 854
4-(4-(trans-4-(4-(trans-4-(1-propenyl)cyclohexyl)butyl) cyclohexyl)butyl)-1-(3-butenyl)oxymethylbenzene
Compound No. 855
4-(trans-4-(trans-4-(1-propenyl)cyclohexyloxycarbonyl) cyclohexyl)-1-n-propyloxymethylbenzene
Compound No. 856
4-(trans-4-(trans-4-(2-propenyl)cyclohexyloxycarbonyloxy) cyclohexyl)-1-(2-propenyl)oxymethylbenzene
Compound No. 857

4-(trans-4-(trans-4-vinylcyclohexyl)cyclohexyloxycarbonyl)-1-(3-butenyl)oxymethylbenzene
Compound No. 858
4-(trans-4-(trans-4-(2-propenyl)cyclohexyl) cyclohexylcarbonyloxy)-1-(2-propenyl)methylbenzene

Example 5

Synthesis of 4"-(trans-4-vinylcyclohexyl)-2'-fluoro-4-n-propyloxymethyl-p-terphenyl (Compound expressed by the general formula (1) wherein $R_1$ is n-$C_3H_7$, $R_2$ is -$CH_2$-, $R_3$ is vinyl group, n and m are 1, ring A and ring C are 1,4-phenylene group, ring B is 2-fluoro-1,4-phenylene group, ring D is 1,4-cyclohexylene group, and $Z_1$, $Z_2$, and $Z_3$ are single bond; Compound No. 859)

(First stage)

Potassium t-butoxide in an amount of 92.1 g (0.81 mol) was added to a solution prepared by dissolving 212.2 g (0.53 mol) of methyltriphenylphosphine iodide in 300 ml of THF and stirred at -50°C for 2 hours. To this solution was added dropwise in 1 hour a solution prepared by dissolving 61.0 g (0.35 mol) of the 4-(4-oxocyclohexyl)benzene prepared by using 1,4-dioxyspiro[2,5]octane-8-on as starting material according to a method described in the first stage to the third stage in Example 3 in 1 ℓ of THF, and stirred at the same temperature for 2 hours. Water in an amount of 300 ml was added to the reaction solution at a temperature lower than 0°C to terminate the reaction. Water layer separated from the reaction solution was extracted with toluene (twice, each time 1 ℓ). After the extract was washed with saturated aqueous sodium chloride solution (thrice, each time 500 ml) and dried over anhydrous magnesium sulfate, the solvent was distilled off to obtain a crude product. The crude product thus obtained was purified by silica gel column chromatography (eluent: toluene) and recrystallized from n-heptane to obtain 60.2 g of 4-(4-vinylcyclohexyl)benzene.

(Second stage)

Iodic acid in an amount of 34.1 g (0.19 mol), 45.1 g (0.18 mol) of iodine, 15.9 g (0.16 mol) of a concentrated sulfuric acid, 120 ml of water, and 800 ml of acetic acid were added to a solution prepared by dissolving 60.2 g (0.32 mol) of the 4-(4-vinylcyclohexyl)benzene obtained by the first stage in 150 ml of carbon tetrachloride, and heated to reflux for 8 hours. The reaction solution was poured into 1 ℓ of an ice water and extracted with toluene (thrice, each time 1 ℓ). After the extract was washed with saturated aqueous sodium chloride solution (thrice, each time 500 ml) and dried over anhydrous magnesium sulfate, the solvent was distilled off to obtain a crude product. The product thus obtained was purified by silica gel column chromatography (eluent: toluene) to obtain 92.8 g 4-(4-vinylcyclohexyl)-1-iodobenzene.

(Third stage)

Anhydrous ferric chloride in an amount of 0.20 g (0.0012 mol) was added to a solution prepared by dissolving 40.0 g (0.20 mol) of the 2'-fluoro-1-methoxymethylbiphenyl obtained according to a method described in Laid-open Japanese Patent Publication No. Hei 2-240039 in 500 ml of chloroform, and cooled down to -20°C while stirring. Bromine in an amount of 41.6 g (0.26 mol) was added dropwise thereto in 1 hour and then stirred at -10°C for 1 hour. After 200 ml of water was added to the reaction solution to terminate the reaction, the reaction product was extracted with methylene chloride (thrice, each time 500 ml). After the extract was washed with 300 ml of saturated aqueous solution of sodium thiosulfate and saturated aqueous sodium chloride solution (thrice, each time 400 ml) in turn and dried over anhydrous magnesium sulfate, the solvent was distilled off to obtain a crude product. The crude product thus obtained was purified by silica gel column chromatography (eluent: n-heptane) to obtain 32.1 g of 2'-fluoro-4'-bromo-1-methoxymethylbiphenyl.

(Fourth stage)

Solution prepared by dissolving the 2'-fluoro-4'-bromo-1-methoxymethylbiphenyl obtained by the third stage in 50 ml of THF was added dropwise in 2 hours to a liquid prepared by suspending 2.6 g (0.11 mol) of magnesium in 10 ml of THF. After finishing of the dropping, 100 ml of THF was added thereto and stirred at 50°C for 1 hour to prepare a Grignard reagent.

(Fifth stage)

Palladium chloride in an amount of 0.28 g (0.0015 mol) was added to a solution prepared by dissolving 24.1 g (0.077 mol) of the 4-(4-vinylcyclohexyl)-1-iodobenzene in 100 ml of THF and heated up to 60°C, and then the Grignard

reagent obtained by the fourth stage was added dropwise thereto while stirring in 30 min. After finishing of the dropping, the solution was stirred at the same temperature for 2 hours. Then, the reaction solution was cooled with ice, 100 ml of water was added thereto, and the reaction product was extracted with toluene (thrice, each time 300 ml). After the extract was washed with saturated aqueous sodium chloride solution (thrice, each time 200 ml) and dried over anhydrous magnesium sulfate, the solvent was distilled off to obtain a crude product. The crude product thus obtained was purified by silica gel column chromatography (eluent: toluene) to obtain 5.6 g (0.015 mol) of 4"-(trans-4-vinylcyclohexyl)-2'-fluoro-4-n-propyloxymethyl-p-terphenyl.

Various spectrum data of this compound well supported its structure.

According to the preparation method described above, the following compounds can be prepared:

Compound No. 860
4"-(trans-4-vinylcyclohexyl)-4-methoxymethyl-p-terphenyl
Compound No. 861
4"-(trans-4-vinylcyclohexyl)-4-n-propyloxymethyl-p-terphenyl
Compound No. 862
4"-(trans-4-vinylcyclohexyl)-4-n-pentyloxymethyl-p-terphenyl
Compound No. 863
4"-(trans-4-vinylcyclohexyl)-4-ethyloxymethyl-p-terphenyl
Compound No. 864
4"-(trans-4-vinylcyclohexyl)-4-(2-propenyl)oxymethyl-p-terphenyl
Compound No. 865
4"-(trans-4-vinylcyclohexyl)-4-(3-butenyl)oxymethyl-p-terphenyl
Compound No. 866
4"-(trans-4-vinylcyclohexyl)-2'-fluoro-4-methoxymethyl-p-terphenyl
Compound No. 867
4"-(trans-4-vinylcyclohexyl)-2'-fluoro-4-(2-pentenyl)oxymethyl-p-terphenyl
Compound No. 868
4"-(trans-4-vinylcyclohexyl)-2'-fluoro-4-n-propyloxymethyl-p-terphenyl
Compound No. 869
4"-(trans-4-vinylcyclohexyl)-3'-fluoro-4-methoxymethyl-p-terphenyl
Compound No. 870
4"-(trans-4-vinylcyclohexyl)-3'-fluoro-4-n-butoxymethyl-p-terphenyl
Compound No. 871
4"-(trans-4-vinylcyclohexyl)-3'-fluoro-4-n-pentyloxymethyl-p-terphenyl
Compound No. 872
4"-(trans-4-vinylcyclohexyl)-2-fluoro-4-methoxymethyl-p-terphenyl
Compound No. 873
4"-(trans-4-vinylcyclohexyl)-2-fluoro-4-(2-propenyl)oxymethyl-p-terphenyl
Compound No. 874
4"-(trans-4-vinylcyclohexyl)-2-fluoro-4-n-pentyloxymethyl-p-terphenyl
Compound No. 875
4"-(trans-4-vinylcyclohexyl)-3-fluoro-4-methoxymethyl-p-terphenyl
Compound No. 876
4"-(trans-4-vinylcyclohexyl)-3-fluoro-4-n-propyloxymethyl-p-terphenyl
Compound No. 877
4"-(trans-4-vinylcyclohexyl)-3-fluoro-4-n-pentyloxymethyl-p-terphenyl
Compound No. 878
2"-fluoro-4"-(trans-4-vinylcyclohexyl)-4-methoxymethyl-p-terphenyl
Compound No. 879
2"-fluoro-4"-(trans-4-vinylcyclohexyl)-4-n-propyloxymethyl-p-terphenyl
Compound No. 880
2"-fluoro-4"-(trans-4-vinylcyclohexyl)-4-n-pentyloxymethyl-p-terphenyl
Compound No. 881
3"-fluoro-4"-(trans-4-vinylcyclohexyl)-4-methoxy-n-propyl-p-terphenyl
Compound No. 882
3"-fluoro-4"-(trans-4-vinylcyclohexyl)-4-n-propyloxymethyl-p-terphenyl
Compound No. 883
3"-fluoro-4"-(trans-4-vinylcyclohexyl)-4-n-pentyloxymethyl-p-terphenyl

Compound No. 884
4"-(trans-4-vinylcyclohexyl)-3",2'-difluoro-4-methoxymethyl-p-terphenyl
Compound No. 885
4"-(trans-4-vinylcyclohexyl)-2",3'-difluoro-4-n-propyloxymethyl-p-terphenyl
Compound No. 886
4"-(trans-4-vinylcyclohexyl)-2',3'-difluoro-4-n-propyloxymethyl-p-terphenyl
Compound No. 887
4"-(trans-4-vinylcyclohexyl)-2',3',2-trifluoro-4-methoxymethyl-p-terphenyl
Compound No. 888
4"-(trans-4-vinylcyclohexyl)-2',5'-difluoro-4-n-propyloxymethyl-p-terphenyl
Compound No. 889
4"-(trans-4-vinylcyclohexyl)-2",2',2-trifluoro-4-n-propyloxymethyl-p-terphenyl
Compound No. 890
4"-(trans-4-(1-propenyl)cyclohexyl)-4-methoxymethyl-p-terphenyl
Compound No. 891
4"-(trans-4-(1-propenyl)cyclohexyl)-4-n-propyloxymethyl-p-terphenyl
Compound No. 892
4"-(trans-4-(2-propenyl)cyclohexyl)-4-methoxyethyl-p-terphenyl
Compound No. 893
4"-(trans-4-(2-propenyl)cyclohexyl)-4-n-propyloxymethyl-p-terphenyl
Compound No. 894
4"-(trans-4-(2-propenyl)cyclohexyl)-4-(2-propenyl)oxymethyl-p-terphenyl
Compound No. 895
4"-(trans-4-(2-propenyl)cyclohexyl)-4-(3-butenyl)oxymethyl-p-terphenyl
Compound No. 896
4"-(trans-4-(1-butenyl)cyclohexyl)-4-methoxymethyl-p-terphenyl
Compound No. 897
4"-(trans-4-(1-butenyl)cyclohexyl)-4-n-propyloxymethyl-p-terphenyl
Compound No. 898
4"-(trans-4-(2-butenyl)cyclohexyl)-4-methoxymethyl-p-terphenyl
Compound No. 899
4"-(trans-4-(2-butenyl)cyclohexyl)-4-n-propyloxymethyl-p-terphenyl
Compound No. 900
4"-(trans-4-(3-butenyl)cyclohexyl)-4-methoxymethyl-p-terphenyl
Compound No. 901
4"-(trans-4-(3-butenyl)cyclohexyl)-4-(2-propenyl)oxymethyl-p-terphenyl
Compound No. 902
4"-(trans-4-(1-pentenyl)cyclohexyl)-4-methoxymethyl-p-terphenyl
Compound No. 903
4"-(trans-4-(1-pentenyl)cyclohexyl)-4-(2-butenyl)oxymethyl-p-terphenyl
Compound No. 904
4"-(trans-4-(2-pentenyl)cyclohexyl)-4-(2-propenyl)oxymethyl-p-terphenyl
Compound No. 905
4"-(trans-4-(2-pentenyl)cyclohexyl)-4-n-propyloxymethyl-p-terphenyl
Compound No. 906
4"-(trans-4-(3-pentenyl)cyclohexyl)-4-ethyloxymethyl-p-terphenyl
Compound No. 907
4"-(trans-4-(3-pentenyl)cyclohexyl)-4-n-propyloxymethyl-p-terphenyl
Compound No. 908
4"-(trans-4-(4-pentenyl)cyclohexyl)-4-methoxymethyl-p-terphenyl
Compound No. 909
4"-(trans-4-(4-pentenyl)cyclohexyl)-4-(3-butenyl)oxymethyl-p-terphenyl
Compound No. 910
4"-(trans-4-(2-propenyl)cyclohexyl)-2'-fluoro-4-n-propyloxymethyl-p-terphenyl
Compound No. 911
4"-(trans-4-(1-pentenyl)cyclohexyl)-2'-fluoro-4-n-pentyloxymethyl-p-terphenyl
Compound No. 912
4"-(trans-4-(3-pentenyl)cyclohexyl)-3'-fluoro-4-methoxymethyl-p-terphenyl

Compound No. 913
4"-(trans-4-vinylcyclohexyl)-3'-fluoro-4-methoxy-n-propenyl-p-terphenyl
Compound No. 914
4"-(trans-4-(1-propenyl)cyclohexyl)-2-fluoro-4-methoxymethyl-p-terphenyl
Compound No. 915
4"-(trans-4-(1-butenyl)cyclohexyl)-3-fluoro-4-(2-propenyl)oxymethyl-p-terphenyl
Compound No. 916
4"-(trans-4-(4-pentenyl)cyclohexyl)-3-fluoro-4-methoxymethyl-p-terphenyl
Compound No. 917
4"-(2-(trans-4-vinylcyclohexyl)ethyl)-2'-fluoro-4-n-propyloxymethyl-p-terphenyl
Compound No. 918
4"-(4-(trans-4-vinylcyclohexyl)butyl)-3'-fluoro-4-n-butoxymethyl-p-terphenyl
Compound No. 919
4"-((trans-4-vinylcyclohexyl)methoxymethyl)-2-fluoro-4-n-pentyloxymethyl-p-terphenyl
Compound No. 920
4"-((trans-4-(2-propenyl)cyclohexyl)oxymethyl)-3-fluoro-4-methoxymethyl-p-terphenyl
Compound No. 921
4"-((trans-4-vinylcyclohexyl)difluoromethyloxy)-2'-fluoro-4-n-propyloxymethyl-p-terphenyl
Compound No. 922
4"-((trans-4-vinylcyclohexyl)oxydifluoromethyl)-3'-fluoro-4-n-pentyloxymethyl-p-terphenyl
Compound No. 923
2"-fluoro-4"-(4-(trans-4-vinylcyclohexyl)-2-butenyl)-4-methoxymethyl-p-terphenyl
Compound No. 924
3"-fluoro-4"-(4-(trans-4-vinylcyclohexyl)-1,4-dibutenyl)-4-n-propyloxymethyl-p-terphenyl
Compound No. 925
4"-(4-(trans-4-vinylcyclohexyl)-3-butenyl)-4-n-pentyloxymethyl-p-terphenyl
Compound No. 926
4'-(2-(4-(trans-4-(2-propenyl)cyclohexyl)phenyl)ethyl)-2'-fluoro-4-methoxymethylbiphenyl
Compound No. 927
4'-(2-(4-(trans-4-(2-propenyl)cyclohexyl)phenyl)ethyl)-2'-fluoro-4-n-propyloxymethylbiphenyl
Compound No. 928
4'-(4-(4-(trans-4-vinylcyclohexyl)phenyl)butyl)-3'-fluoro-4-n-propyloxymethyl-p-terphenyl
Compound No. 929
4-(2-(4'-(trans-4-(4-pentenyl)cyclohexyl)biphenyl)ethyl)-2-fluoro-4-methoxymethylbenzene
Compound No. 930
4-(4-(2'-fluoro-4'-(trans-4-vinylcyclohexyl)biphenyl)butyl)-4-n-propyloxymethylbenzene
Compound No. 931
4-(4-(2'-fluoro-4'-(trans-4-vinylcyclohexyl)biphenyl)-2-butyl)-4-n-pentyloxymethylbenzene
Compound No. 932
4'-(2-(4-(2-(trans-4-(2-propenyl)cyclohexyl)ethyl)-2-fluoro-4-n-propyloxymethylbiphenyl
Compound No. 933
4-(2-(4-(4-(2-(trans-4-vinylcyclohexyl)ethyl)phenyl) phenyl)ethyl)-2-fluoro-1-n-propyloxymethylbenzene
Compound No. 934
4-(2-(4-(2-(4-(2-(trans-4-vinylcyclohexyl)ethyl)phenyl)ethyl) phenyl)ethyl)-2-fluoro-1-n-propyloxymethylbenzene
Compound No. 935
4'-(2-(4-(4-(trans-4-vinylcyclohexyl)butyl)phenyl)ethyl)-2-fluoro-4-methoxymethylbiphenyl
Compound No. 936
4-(4-(4-(4-(2-(trans-4-vinylcyclohexyl)ethyl)phenyl) phenyl)butyl)-2-fluoro-1-n-propyloxymethylbenzene
Compound No. 937
4-(4-(4-(4-(4-(trans-4-(2-propenyl)cyclohexyl)phenyl)butyl) phenyl)butyl)-2-fluoro-1-n-propyloxymethylbenzene
Compound No. 938
4-(4-(4-(4-(4-(4-(trans-4-vinylcyclohexyl)butyl)phenyl)butyl) phenyl)butyl)-2-fluoro-1-n-propyloxymethylbenzene
Compound No. 939
4'-(trans-4-(trans-4-vinylcyclohexyl)cyclohexyl)-4-methoxymethylbiphenyl
Compound No. 940
4'-(trans-4-(trans-4-vinylcyclohexyl)cyclohexyl)-4-n-propyloxymethylbiphenyl
Compound No. 941
4'-(trans-4-(trans-4-vinylcyclohexyl)cyclohexyl)-4-(2-propenyl)oxymethylbiphenyl

Compound No. 942
4'-(trans-4-(trans-4-(1-propenyl)cyclohexyl)cyclohexyl)-4-n-propyloxymethylbiphenyl
Compound No. 943
4'-(trans-4-(trans-4-(2-propenyl)cyclohexyl)cyclohexyl)-4-n-propyloxymethylbiphenyl
Compound No. 944
4'-(trans-4-(trans-4-(1-butenyl)cyclohexyl)cyclohexyl)-4-n-propyloxymethylbiphenyl
Compound No. 945
4'-(trans-4-(trans-4-(2-butenyl)cyclohexyl)cyclohexyl)-4-(2-propenyl)oxymethylbiphenyl
Compound No. 946
4'-(trans-4-(trans-4-(3-butenyl)cyclohexyl)cyclohexyl)-4-n-propyloxymethylbiphenyl
Compound No. 947
4-(trans-4-(trans-4-(trans-4-vinylcyclohexyl)cyclohexyl) cyclohexyl)-1-n-propyloxymethylbenzene
Compound No. 948
4-(trans-4-(trans-4-(trans-4-(2-propenyl)cyclohexyl)cyclohexyl) cyclohexyl)-1-methoxymethylbenzene
Compound No. 949
4-(trans-4-(4-(trans-4-vinylcyclohexyl)phenyl)cyclohexyl)-1-methoxymethylbenzene
Compound No. 950
4-(trans-4-(2-fluoro-4-(trans-4-vinylcyclohexyl)phenyl) cyclohexyl)-1-n-propyloxymethylbenzene
Compound No. 951
4-(trans-4-(trans-4-vinylcyclohexyl)cyclohexyl)-4-n-propyloxymethylbiphenyl
Compound No. 952
4-(trans-4-(trans-4-(2-propenyl)cyclohexyl)cyclohexyl)-4-methoxymethylbiphenyl
Compound No. 953
4-(trans-4-(trans-4-(2-(trans-4-vinylcyclohexyl)ethyl) cyclohexyl)cyclohexyl)-1-n-propyloxymethylbenzene
Compound No. 954
4-(trans-4-(4-(trans-4-(trans-4-(2-propenyl)cyclohexyl) cyclohexyl)butyl)cyclohexyl)-1-methoxymethylbenzene
Compound No. 955
4-(trans-4-(4-(4-(2-(trans-4-vinylcyclohexyl)ethyl)phenyl) butyl)cycloheyxl)-1-methoxymethylbenzene
Compound No. 956
4-(2-(trans-4-(2-(2-fluoro-4-(2-(trans-4-vinylcyclohexyl)ethyl) phenyl)ethyl)cycloheyxl)ethyl)-1-n-propyloxymethylbenzene

Example 6 (Use Example 1)

Liquid crystal composition comprising the following compounds in the amount shown below

| | |
|---|---|
| 4-(trans-4-propylcyclohexyl)benzonitrile | 24 % (by weight, hereinafter the same unit is used) |
| 4-(trans-4-pentylcyclohexyl)benzonitrile | 36 % |
| 4-(trans-4-heptylcyclohexyl)benzonitrile | 25 % |
| 4-(4-propylphenyl)benzonitrile | 15 % |

had a clearing point (Cp) of nematic liquid crystal of 72.4°C and viscosity at 20°C ($\eta_{20}$) of 27.0 mPa • s. When this liquid crystal composition was filled in a TN cell (twisted nematic cell) having a cell thickness of 9 $\mu$m, driving threshold voltage (Vth) of the liquid crystal composition was 1.78 V, value of dielectric anisotropy ($\Delta\varepsilon$) was +11.0, and value of optical anisotropy ($\Delta n$) was 0.137.

This liquid crystal composition (hereinafter referred to as Mother liquid crystal A) in an amount of 85 parts by weight was mixed with 15 parts by weight of the 4'-(trans-4-vinylcyclohexyl)-4-n-propyloxymethylbiphenyl obtained in Example 2, and physical properties of the mixture were determined. As the result, it was found that Cp was 103.0°C, $\eta_{20}$: 33.2 mPa • s, Vth: 1.68 V, $\Delta\varepsilon$: 5.0, $\Delta n$: 0.150, and the ratio of elastic constants K33/K11 was 2.00.

While this liquid crystal composition was left in a freezer at -20°C for 40 days, separation of crystals or development of smectic phase was not noticed.

Example 7 (Use Example 2)

Mother liquid crystal A shown in Example 6 in an amount of 85 parts by weight was mixed with 15 parts by weight of the 4-(trans-4-(trans-4-vinylcyclohexyl)cyclohexyl)-1-n-propyloxymethylbenzene obtained in Example 3, and physical properties of the mixture were determined. As the result, it was found that Cp was 81.7°C, $\eta_{20}$: 27.0 mPa • s, Vth: 1.73 V, $\Delta\varepsilon$: 3.0, $\Delta n$: 0.077, and the ratio of elastic constants K33/K11 was 2.18.

While this liquid crystal composition was left in a freezer at -20°C for 40 days, separation of crystals or development of smectic phase was not noticed.

Example 8 (Use Example 3)

Mother liquid crystal A shown in Example 6 in an amount of 85 parts by weight was mixed with 15 parts by weight of the 4-(trans-4-(trans-4-vinylcyclohexyl)cyclohexyl)-1-n-propyloxymethylbenzene obtained in Example 4, and physical properties of the mixture were determined. As the result, it was found that Cp was 89.0°C, $\eta_{20}$: 18.9 mPa • s, Vth: 1.68 V, $\Delta\varepsilon$: 1.7, $\Delta n$: 0.084, and the ratio of elastic constants K33/K11 was 2.35.

While this liquid crystal composition was left in a freezer at -20°C for 40 days, separation of crystals or development of smectic phase was not noticed.

INDUSTRIAL APPLICABILITY

As demonstrated in Examples 6 to 8, any of the compounds of the present invention exhibited a low viscosity, excellent mutual solubility, and good mutual solubility particularly at low temperatures. Also, any of the compounds of the present invention exhibited a larger ratio of elastic constants compared with known compounds described above.

These excellent characteristics are originated from the structure of the compounds of the present invention in which one terminal group is an alkoxyalkylbenzene or alkenyloxyalkylbenzene, and the other terminal group is an alkenyl group.

As described above, the compounds of the present invention have such excellent characteristics as 1) a low viscosity, 2) excellent mutual solubility, particularly at low temperatures, and 3) large ratio of elastic constants.

When a liquid crystal composition comprising the compound of the present invention is used from now on as the material for liquid crystal displays of STN mode, displays are expected to have more improved characteristics than those of conventional materials.

**Claims**

1. A liquid crystalline compound expressed by the general formula (1)

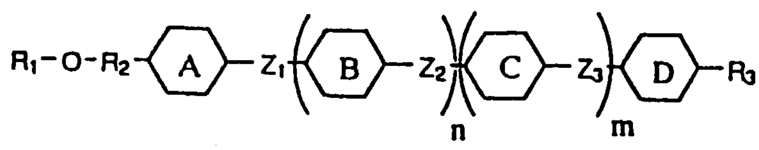

(1)

wherein $R_1$ represents an alkyl group having 1 to 15 carbon atoms or an alkenyl group having 2 to 15 carbon atoms; $R_2$ represents an alkylene group having 1 to 5 carbon atoms; $R_3$ represents an alkenyl group having 2 to 15 carbon atoms; ring A represents 1,4-phenylene group one or more hydrogen atoms on the ring may be replaced by a halogen atom; rings B, C, and D independently represent 1,4-cyclohexylene group, or 1,4-phenylene group one or more hydrogen atoms on the ring may be replaced by a halogen atom; $Z_1$, $Z_2$, and $Z_3$ independently represent an alkylene group having 1 to 5 carbon atoms, -CH=CH-, -C≡C-, $-CH_2-CH_2-CH=CH-$, $-CH=CH-CH_2-CH_2-$, $-CH_2-CH=CH-CH_2-$, or single bond, one or not-adjacent two or more $-CH_2-CH_2-$ groups in these groups may be replaced by $-CH_2O-$, $-OCH_2-$ $-CF_2O-$, $-OCF_2-$, -COO-, or -OCO-; and n and m are 0 or 1.

2. The liquid crystalline compound according to claim 1 wherein n and m are 0 in the general formula (1).

3. The liquid crystalline compound according to claim 2 wherein $R_2$ is methylene group and ring D is 1,4-cyclohexylene group in the general formula (1).

4. The liquid crystalline compound according to claim 1 wherein n is 0, and m is 1 in the general formula (1).

5. The liquid crystalline compound according to claim 4 wherein $R_2$ is methylene group, and ring D is 1,4-cyclohexylene group in the general formula (1).

6. The liquid crystalline compound according to claim 1 wherein both n and m are 1 in the general formula (1).

7. The liquid crystalline compound according to claim 6 wherein $R_2$ is methylene group, and ring D represents 1,4-cyclohexylene group in the general formula (1).

8. A liquid crystal composition comprising at least 2 components and comprising at least one liquid crystalline compound defined in claim 1.

9. A liquid crystal composition comprising, as a first component, at least one liquid crystalline compound defined in any one of claims 1 to 7; and comprising, as a second component, at least one compound selected from the group consisting of the compounds expressed by any one of the general formulas (2), (3), and (4)

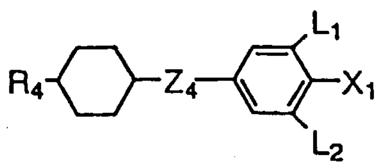

(2)

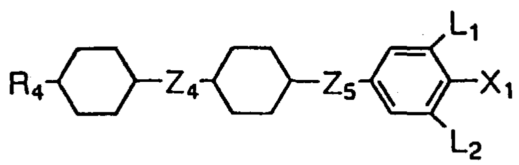

(3)

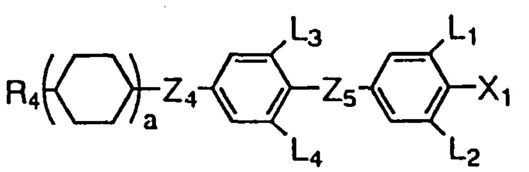

(4)

wherein $R_4$ represents an alkyl group having 1 to 10 carbon atoms; $X_1$ represents F, Cl, $OCF_3$, $OCF_2H$, $CF_3$, $CF_2H$, or $CFH_2$; $L_1$, $L_2$, $L_3$, and $L_4$ independently represent H or F; $Z_4$ and $Z_5$ independently represent $-(CH_2)_2-$, -CH=CH-, or single bond; and a is 1 or 2.

10. A liquid crystal composition comprising, as a first component, at least one liquid crystalline compound defined in any one of claims 1 to 7; and comprising, as a second component, at least one compound selected from the group consisting of the compounds expressed by any one of the general formulas (5), (6), (7), (8), and (9)

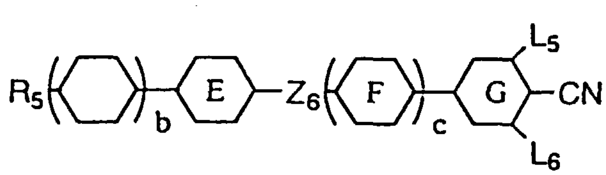

(5)

wherein $R_5$ represents F, an alkyl group having 1 to 10 carbon atoms, or an alkenyl group having 2 to 10 carbon atoms provided that one or not-adjacent two or more methylene ($-CH_2-$) groups in the alkyl or alkenyl group may be replaced by oxygen (-O-) atom; ring E represents trans-1,4-cyclohexylene group, 1,4-phenylene group, pyrimidine-2,5-diyl group, or 1,3-dioxane-2,5-diyl group; ring F represents trans-1,4-cyclohexylene group, 1,4-phenylene group, or pyrimidine-2,5-diyl group; ring G represents trans-1,4-cyclohexylene group or 1,4-phenylene group; $Z_6$ represents $-(CH_2)_2-$, -COO-, or single bond; $L_5$ and $L_6$ independently represent H or F; and b and c are independently 0 or 1,

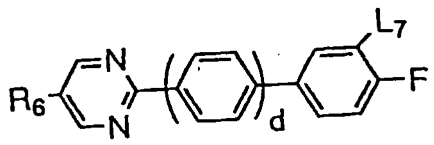

(6)

wherein $R_6$ represents an alkyl group having 1 to 10 carbon atoms; $L_7$ represents H or F; and d is 0 or 1,

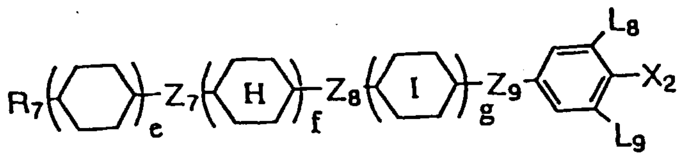

(7)

wherein $R_7$ represents an alkyl group having 1 to 10 carbon atoms, ring H and ring I independently represent trans-1,4-cyclohexylene group or 1,4-phenylene group; $Z_7$ and $Z_8$ independently represent -COO- or single bond; $Z_9$ represents -COO- or -C≡C-; $L_8$ and $L_9$ independently represent H or F; $X_2$ represents F, $OCF_3$, $OCF_2H$, $CF_3$, $CF_2H$, or $CFH_2$; and e, f, and g are independently 0 or 1,

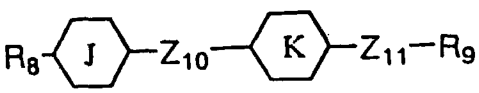

(8)

wherein $R_8$ and $R_9$ independently represent an alkyl group having 1 to 10 carbon atoms or an alkenyl group having 2 to 10 carbon atoms provided that one or not-adjacent two or more methylene $(-CH_2-)$ groups in the alkyl or alkenyl group may be replaced by oxygen (-O-) atom; ring J represents trans-1,4-cyclohexylene group, 1,4-phenylene group, or pyrimidine-2,5-diyl group; ring K represents trans-1,4-cyclohexylene group or 1,4-phenylene group; $Z_{10}$ represents -C≡C-, -COO-, $-(CH_2)_2-$, -CH=CH-C≡C-, or single bond; and $Z_{11}$ represents -COO- or single bond,

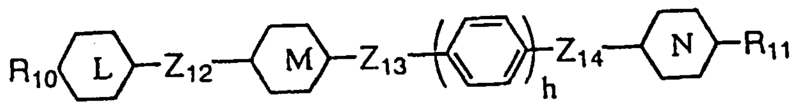

(9)

wherein $R_{10}$ and $R_{11}$ independently represent an alkyl group having 1 to 10 carbon atoms or an alkenyl group having 2 to 10 carbon atoms provided that one or not-adjacent two or more methylene $(-CH_2-)$ groups in the alkyl or alkenyl group may be replaced by oxygen (-O-) atom; ring L represents trans-1,4-cyclohexylene group, 1,4-phenylene group, or pyrimidine-2,5-diyl group; ring M represents trans-1,4-cyclohexylene group, pyrimidine-2,5-diyl group, or 1,4-phenylene group one or more hydrogen atoms on the ring may be replaced by fluorine atom; ring N represents trans-1,4-cyclohexylene group or 1,4-phenylene group, $Z_{12}$ and $Z_{14}$ independently represent -COO-, $-(CH_2)_2-$ or single bond; $Z_{13}$ represents -CH=CH-, -C≡C-, -COO-, or single bond; and h is 0 or 1.

11. A liquid crystal composition comprising, as a first component, at least one liquid crystalline compound defined in any one of claims 1 to 7; comprising, as a part of a second component, at least one compound selected from the group consisting of the compounds expressed by any one of the general formulas (2), (3), and (4); and comprising, as another part of the second component, at least one compound selected from the group consisting of the compounds expressed by any one of the general formulas (5), (6), (7), (8), and (9).

**12.** A liquid crystal display device utilizing a liquid crystal composition defined in any one of claims 8 to 11.

INTERNATIONAL SEARCH REPORT

International application No.
PCT/JP97/00148

A. CLASSIFICATION OF SUBJECT MATTER Int. Cl[6] C07C43/166, 43/176, 43/188, 43/192, 43/215, 43/225, 69/734, 69/757, 69/92, 69/587, 69/608, 69/612, 69/65, 69/73, 69/74, 69/76, C09K19/30, 19/10, 19/42, G02F1/13

According to International Patent Classification (IPC) or to both national classification and IPC

B. FIELDS SEARCHED

Minimum documentation searched (classification system followed by classification symbols)
Int. Cl[6] C07C43/166, 43/176, 43/188, 43/192, 43/215, 43/225, 69/734, 69/757, 69/92, 69/587, 69/608, 69/612, 69/65, 69/73, 69/74, 69/76, C09K19/30, 19/10-20, 19/42-46, G02F1/13

Documentation searched other than minimum documentation to the extent that such documents are included in the fields searched

Electronic data base consulted during the international search (name of data base and, where practicable, search terms used)
CAS ONLINE

C. DOCUMENTS CONSIDERED TO BE RELEVANT

| Category* | Citation of document, with indication, where appropriate, of the relevant passages | Relevant to claim No. |
|---|---|---|
| PX | EP, 754668, A2 (Rolic AG.), January 22, 1997 (22. 01. 97) (Family: none) | 1-5, 8-12 |
| A | JP, 4-500214, A (Merck Patent GmbH.), January 16, 1992 (16. 01. 92) & WO, 90/15114, A & EP, 427837, A | 1 - 12 |
| A | JP, 7-53432, A (Merck Patent GmbH.), February 28, 1995 (28. 02. 95) & DE, 4426799, A1 | 1 - 12 |
| A | DE, 4211694, A1 (Merck Patent GmbH.), November 4, 1993 (04. 11. 93) (Family: none) | 1 - 12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| April 7, 1997 (07. 04. 97) | April 15, 1997 (15. 04. 97) |
| Name and mailing address of the ISA/ Japanese Patent Office | Authorized officer |
| Facsimile No. | Telephone No. |

Form PCT/ISA/210 (second sheet) (July 1992)